# EUROPEAN PATENT SPECIFICATION

(11) **EP 3 418 299 B1**
(45) Date of publication and mention of the grant of the patent: **13.11.2024**
(21) Application number: 18171097.1
(22) Date of filing: 11.09.2008
(51) Int. Cl.: C07K 16/00, C07K 16/28, A61K 39/395

(54) **HOMOGENEOUS ANTIBODY POPULATIONS**
HOMOGENE ANTIKÖRPERPOPULATIONEN
POPULATIONS D'ANTICORPS HOMOGÈNES

(30) Priority: 14.09.2007 US 972688 P
(43) Date of publication of application: 26.12.2018
(62) Divisional of application: 08799478.6
(73) Proprietor: Amgen Inc., Thousand Oaks, California 91320-1799 (US)
(72) Inventor: Dillon, Thomas M., Ventura, CA 93003 (US); Bondarenko, Pavel, Thousand Oaks, CA 91320 (US); Wypych, Jette, Calabasas, CA 91302 (US); Allen, Martin J., Webster Groves, MO 63119 (US); Balland, Alain, 88400 Biberach an der Riss (DE); Ricci, Margaret Speed, Camarillo, CA 93012 (US); Guo, Amy, Mercer Island, WA 98040 (US); Shen, Wenyan, Redwood City, CA 94065 (US); Sun, Jeonghoon, Poway, CA 92064 (US); Vezina, Christopher John, Newbury Park, CA 91320 (US)
(74) Representative: Grünecker Patent- und Rechtsanwälte PartG mbB

(56) References cited:
- WO-A2-2005/000899
- WO-A2-2006/047340
- GLASER SCOTT M ET AL: "Novel antibody hinge regions for efficient production of CH2 domain-deleted antibodies.", THE JOURNAL OF BIOLOGICAL CHEMISTRY 16 DEC 2005, vol. 280, no. 50, 16 December 2005 (2005-12-16), pages 41494 - 41503, XP002512402, ISSN: 0021-9258
- ANGAL S ET AL: "A SINGLE AMINO ACID SUBSTITUTION ABOLISHES THE HETEROGENEITY OF CHIMERIC MOUSE/HUMAN (IGG4) ANTIBODY", MOLECULAR IMMUNOLOGY, PERGAMON, GB, vol. 30, no. 1, 1 January 1993 (1993-01-01), pages 105 - 108, XP000770218, ISSN: 0161-5890
- DILLON ET AL: "Optimization of a reversed-phase high-performance liquid chromatography/mass spectrometry method for characterizing recombinant antibody heterogeneity and stability", JOURNAL OF CHROMATOGRAPHY, ELSEVIER SCIENCE PUBLISHERS B.V. AMSTERDAM, NL, vol. 1120, no. 1-2, 7 July 2006 (2006-07-07), pages 112 - 120, XP005504307, ISSN: 0021-9673

## Description

### RELATED APPLICATIONS

This application claims priority to U.S. Provisional Patent Application No: 60/972,688 filed on September 14, 2007.

### BACKGROUND OF THE INVENTION

### Field of the Invention

The invention provides a method of making a modified IgG2 anti-EGFR antibody comprising: modifying a nucleotide sequence encoding the heavy chain polypeptide of an IgG2 anti-EGFR antibody such that at least one encoded amino acid is modified; introducing said nucleic acid into a host cell; and culturing said host cell such that a plurality of modified IgG2 anti-EGFR antibodies is expressed and secreted, wherein a substantial population that is at least 50% of the total population of said plurality of modified IgG2 antibodies are a single conformational isoform, wherein said modification comprises a heavy chain polypeptide modification of said antibody, and wherein said heavy chain polypeptide modification comprises a substitution of the cysteine residue at Eu position 131 of said heavy chain polypeptide with serine (C131S), and wherein said heavy chain polypeptide modification further comprises a mutation of a cysteine residue within the hinge region, and wherein said mutation of a cysteine residue within said hinge region of said heavy chain polypeptide comprises a mutation of the cysteine residue at Eu position 219 or 220 of said heavy chain polypeptide to serine (C219S or C220S), and wherein the heavy chain of said modified IgG2 anti-EGFR antibody comprises the variable region SEQ ID NO: 57.

### Description of the Related Art

In the 1960's, extensive studies performed with specific polyclonal rabbit antisera against homogeneous human IgG myeloma proteins revealed the existence of four distinct subgroups of human IgG, which were designated IgG1, IgG2, IgG3 and IgG4, respectively. The four subclasses show large homology in the amino acid sequences of the constant domains of the y-heavy chains. The four IgG subclasses show their most conspicuous differences in the amino acid composition and structure of the 'hinge region', which is the part of the molecule containing disulfide bonds between the two heavy chains. This region, between the Fab arms (Fragment antigen binding) and the two carboxy-terminal domains (C_{H}2 and C_{H}3) of both heavy chains, helps to define the flexibility of the molecule.

The upper hinge (towards the amino-terminal) segment allows variability of the angle between the Fab arms (Fab-Fab flexibility) as well as rotational flexibility of each individual Fab. The flexibility of the lower hinge region (towards the carboxy-terminal) helps determine the position of the Fab-arms relative to the Fc region (Fab-Fc flexibility). Hinge-dependent Fab-Fab and Fab-Fc flexibility may be important in triggering further effector functions such as complement activation and Fc receptor binding.

Disulfide bond formation in proteins *in vivo* is a complex process, which is affected by the redox potential of the environment and specialized thiol-disulfide exchanging enzymes (Creighton, Methods Enzymol. 107, 305-329, 1984; Houee-Levin, Methods Enzymol. 353, 35-44,2002; Ritz and Beckwith, Roles of thiol-redox pathways in bacteria, Annu. Rev. Microbiol. 55, 21-48, 2001.) The disulfides are formed in cells during or shortly after secretion of the nascent chains into the endoplasmic reticulum (Creighton, Methods Enzymol. 107, 305-329, 1984). Several conformational isoforms of the same protein, but with different disulfide structures, can be generated during recombinant protein production in mammalian cells due to a failing disulfide formation process, close proximity of three or more cysteine residues in the protein structure or surface exposure of unpaired cysteine residues.

Although the most currently studied therapeutic mAbs are of the IgG1 or IgG4 subtypes, IgG2 antibodies may be preferred over other subclasses for certain indications due to their low level of effector functions (Canfield and Morrison, J. Exp. Med. 173, 1483-1491, 1991). However, there is limited structural information documenting the assignment of disulfide bonds in recombinant IgG2-type monoclonal antibodies

Recent advances in antibody therapeutics have caused a renewed interest in improving the understanding of antibody structure and its relationship to biological function. IgG1 and IgG2 subclasses have attracted special interest, because they are the most abundant, long lasting and stable immunoglobulins in circulation.

It has been suggested in several previous reports, that IgG2 molecules contain free thiol groups and are structurally heterogeneous as compared to other subclasses of gamma globulins. In one report, the content of free thiol groups was determined for all four human IgG antibodies by the reaction with 5,5'-dithio(2,2'-dinitro)benzoate (DTNB)(Schauenstein et al 1986 Int. Arch. Allergy Immunol., 80:174-179). The uncovered free thiols (about 0.24 per mole of human IgG) were assigned to IgG2 subclass. Others have also reported that all four human IgG subclasses were subjected to reduction of interchain disulfide bonds by thioredoxin with thioredoxin reductase and NADPH. IgG2 was found to be different from other subclasses in two effects: 1) it resisted reduction and 2) consumed NADPH reagent. The later finding suggested that the reagent was consumed by reduction of a labile interchain or surface-exposed mixed disulfide. In yet another study, IgG2 covalent dimers were detected in pooled human gamma globulin and several normal sera (Yoo et al., 2003, J. Immunol., 170:3134-3138). Cyanogen bromide cleavage analysis of the dimers indicated that one or more cysteine residues in the hinge are involved in dimer assembly, again suggesting presence of free or labile cysteines in hinge of IgG2. A study by Phillips et al. (J. Immun., 31:1201-1210, 1994), using sedimentation and electron microscopy analysis, identified multiple shapes of IgG2 molecules and their complexes with bivalent hapten and only a single form for other three subclasses of human gamma globulins. Yet in none of this seminal work was there a reference to structural heterogeneity existing within the IgG2 subclass. It has been reported that a serine to proline mutation in the IgG4 hinge reduces heterogeneity by eliminating HL "half antibodies" and enhancing formation of H2L2 tetramers (Angal et al., 1993, Mol. Immunol., 30:105-108, incorporated herein by reference in its entirety). Further, it has been reported that hybrid IgG isotypes with the CH1, CH2, and/or CH3 domains of an IgG2 antibody, combined with the hinge region from IgG1 are useful for high-level expression (U.S. Patent No. 7,148,321.

According to a recent report, well over 200 structures of antibody fragments, mainly Fab and Fab', have been determined (Saphire et al., 2002, J. Mol. Biol., 319:9-18). Crystals of intact antibodies have been reported only ten times and only seven of these crystals provided partial or complete structures. All these structures were either murine IgG or human IgGI antibodies, but not human IgG2 (Saphire et al., 2002, J. Mol. Biol., 319:9-18). Entire structures of IgGs with full-length hinges have been reported only three times: mAb 231, a murine IgG2a (Harris et al., 1992, Nature, 360:369-372; Larson et al., 1991, J. Mol. Biol., 222 :17-19), mAb 61.1.3, a murine IgG1 (Harris et al., 1998, J. Mol. Biol., 275 : 861-872); and a human IgG1 b12, directed against HIV-1 gp120 (Saphire et al., 2001, Science, 293 : 1155-1159; Saphire et al., 2002, J. Mol. Biol., 319: 9-18). Fragments of the crystal image of a human IgG1 antibody near the hinge from PDB number 1HZH are available (Saphire et al., 2001, Science, 293: 1155-1159). Recently-developed methods of analysis of intact antibodies by using reversed-phase chromatography on-line with mass spectrometry have been reported which have helped to facilitate discovery and characterization of heterogeneity of human IgG2 antibodies (Dillon et al., 2004, J. Chromatogr. A, 1053:299-305).

Recently, structural heterogeneity in the IgG2 subclass has been observed, although the reasons underlying this heterogeneity have remained unexplained. For example, U.S. patent application Pub. No: 2005/0161399, Dillon et al., discusses a reversed-phase LC/MS method of analyzing high molecular weight proteins, including antibodies. In addition, U.S. patent application Pub. No: 2006/194280, Dillon et al., is directed to methods of transiently enriching particular IgG isoforms by subjecting preparations of recombinant IgG proteins with a reduction/oxidation coupling reagent and optionally a chaotropic agent.

WO 2006/047340 A2 describes a method for refolding of recombinant antibodies.

WO 2005/000899 A2 describes modified binding molecules comprising connecting peptides.

Glaser S.M. et al., The Journal of Biological Chemistry, published 16 December 2005, vol. 280, no. 50, pages 41494-41503, describes antibody hinge regions for efficient production of C_{H}2 domain-deleted antibodies.

Angal S. et al., Molecular Immunology, Pergamon, GB, vol. 30, no. 1, published 1 January 1993, pages 105-108, describes an amino acid substitution in the hinge of an IgG4 antibody that abolishes heterogeneity.

Dillon T.M. et al., Journal of Chromatography, Elsevier Science Publishers B.V. Amsterdam, NL, vol. 1120, no. 1-2, published 30 January 2006, pages 112-120, describes an optimized reversed-phase high-performance liquid chromatography/mass spectrometry method for characterizing recombinant antibody heterogeneity and stability.

### SUMMARY OF THE INVENTION

The invention is defined in the appended claims. Embodiments of the invention are directed to providing efficient and economic production, purification and analysis of homogeneous populations of antibody subtypes where the desired conformational isoform has been produced. More particularly, the invention describes methods of making homogeneous IgG2 disulfide forms by engineering human IgG2 antibodies to produce improved pharmaceutical properties, including improved storage stability. As described in further detail hereinbelow, the substitution, of defined amino acid residues in the IgG2 molecule can facilitate the elimination of disulfide heterogeneity and thus produce structurally homogeneous, more active single conformational isoforms of the IgG2 antibody.

In accordance with the above, described herein is a monoclonal IgG2 antibody, comprising: a light chain polypeptide; and a heavy chain polypeptide having a hinge region, wherein the antibody comprises an amino acid modification in the heavy or light chain polypeptide such that the modification provides a light chain polypeptide that primarily forms an interchain disulfide bond with amino acids in the hinge region of the heavy chain polypeptide through the most C-terminal cysteine residue of the light chain polypeptide. The modification comprises a heavy chain polypeptide modification. In certain aspects, the modification can comprise a substitution of a cysteine residue at Eu position 131 of the heavy chain polypeptide. In certain aspects the hinge region comprises Eu amino acids 200 to 238 of the heavy chain polypeptide. In certain aspects, the most C-terminal cysteine residue of the light chain polypeptide is the cysteine residue at Eu position 214 of the light chain polypeptide. In certain aspects, the light chain polypeptide always forms an interchain disulfide bond only with amino acids in the hinge region of the heavy chain polypeptide through the most C-terminal cysteine residue of the light chain polypeptide.

Also described herein is a monoclonal lgG2 antibody, comprising: a light chain polypeptide; and a heavy chain polypeptide having a hinge region, wherein the antibody comprises an amino acid modification in the heavy or light chain polypeptide such that the modification provides a light chain polypeptide primarily forms an interchain disulfide bond with amino acids outside the hinge region of the heavy chain polypeptide through the most C-terminal cysteine residue of the light chain polypeptide. In certain aspects, the most C-terminal cysteine residue of the light chain polypeptide is the cysteine residue at Eu position 214 of the light chain polypeptide. In certain aspects, the amino acid outside the hinge region is a cysteine residue at Eu position 131 of the heavy chain polypeptide. In certain aspects, the heavy chain polypeptide modification comprises a mutation of a cysteine reside within the hinge region. The mutated cysteine residue can be, for example at Eu position 219 or 220 of the heavy chain polypeptide. In certain aspects, the most C-terminal cysteine residue of the light chain polypeptide is the cysteine residue at Eu position 214 of the light chain polypeptide.

The hinge region comprises Eu amino acids 200 to 238 of the heavy chain polypeptide. In certain aspects, the light chain polypeptide always forms an interchain disulfide bond only with amino acids outside the hinge region of the heavy chain polypeptide through the most C-terminal cysteine residue of the light chain polypeptide.

Further described is a therapeutic antibody formulation, comprising: a plurality of IgG2 antibodies that bind a therapeutic target of interest, wherein the formulation primarily comprises a single conformational isoform of the antibodies and wherein the antibodies comprise at least one amino acid modification; and a pharmaceutically acceptable carrier. In certain aspects, the modification comprises a heavy chain polypeptide modification of the antibodies. In certain aspects, the heavy chain polypeptide modification comprises a substitution of a cysteine residue at Eu position 131 of the heavy chain polypeptide.

In certain aspects, the heavy chain polypeptide modification comprises a mutation of a cysteine reside within the hinge region. In certain aspects, the mutation of a cysteine residue comprises a mutation of a cysteine residue at Eu position 219 of the heavy chain polypeptide.

In certain aspects, the mutation of a cysteine residue comprises a mutation of a cysteine residue at Eu position 220 of the heavy chain polypeptide.

The invention provides a method of making a modified IgG2 anti-EGFR antibody comprising: modifying a nucleotide sequence encoding the heavy or light chain polypeptide of an IgG2 anti-EGFR antibody such that at least one encoded amino acid is modified; introducing said nucleic acid into a host cell; and culturing said host cell such that a plurality of modified IgG2 anti-EGFR antibodies is expressed and secreted, wherein a substantial population that is at least 50% of the total population of said plurality of modified IgG2 antibodies are a single conformational isoform, wherein said modification comprises a heavy chain polypeptide modification of said antibody, and wherein said heavy chain polypeptide modification comprises a substitution of the cysteine residue at Eu position 131 of said heavy chain polypeptide with serine (C131S), and wherein said heavy chain polypeptide modification further comprises a mutation of a cysteine residue within the hinge region, and wherein said mutation of a cysteine residue within said hinge region of said heavy chain polypeptide comprises a mutation of the cysteine residue at Eu position 219 or 220 of said heavy chain polypeptide to serine (C219S or C220S), and wherein the heavy chain of said modified IgG2 anti-EGFR antibody comprises the variable region SEQ ID NO: 57.

Also provided herein are nucleic acid molecules that encode the monoclonal IgG2 antibodies described above, vectors that comprise the nucleic acid molecules, and host cells that comprise the vectors. In certain aspects, the host cells can be selected from the group consisting of CHO, VERO, NSO, BK, HeLa, CV1, Cos, MDCK, 293, 3T3, PC12 and WI38 cells. Also provided herein is a hybridoma cell expressing the IgG2 antibodies described above.

Described, but not part of the invetion, is an anti-RANKL antibody with increased structural homogeneity. For example, antibodies against RANKL are described in WO 03/002713, and exemplified by heavy chain comprising the variable region amino acid sequence set forth herein as SEQ ID NO: 60 and light chain comprising the variable region amino acid sequence set forth herein as SEQ ID NO: 61. Accordingly, modifications can be made to an anti-RANKL antibody according to the present invention, such that the light chain polypeptide primarily forms an interchain disulfide bond only with amino acids in the hinge region through the most C-terminal cysteine residue of the light chain. Such modifications can include substitution or deletion of a cysteine residue at Eu position 131 of the heavy chain polypeptide of the anti-RANKL antibody. Other modifications can be made to an anti-RANKL antibody such that the light chain primarily forms an interchain disulfide bond only with amino acids outside the hinge region through the most C-terminal cysteine residue of the light chain. Such modifications can include substitution or deletion of a cysteine residue in the hinge region of the anti-RANKL antibody. Exemplary substitutions or deletions may be at Eu position 219 or 220 of the heavy chain polypeptide of the anti-RANKL antibody. Further modifications to anti-RANKL antibodies contemplated herein include insertions of one or more amino acids in the hinge region of the heavy chain polypeptide or insertions of one or more amino acids in the C-terminal region of the light chain polypeptide.

Also provided herein is an anti-EGFR antibody with increased structural homogeneity. For example, antibodies against EGFR are described in U.S. Patent No. 6,235,883, and exemplified by heavy chain comprising the variable region amino acid sequence set forth herein as SEQ ID NO: 57 and light chain comprising the variable region amino acid sequence set forth herein as SEQ ID NO: 49. Other embodiments include an antibody comprising a heavy chain variable region amino acid sequence set forth herein as any one of SEQ ID NOs: 34, 36, 38, 40, 42, 44, 46, 48 and 50-57, and comprising a light chain variable region amino acid sequence set forth herein as any one of SEQ ID NOs: 35, 37, 39, 41, 43, 45, 47 or 49. Accordingly, modifications can be made to an anti-EGFR antibody according to the present invention, such that the light chain polypeptide primarily forms an interchain disulfide bond only with amino acids in the hinge region through the most C-terminal cysteine residue of the light chain. Such modifications can include substitution or deletion of a cysteine residue at Eu position 131 of the heavy chain polypeptide of the anti-EGFR antibody. Other modifications can be made to an anti-EGFR antibody such that the light chain primarily forms an interchain disulfide bond only with amino acids outside the hinge region through the most C-terminal cysteine residue of the light chain. Such modifications can include substitution of a cysteine residue in the hinge region of the anti-EGFR antibody. Substitutions are at Eu position 219 or 220 of the heavy chain polypeptide of the anti-EGFR antibody.

Described, but not part of invention is an anti-IL-1R antibody with increased structural homogeneity. In one particular embodiment, the antibody is an anti-IL-1R type 1 antibody. For example, antibodies against IL-1R are described in U.S. Patent Pub. No. 2004/097712, and exemplified by heavy chain comprising the variable region amino acid sequence set forth herein as any one of SEQ ID NOs: 62, 64, 65, 78, 80 or 81 and light chain comprising the variable region amino acid sequence set forth herein as any one of SEQ ID NOs: 63, 66, 79 or 82. Other embodiments include an antibody comprising a heavy chain amino acid sequence set forth herein as any one of SEQ ID NOs: 67-75, and comprising a light chain amino acid sequence set forth herein as any one of SEQ ID NOs: 76-77. Accordingly, modifications can be made to an anti-IL-1R antibody according to the present invention, such that the light chain polypeptide primarily forms an interchain disulfide bond only with amino acids in the hinge region through the most C-terminal cysteine residue of the light chain. Such modifications can include substitution or deletion of a cysteine residue at Eu position 131 of the heavy chain polypeptide of the anti-IL-1R antibody. Other modifications can be made to an anti- IL-1R antibody such that the light chain primarily forms an interchain disulfide bond only with amino acids outside the hinge region through the most C-terminal cysteine residue of the light chain. Such modifications can include substitution or deletion of a cysteine residue in the hinge region of the anti-IL-1R antibody. Exemplary substitutions or deletions may be at Eu position 219 or 220 of the heavy chain polypeptide of the anti-IL-1R antibody. Further modifications to anti-IL-1R antibodies contemplated herein include insertions of one or more amino acids in the hinge region of the heavy chain polypeptide or insertions of one or more amino acids in the C-terminal region of the light chain polypeptide.

Described, but not part of the invention is a method of increasing the formulated and *in vivo* stability of a modified IgG2 antibody comprising: modifying a nucleotide sequence encoding the heavy or light chain polypeptide of an IgG2 antibody such that at least one encoded amino acid is modified; introducing the nucleic acid into a host cell; and culturing the host cell such that a plurality of modified IgG2 antibodies is expressed and secreted, wherein the plurality of modified IgG2 antibodies are primarily a single conformational isoform.

Described, but not part of the invetion is a method of increasing the potency of a modified IgG2 therapeutic antibody comprising: modifying a nucleotide sequence encoding the heavy or light chain polypeptide of an IgG2 antibody such that at least one encoded amino acid is modified; introducing the nucleic acid into a host cell; and culturing the host cell such that a plurality of modified IgG2 antibodies is expressed and secreted, wherein the plurality of modified IgG2 antibodies are primarily a single conformational isoform.

Other features and advantages of the invention will become apparent from the following detailed description.

### BRIEF DESCRIPTION OF THE DRAWINGS

The following drawings form part of the present specification and are included to further illustrate aspects of the present invention. The invention may be better understood by reference to the drawings in combination with the detailed description of the specific embodiments presented herein.
Figure 1 is a schematic representation of the human IgG subtypes indicating disulfide connections. Shown are IgG1 (Figure 1A), IgG2 (Figure 1B), IgG3 (Figure IC) and IgG4 (Figure 1D). IgG1 is the only isotype where the light chain was proposed to connect via the first Cys residue in the heavy chain hinge region
Figure 2 is a schematic representation of four structural isoforms of anti-RANKL IgG2 antibody. The forms are referred to as structural isoforms IgG2-A, (Figure 2A), IgG2-B1 (Figure 2B), IgG2-B2 (Figure 2C) and IgG2-A/B (Figure 2D).
Figure 3 is a schematic representation of the IgG1 and IgG2 subtypes, setting forth the Edelman or Eu numbering convention of IgG antibodies. Light chain (LC) is represented by a dashed line. The Eu number of particular HC hinge region and LC amino acids is indicated. The IgG2 hinge region sequence is set forth as SEQ ID NO: 5 and the IgGI hinge region sequence is set forth as SEQ ID NO: 6. Variable disulfide bond interactions are indicated by dotted lines.
Figure 4 is a schematic representation of different IgG2 cysteine mutants. Shown are a wild type (WT) IgG2 antibody, and Cys219Ser (C219S), Cys220Ser (C220S) and Cys 131 Ser (C131S) heavy chain mutants. The hinge region sequence of each antibody is set forth as SEQ ID NO: 7 (WT and C131S), SEQ ID NO: 8 (C219S), and SEQ ID NO: 9 (C220S). Light chain (LC) is represented by a dashed line, and variable disulfide bond interactions are indicated by dotted lines.
Figure 5 is a schematic representation of IgG2 antibodies with either λ- or κ- light chains. The light chains are indicated by a dashed line. The hinge region sequence of each antibody is set forth as SEQ ID NO: 7. Variable disulfide bond interactions are indicated by dotted lines.
Figure 6 is an IgG2 three dimensional model illustrating the C_{H}1 and hinge region of a single IgG2 heavy chain. Also shown is the LC Cys residue that disulfide bonds with the heavy chain from the published structure of an IgG1. To illustrate one possible orientation of the LC Cys214 residue in human IgG2, the position of the LC Cys214 residue of a human IgG4 Fab is shown. The C_{H}1 Cys that disulfide bonds to the LC Cys214 in human IgG4 aligns with the position of Cys 1 31 in human IgG2.
Figure 7 is a schematic representation of IgG2 cysteine mutants made to the anti-RANKL antibody. Shown are a wild type (WT) IgG2 antibody, and Cys131Ser (C131S) mutants. The hinge region sequence of each antibody is set forth as SEQ ID NO: 7. Variable disulfide bond interactions are indicated by dotted lines.
Figure 8 is a schematic representation of a wild type and a Cys131Ser mutation. Mutation of Cys 131 to Ser 131 prevents the inter heavy-light disulfide bond found in structural isoform IgG2-A.
Figure 9 shows SE-HPLC analysis of anti-RANKL (solid line) and anti-RANKL (Cys131Ser) mutant (dotted line).
Figure 10 shows CEX-HPLC analysis at pH 7.5 of anti-RANKL (bottom trace) and anti-RANKL mutant (top trace).
Figure 11 shows CEX-HPLC analysis at pH 5 of anti-RANKL (bottom trace) and anti-RANKL mutant (top trace).
Figure 12 shows non-reduced CE-SDS analysis of anti-RANKL (bottom trace) and anti-RANKL mutant (top trace).
Figure 13 shows reduced CE-SDS analysis of anti-RANKL (bottom trace) and anti-RANKL mutant (top trace).
Figure 14 shows analysis of anti-RANKL (Cys131Ser) mutant and anti-RANKL wild-type by non-reduced SDS-PAGE. The samples in each lane are indicated.
Figure 15 shows analysis of anti-RANKL (Cys131Ser) mutant and anti-RANKL wild-type by reduced SDS-PAGE. The samples in each lane are indicated.
Figure 16 shows RP-HPLC analysis of anti-RANKL (top trace) and anti-RANKL (Cys131Ser) mutant (bottom trace).
Figure 17 shows non-reduced Lys-C peptide mapping of anti-RANKL (top trace) and anti-RANKL (Cys131Ser) mutant (bottom trace).
Figure 18 shows reduced Lys-C peptide mapping of anti-RANKL (top trace) and anti-RANKL mutant (bottom trace).
Figure 19 is a schematic representation of IgG2 cysteine mutants made to the anti-IL-1R antibody. Shown in Figure 19A are a wild type (WT) IgG2 antibody, Cys219Ser (C219S) and Cys131Ser (C131S) mutants. Figure 19B sets forth disulfide connectivity according to peptide mapping analysis. The hinge region sequence of each antibody is set forth as SEQ ID NO: 7 (WT and C131S) and SEQ ID NO: 8 (C219S). Light chain (LC) is represented by a dashed line, and variable disulfide bond interactions are indicated by dotted lines.
Figure 20 shows RP-HPLC analysis of anti-IL-1R wild type and produced mutants. Wild type anti-IL-1R produced from stably transfected (Ref. Std) and transiently transfected CHO cells are indicated. Anti-IL-lR C219S and C131S mutants are indicated.
Figure 21 is a graph showing the results of an IL-6 production inhibition assay. Percentage of control production of IL-6 is plotted as a function of log antibody concentration in pM. Results for anti-IL-1R wild type and C131S and C219S mutants are shown, and the calculated IC50 values are presented in the table above the graph.
Figure 22 is a schematic representation of IgG2 cysteine mutants made to the anti-EGFr antibody. Shown are a wild type (WT) IgG2 antibody, Cys219Ser (C219S), Cys220Ser (C220S) mutants and C219S/C220S double mutant. The hinge region sequence of each antibody is set forth as SEQ ID NO: 7 (WT and C131S), SEQ ID NO: 8 (C219S), SEQ ID NO: 9 (C220S), and SEQ ID NO: 10 (C219S/C220S). Light chain (LC) is represented by a dashed line, and variable disulfide bond interactions are indicated by dotted lines.

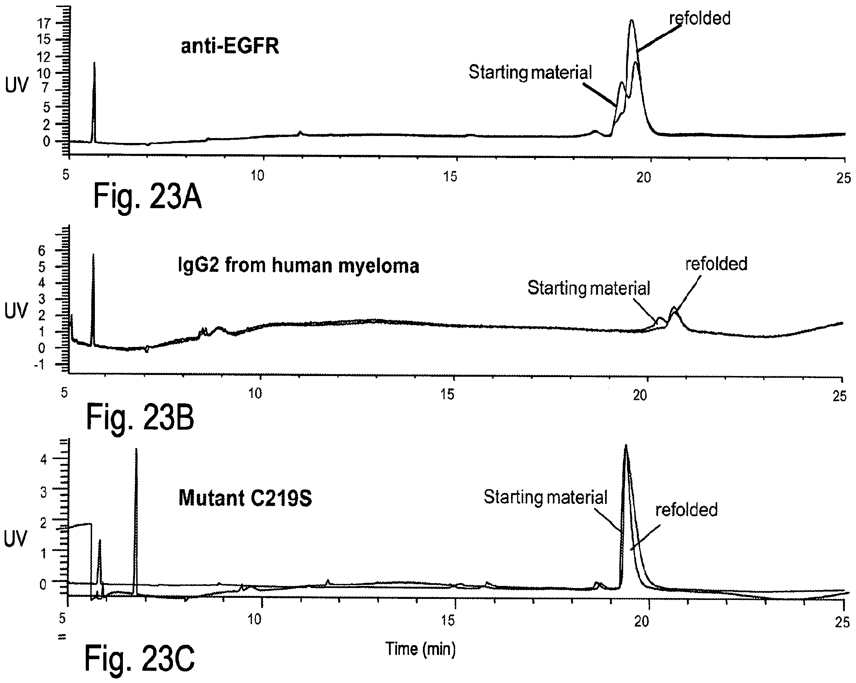

Figure 23 shows Separation of IgG2 molecules by non-reduced CE-SDS. (A) anti-EGFr and (B) commercially available human IgG2 isolated from myeloma patient exhibit the doublet typical of structural isoforms (indicated as "starting material"). Application of cysteine / cystine redox potential leads to re-arrangement of disulfide bridges, resulting in decrease of isoform 1 and increase of isoform 2 (indicated as "refolded"). (C) Single cysteine mutants C219S (shown in figure) and C220S (data not shown) are resolved in a single peak expected of a homogeneous structure. The refolding treatment does not affect the mutants.

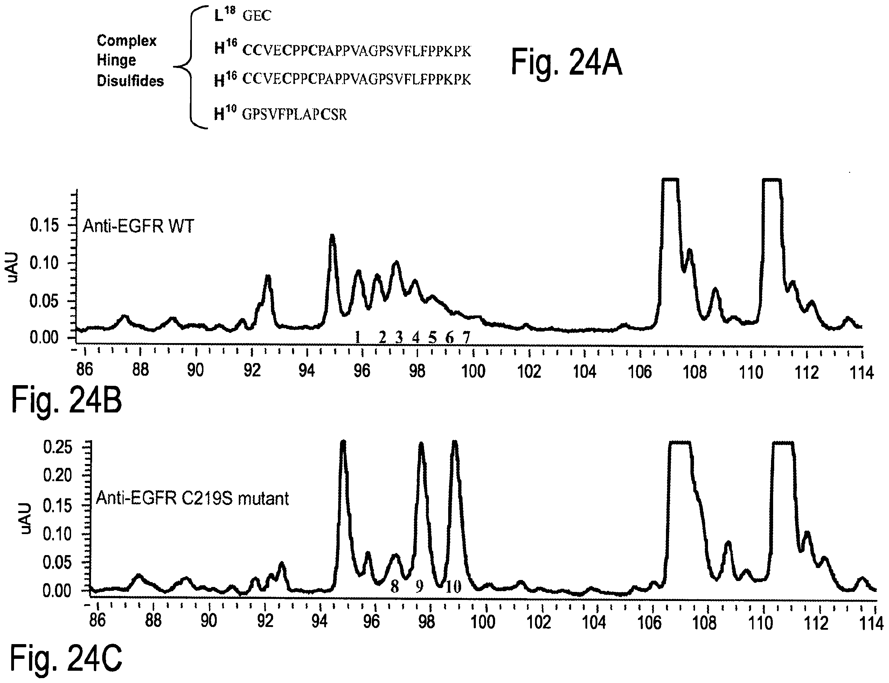

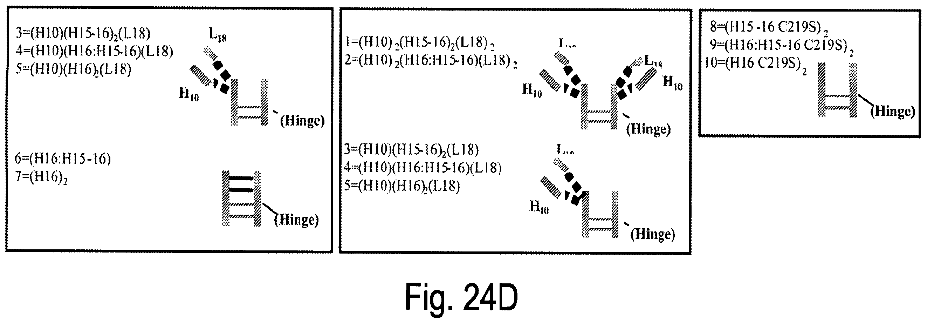

Figure 24 shows LC-MS analysis of anti-EGFr disulfide-linked heteropeptides. (A) Sequences of the 4 tryptic peptides involved in the complex hinge disulfide-linked peptides. In this complex the tryptic peptide representing the hinge region, H₁₆ (SEQ ID NO: 11), is found covalently bound to the tryptic peptides H₁₀ (SEQ ID NO: 12) and L₁₈, normally involved in the inter-chain connection C_{H}1-C_{L}. (B) Trypsin peptide mapping of anti-EGFr reference material. Different arrangements of the 4 peptides, labeled 1-7, are separated by LC-MS. (C) Anti-EGFr which has been mutated at C219 and/or C220 does not show these hinge complex disulfide linked peptides, but rather exhibits full recovery of the expected linearly paired hinge region. These peptides, labeled 8-10, are 219/220 C-S hinge dimers, different by one residue from the anti-EGFr hinge dimers 6,7. (D) Schematic representation of the hinge complex disulfide linked peptides for these IgG2 molecules.
Figure 25 shows insertion mutations of IgG2 in the hinge region, as set forth in SEQ ID NOs: 13-22.
Figure 26 is a schematic representation of insertion mutants of sequences 2-4 (SEQ ID NOs: 14-16) made to anti-IL-1R antibody.
Figure 27 is a schematic representation of insertion mutants of sequences 9-10 (SEQ ID NOs: 21-22) made to anti-IL-1R antibody.
Figure 28 is a schematic representation of insertion mutants of sequences 5-8 (SEQ ID NOs: 17-20) made to anti-IL-1R antibody.
Figure 29 shows RP-HPLC of a control anti-IL-1R antibody and anti-IL-1R samples with redox enriched forms 1 and 3. The forms were enriched in a redox buffer without and with 0.9M GuHCl, respectively.
Figure 30 shows representative data from one experiment showing the inhibition of IL-1b-induced IL-6 production in human chondrocytes by the anti IL-1R1 IgG2 control antibody (black squares), redox enriched form 1 (up triangles), and redox enriched form 3 (down triangles). The inhibition of IL-6 is plotted as a function of concentration of each antibody sample.
Figures 31 A-B show IC50 values for the inhibition of IL-1b-induced IL-6 in chondrocytes (A) and whole blood assays (B) by the control antibody (diamonds), redox enriched form 1 (up triangles), and redox enriched form 3 (down triangles) (n=6). The black bars represent the means. *P>0.05; **P<0.05; ***P<0.01; ****P<0.001.
Figures 32 A-C show reversed phase HPLC analysis of unpurified IgG2 samples from cell culture media. Shown are the results for the wild type (WT) sample (A), the cAc insertion construct (B) (hinge region set forth as SEQ ID NO: 32), and the cPPc construct (C) (hinge region set forth as SEQ ID NO: 33).
Figure 33 is a schematic representation of IgG2 antibody variants with κ-light chains. The light chains are indicated by a dashed line. Sequences 11-13 (SEQ ID NOs: 23-25) are shown. The hinge region sequence of each antibody is set forth as SEQ ID NO: 7.
Figure 34 is a schematic representation of IgG2 antibody variants with λ-light chains. The light chains are indicated by a dashed line. Sequences 14-16 (SEQ ID NOs: 26-28) are shown. The hinge region sequence of each antibody is set forth as SEQ ID NO: 7.
Figure 35 is a schematic representation of IgG2 antibody variants with λ-light chains. The light chains are indicated by a dashed line. Sequences 17-19 (SEQ ID NOs: 29-31) are shown. The hinge region sequence of each antibody is set forth as SEQ ID NO: 7.

### DETAILED DESCRIPTION

The invention is defined in the appended claims. Structural heterogeneity of antibodies in the IgG2 subclass has been observed, although the reasons underlying this heterogeneity have remained unexplained. For example, U.S. patent application Pub. No: 2006/194280, Dillon et al., is directed to methods of transiently enriching particular IgG isoforms by subjecting preparations of recombinant IgG proteins with a reduction/oxidation coupling reagent and optionally a chaotropic agent. However, although refolding methods can yield enriched populations of IgG isoforms, the effect is transient, and the antibodies can revert to a more heterogeneous population of isoforms over time. Thus, embodiments of the invention described herein provide methods of producing homogenous antibody populations that are stable *in vivo* and remain homogenous over time.

Accordingly, embodiments of the invention relate to structurally homogenous recombinant proteins and methods of producing such structurally homogeneous recombinant proteins. In one embodiment, the recombinant proteins are recombinant antibodies. In one embodiment the recombinant proteins are immunoglobulin proteins. In another embodiment, the recombinant proteins are antibodies of the IgG2 isotype. Solutions containing structurally homogenous recombinant proteins improved activity *in vivo* or *in vitro* in comparison to solutions having heterogeneous recombinant proteins.

In accordance with the above, described herein is the characterization of distinct structural isoforms of IgG2 antibodies. Embodiments of the invention include modified antibodies having an IgG2 structure that differs from the conventionally accepted immunoglobulin structural model. For example, the various structural isoforms produced by the methods described herein were found to selectively eliminate some of the inter-chain disulfide bridges, in comparison to wild-type isoforms.

Thus, embodiments of the invention provide efficient and economic production, purification and analysis of homogeneous populations of as defined in the claims, a single desired conformational isoform of an antibody is produced. More particularly, embodiments include methods of making homogeneous populations of antibodies, wherein the population comprises a single lgG2 disulfide form, in contrast to wild-type populations of IgG2 antibodies which exist in multiple disulfide forms. In one embodiment, the amino acid sequences of these antibodies are modified to only fold into a predetermined form. In another embodiment, the antibodies are modified to provide improved pharmaceutical properties. As described in further detail below, the substitution of amino acid residues in the IgG2 molecule were found to facilitate the elimination of disulfide heterogeneity and thus produce structurally homogeneous, more active single conformational isoforms of IgG2 antibodies. Besides having equivalent or improved potency compared to heterogeneous antibody populations, structurally homogeneous populations of antibodies have the added advantage of simplified manufacture and processing.

Described herein is a monoclonal IgG2 antibody, comprising a light chain polypeptide and a heavy chain polypeptide having a hinge region, wherein the antibody comprises an amino acid modification in the heavy or light chain polypeptide such that the light chain polypeptide primarily forms an interchain disulfide bond with amino acids in the hinge region of the heavy chain polypeptide through a C-terminal cysteine residue at Eu position 214 of the light chain polypeptide. In certain aspects, the modification comprises a heavy chain polypeptide modification. In certain aspects, the modification can comprise a substitution or deletion of a cysteine residue at Eu position 131 of the heavy chain polypeptide. In certain aspects the hinge region comprises Eu amino acids 200 to 238 of the heavy chain polypeptide. In certain aspects, the light chain polypeptide always forms an interchain disulfide bond only with amino acids in the hinge region of the heavy chain polypeptide through the most C-terminal cysteine residue of the light chain polypeptide.

As used herein, the term primarily forms, primarily only forms, and like terms refer to antibodies that, when detected using standard methods, a substantial population exists in a single conformational isoform. Typically, a substantial population is at least 50%, at least 60%, at least 70%, at least 80%, at least 90% and more typically at least 91%, 92%, 93%, 94%, 95%, 96%, 97%, 98% or 99% of the total population.

As will be appreciated by those of skill in the art, an interchain disulfide bond is a disulfide bond between two cysteines on different antibody chains. Typically, an interchain disulfide bond is between a heavy chain cysteine and a light chain cysteine or between two cysteines on different heavy chains. An intrachain disulfide bond is a disulfide bond between two cysteines on the same chain. Typically, an intrachain disulfide bond is between a heavy chain cysteine and a heavy chain cysteine, or a disulfide bond between a light chain cysteine and a light chain cysteine on the same light chain.

Further described herein is a monoclonal IgG2 antibody, having a light chain polypeptide and a heavy chain polypeptide having a hinge region, wherein the antibody has an amino acid modification in the heavy or light chain polypeptide such that the light chain polypeptide primarily forms an interchain disulfide bond with amino acids outside the hinge region of the heavy chain polypeptide through a C-terminal cysteine residue at Eu position 214 of the light chain polypeptide. In certain aspects, the amino acid outside the hinge region is a cysteine residue at Eu position 131 of the heavy chain polypeptide. In certain aspects, the heavy chain polypeptide modification comprises a mutation of a cysteine reside within the hinge region. The mutated cysteine residue can be, for example at Eu position 219 or 220 of the heavy chain polypeptide. In some aspects, the hinge region comprises Eu amino acids 200 to 238 of the heavy chain polypeptide.

In certain aspects, the light chain polypeptide always forms an interchain disulfide bond only with amino acids outside the hinge region of the heavy chain polypeptide through the most C-terminal cysteine residue of the light chain polypeptide.

In another embodiment, IgG2 antibodies are modified to provide improved pharmaceutical properties. Pharmaceutical properties include properties such as solubility, metabolism and absorption, as well as storage stability. Accordingly, IgG2 antibodies formulated for pharmaceutical use which are modified according to the present invention possess surprisingly improved shelf-life.

Described but not part of the invetion is a therapeutic antibody formulation, comprising a plurality of IgG2 antibodies that bind a therapeutic target of interest, and a pharmaceutically acceptable carrier wherein the formulation primarily comprises a single conformational isoform of the antibodies and wherein the antibodies comprise at least one amino acid modification. In certain embodiments, the modification comprises a heavy chain polypeptide modification of the antibodies. The heavy chain polypeptide modification can include, for example, a substitution or deletion of a cysteine residue at Eu position 131 of the heavy chain polypeptide. In certain aspects, the heavy chain polypeptide modification comprises a mutation of a cysteine reside within the hinge region. The mutation can be, for example, a mutation of a cysteine residue at Eu position 219 or at Eu position 220 of the heavy chain polypeptide.

### Structure of IgG2 antibodies

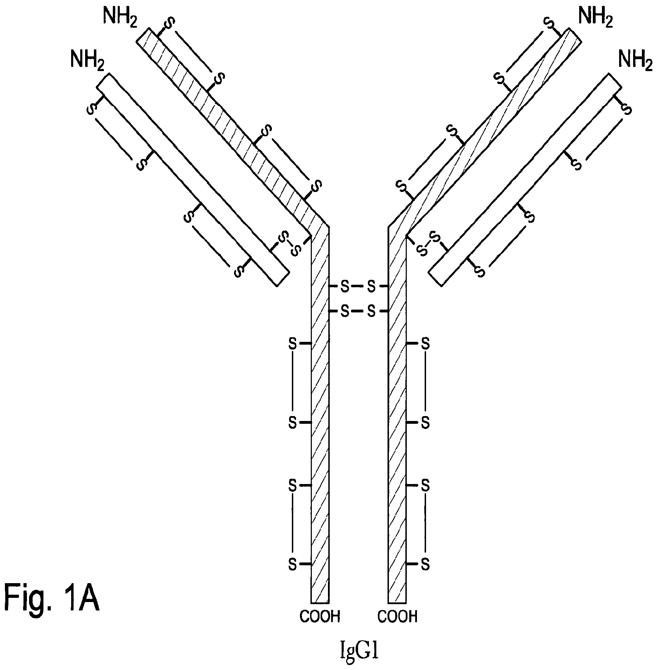

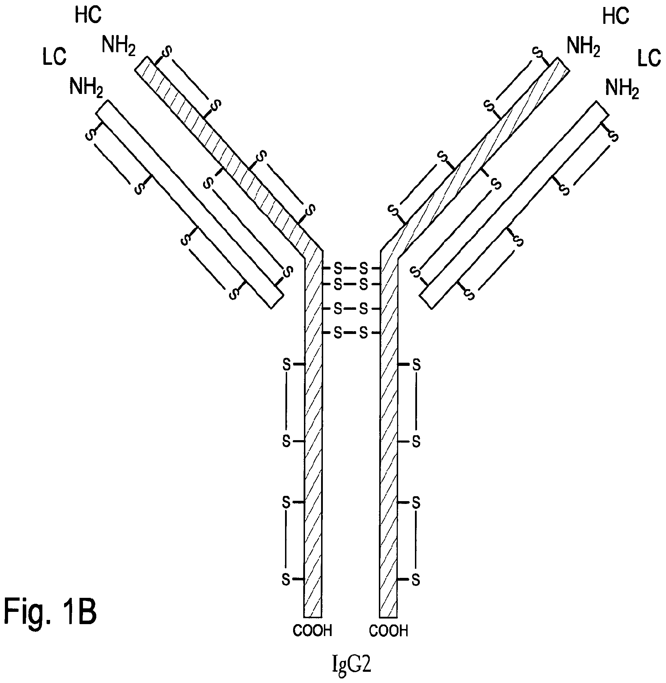

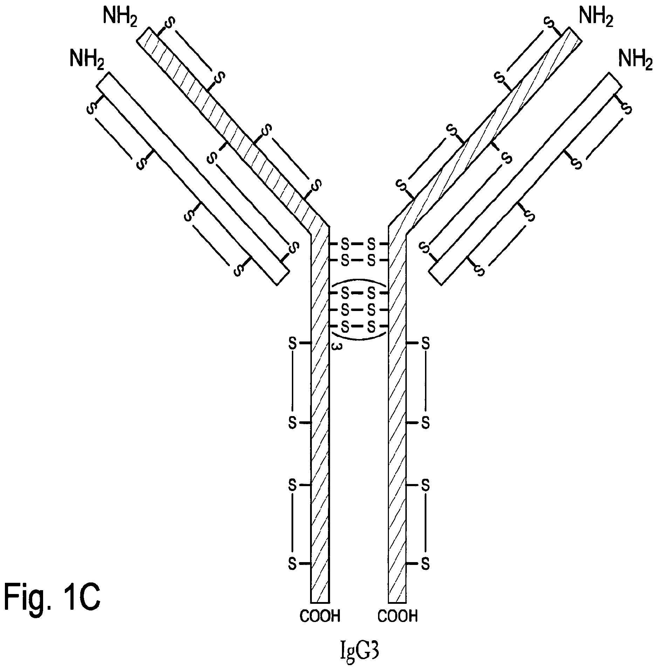

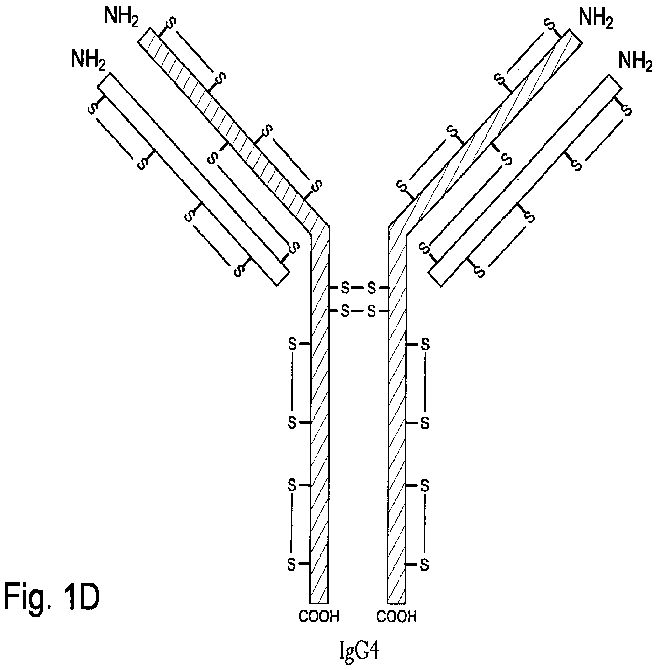

As indicated in Figure 1, IgG subtypes have predicted disulfide bond structures, particularly in their inter-heavy chain bonds and heavy chain/light chain bonds (Frangione, B. et al (1969) Nature 221, 145-148). Many differences between the several illustrated IgG isotypes can be seen in the hinge region. Table 1 illustrates the differences among IgG isotypes within a subset of the hinge residues, termed the core hinge sequence. As shown in Table 1, the IgG2 core hinge is three amino acids shorter than the core hinge of IgG1 (Dangl, J. L. et al (1988) EMBO J. 7, 1989-1994). Additionally, the IgG2 core hinge has one more cysteine than IgG 1.

**Table 1. Amino acid sequence of the core hinge of the human IgG subtypes.**

| **IgG subtype** | **Core hinge sequence** | **SEQ ID NO:** |
|---|---|---|
| IgG1 | EPKSCDKTHTCPPCP | 1 |
| IgG2 | ERKCCVECPPCP | 2 |
| IgG3 | ELKTPLDTTHTCPRCP (EPKSCDTPPPCPRCP)₃ | 3 |
| IgG4 | ESKYGPPCPSCP | 4 |

The hinge region has been defined in various ways (Padlan, E. A. (1994) Mol. Immun. 31, 169-217; Dangl, et al. (1988) EMBO J. 7, 1989-1994, As used herein, the IgG2 hinge region includes the amino acid residues from Cys200 to Pro238, using the Eu numbering convention (Edelman, G.M. et al. (1969) Natl. Acad. Sci. U. S. A. 63:78-85, Thus, a modification to the IgG2 hinge region can include any modification to the portion of an IgG2 antibody that includes Eu positions 200 to 238.

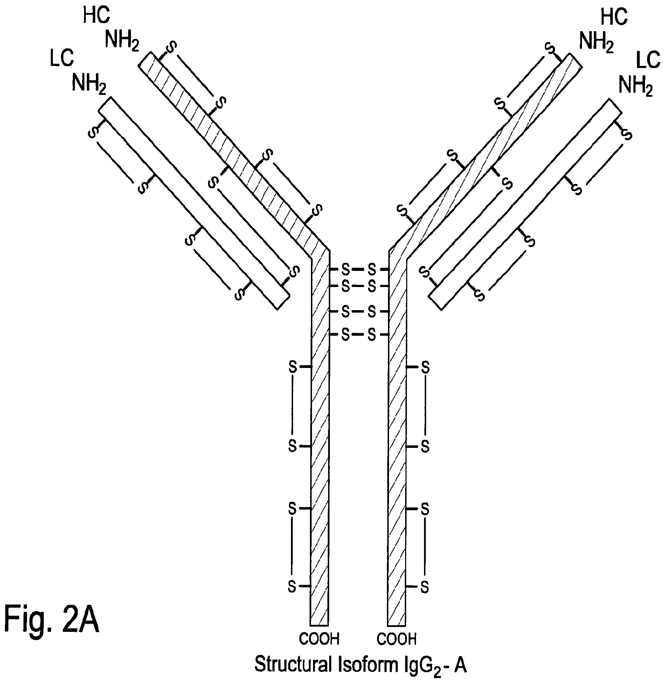

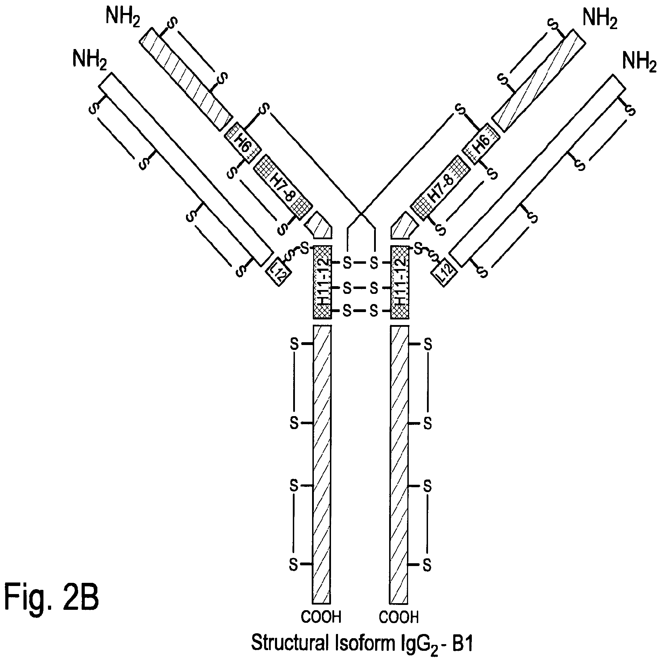

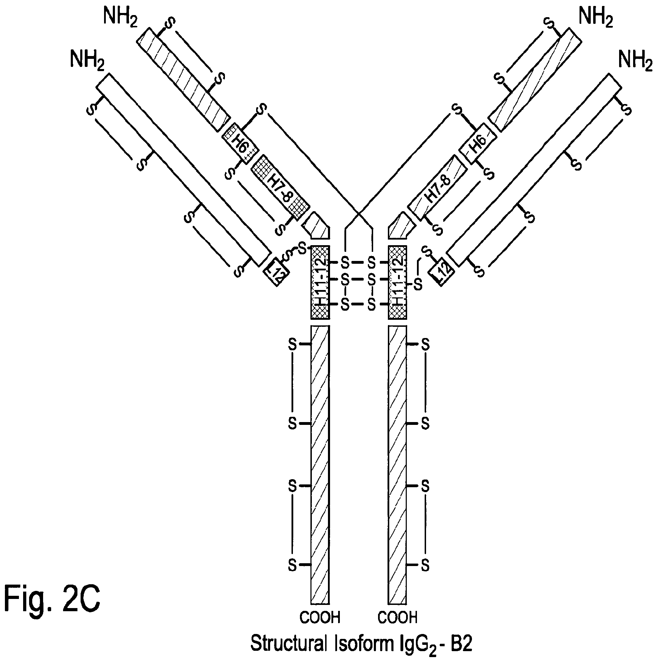

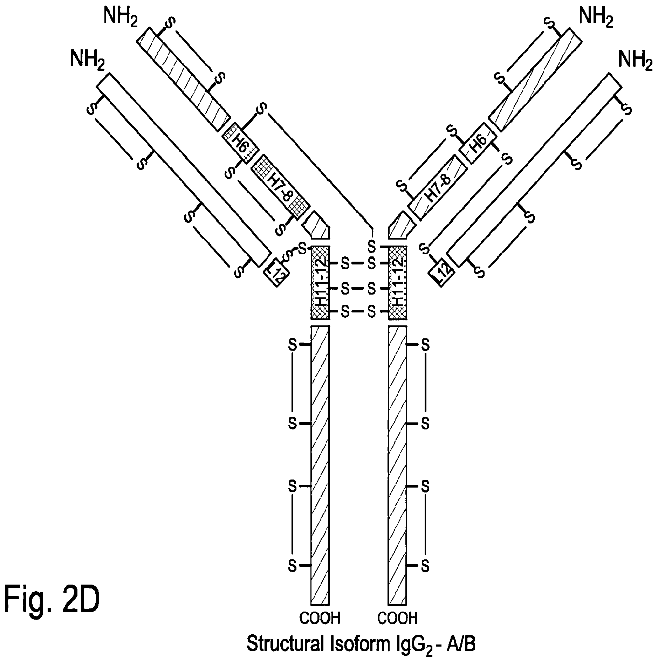

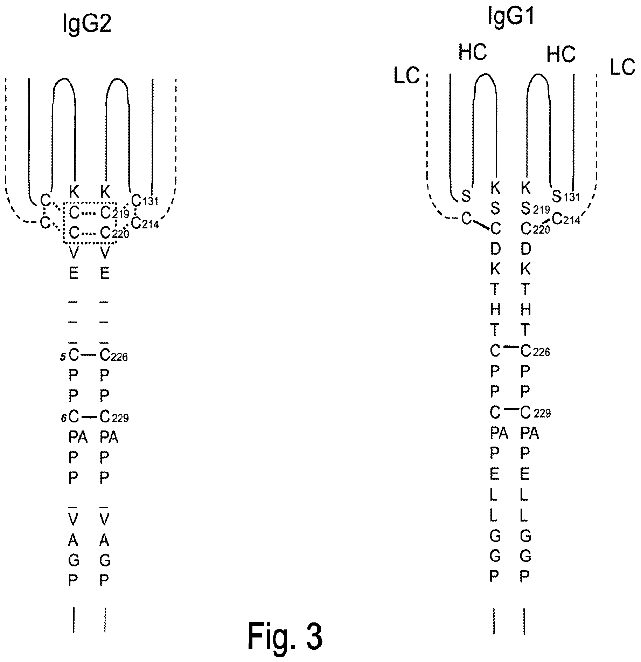

Provided herein is the discovery that disulfide heterogeneity exists within the IgG2 subtype, as shown in Figure 2. Figure 3 highlights the difference between IgG2 and IgG1 with regard to possible disulfide bonding patterns. According to Eu numbering, light chain (LC) is attached to Cys220 of heavy chain (HC) in IgG1. In contrast, the LC Cys214 in IgG2 antibodies is predicted to be in close proximity to three or more potential disulfide bonding partners on the HC: C131, C219 and C220 (Figure 3). Accordingly, as provided in the examples herein, is the discovery that IgG2 antibodies exist in multiple conformational isoforms based on different disulfide bonds in their hinge region. Thus, IgG2 LC 214 is shown to form disulfide bonds with HC C131, C219 and C220, resulting in distinct conformational isoforms.

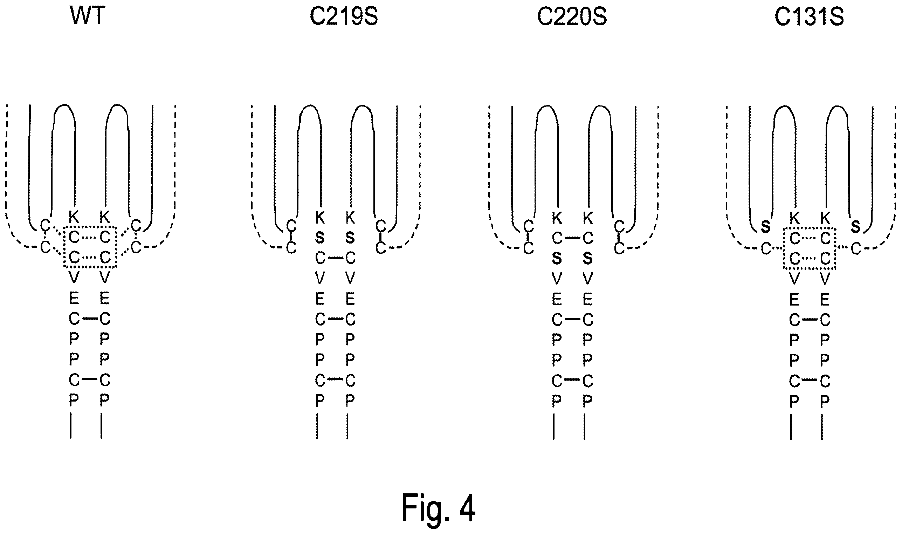

Also provided herein is the surprising discovery that mutations to the cysteines involved in HC-HC and HC-LC disulfide bonds leads to homogenous IgG2 conformational isoforms. Mutations to HC cysteines led to homogeneous populations of antibodies with distinct disulfide connectivities and conformational properties. In particular, as shown in Figure 4, mutations of Cys219 to serine, Cys220 to serine, and Cys131 to serine led to homogenous IgG2 populations. In some embodiments, the mutant antibodies retained the same or greater potency as the wild-type antibody.

Further described herein, but not claimed, are IgG2 antibodies comprising insertion mutations such that the disulfide bonds created between the heavy chain hinge region and the light chain is disrupted. As set forth in Example 11, 1, 2, 3, 4, 5 or more amino acids are inserted into the heavy chain (HC) hinge region polypeptide. Typically, the point of insertion is between C219 and C220, or between K218 and C219 of the HC hinge region. Other insertions can alter the disulfide bonds within the antibody and lead to homogenous populations of antibodies.

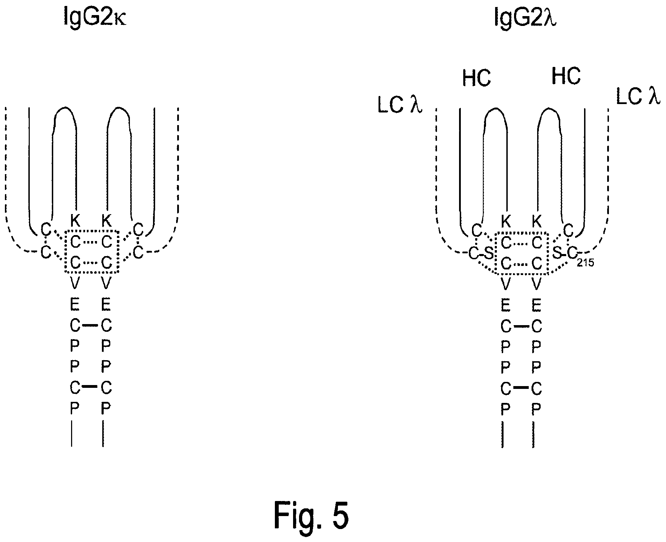

Although the examples of mutations presented herein were performed for IgG2κ, the same mutation can be performed for IgGλ antibodies as well. IgG2X antibodies have λ-light chains instead of κ-light chains. For example, λ light chains have serine S215 after cysteine C214 at the C-terminus (see Figure 5).

### Modifications to Antibody Amino Acid Sequence

The invention is defined in appended claims. One embodiment of the invention includes IgG2 antibodies that are modified to reduce structural and disulfide heterogeneity. Amino acid sequence variants of the antibody are prepared by introducing appropriate nucleotide changes into the polynucleotide encoding the antibody. Alternatively, the amino acid changes can be made during peptide synthesis of the antibodies. Such modifications include substitutions of residues within the polypeptide sequences of the antibody. The amino acid alterations may be introduced in the subject antibody amino acid sequence at the time that sequence is made.

Cysteines normally involved in disulfide linkage formation can be rendered incapable of forming disulfide linkages by any of a variety of methods known in the art. For example, a hinge cysteine can be substituted with another amino acid, such as serine, which is not capable of disulfide bonding. Typically, serine is selected based on its structural similarity to cysteine. The Cys→Ser substitution has the net impact of changing a single cysteine side chain Sulfur atom to Oxygen. Other amino acid substitutions that remove the disulfide bonding potential of a cysteine residue can be envisioned. For example Cys→Ala, Cys→Thr, or Cys→Val substitutions could be constructed. Residues for substitution could also be identified by examination of amino acid substitution matrices such as BLOSUM62 (S. Henikoff, J. G. Henikoff (1992) Proc. Natl. Acad. Sci. USA 89:10915). These substitutions remove the disulfide bonding potential of the cysteine residue of interest. It should be noted, however, that the interpretation of the test results with these mutants needs to take into account the additional structural differences between these substituted residues and the original cysteine. Data obtained with these substitutions, and specifically, data suggesting an alteration in function, could be the result of removing the disulfide bonding potential but could also be (fully or partly) the result of changes in solvent interactions or polypeptide folding. Alternatively a series of mutants could be constructed in which the cysteine residue of interest is mutated to all of the remaining naturally occurring amino acids despite the time-consuming and tedious nature of assessing the specific impact of the amino acid side chains that deviate from that of cysteine.

Amino acid substitution can be achieved by well-known molecular biology techniques, such as site directed mutagenesis of the nucleic acid sequence encoding the hinge region that is to be modified. Suitable techniques include those described in Sambrook et al. MOLECULAR CLONING: A LABORATORY MANUAL, 3rd ed., Cold Spring Harbor Laboratory Press, Cold Spring Harbor, N.Y., (2001) and Ausubel et al., 2006, Current Protocols in Molecular Biology, Greene Publishing Associates and Wiley Interscience, N.Y. Other techniques for generating immunoglobulins with a variant hinge region include synthesizing an oligonucleotide having a sequence that encodes a hinge region in which the codon that encodes the cysteine that is to be substituted is replaced with a codon that encodes the substitute amino acid. This oligonucleotide can then be ligated into a vector backbone comprising other appropriate antibody sequences, such as variable regions and Fc sequences, as appropriate.

Another type of variant is an amino acid substitution variant. These variants have at least one amino acid residue in the antibody molecule replaced by a different residue. Conservative substitutions are shown in Table 2 under the heading of "conservative substitutions". Conservative or preferred substitutions, shown in Table 2, or as further described below in reference to amino acid classes, may be introduced and the products screened.

**Table 2**

| Original | Conservative | Preferred |
|---|---|---|
| Residue | Substitutions | Substitutions |
| Ala (A) | val; leu; ile | val |
| Arg (R) | lys; gln; asn | lys |
| Asn (N) | gln; his; lys; arg | gln |
| Asp (D) | glu | glu |
| Cys (C) | ser; ala; thr; val | ser |
| Gln (Q) | asn | asn |
| Glu (E) | asp | asp |
| Gly (G) | pro; ala | ala |
| His (H) | asn; gln; lys; arg | arg |
| Ile (I) | leu; val; met; ala; phe | leu |
| Leu (L) | ile; val; met; ala; phe | ile |
| Lys (K) | arg; gln; asn | arg |
| Met (M) | leu; phe; ile | leu |
| Phe (F) | leu; val; ile; ala; tyr | leu |
| Pro (P) | ala | ala |
| Ser (S) | thr | thr |
| Thr (T) | ser | ser |
| Trp (W) | tyr; phe | tyr |
| Tyr (Y) | trp; phe; thr; ser | phe |
| Val (V) | ile; leu; met; phe; ala | leu |

Substantial modifications in the biological properties of the antibody are accomplished by selecting substitutions that differ significantly in their effect on maintaining (a) the structure of the polypeptide backbone in the area of the substitution, for example, as a sheet or helical conformation, (b) the charge or hydrophobicity of the molecule at the target site, or (c) the bulk of the side chain. Naturally occurring residues are divided into groups based on common side-chain properties:
(1) hydrophobic: met, ala, val, leu, ile;
(2) neutral hydrophilic: cys, ser, thr;
(3) acidic: asp, glu;
(4) basic: asn, gln, his, lys, arg;
(5) residues that influence chain orientation: gly, pro; and
(6) aromatic: trp, tyr, phe.

Non-conservative substitutions will entail exchanging a member of one of these classes for another class.

Nucleic acid molecules encoding amino acid sequence variants of the antibody are prepared by a variety of methods known in the art. These methods include, but are not limited to, isolation from a natural source (in the case of naturally occurring amino acid sequence variants) or preparation by oligonucleotide-mediated (or site-directed) mutagenesis, PCR mutagenesis, and cassette mutagenesis of an earlier prepared variant or a non-variant version of the antibody.

Mutagenesis may be performed in accordance with any of the techniques known in the art including, but not limited to, synthesizing an oligonucleotide having one or more modifications within the sequence of the heavy chain hinge region of an immunoglobulin to be modified. Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Typically, a primer of about 17 to about 75 nucleotides or more in length is preferred, with about 10 to about 25 or more residues on both sides of the junction of the sequence being altered. A number of such primers introducing a variety of different mutations at one or more positions may be used to generate a library of mutants.

The technique of site-specific or site-directed mutagenesis is well known in the art, as exemplified by Ausubel et al., 2006, Current Protocols in Molecular Biology, Greene Publishing Associates and Wiley Interscience, N.Y.. In general, site-directed mutagenesis is performed by first obtaining a single-stranded vector or melting apart of two strands of a double stranded vector which includes within its sequence a DNA sequence which encodes the desired peptide. An oligonucleotide primer bearing the desired mutated sequence is prepared, generally synthetically. This primer is then annealed with the single-stranded vector, and subjected to DNA polymerizing enzymes such as T7 DNA polymerase, in order to complete the synthesis of the mutation-bearing strand. Thus, a heteroduplex is formed wherein one strand encodes the original non-mutated sequence and the second strand bears the desired mutation. This heteroduplex vector is then used to transform or transfect appropriate cells, such as E. coli or CHO cells, and clones are selected which include recombinant vectors bearing the mutated sequence arrangement.

Alternatively, the use of PCR with commercially available thermostable enzymes such as Taq DNA polymerase may be used to incorporate a mutagenic oligonucleotide primer into an amplified DNA fragment that can then be cloned into an appropriate cloning or expression vector. See, e.g., Tomic et al., Nucleic Acids Res., 18(6):1656, 1987, and Upender et al., Biotechniques, 18(1):29-30, 32, 1995, for PCR-mediated mutagenesis procedures, which are hereby incorporated in their entireties. PCR employing a thermostable ligase in addition to a thermostable polymerase may also be used to incorporate a phosphorylated mutagenic oligonucleotide into an amplified DNA fragment that may then be cloned into an appropriate cloning or expression vector (see e.g., Michael, Biotechniques, 16(3):410-2, 1994.

### Sequencing

Any of a variety of sequencing reactions known in the art can be used to directly sequence the nucleotide sequence encoding an antibody or antibody fragment with an amino acid modification. Examples of sequencing reactions include those based on techniques developed by Maxim and Gilbert (Proc. Natl. Acad. Sci. USA, 74:560, 1977) or Sanger (Proc. Natl. Acad. Sci. USA, 74:5463, 1977). It is also contemplated that any of a variety of automated sequencing procedures can be utilized (Bio/Techniques, 19:448, 1995), including sequencing by mass spectrometry (see, e.g., PCT Publication No. WO 94/16101, Cohen et al., Adv. Chromatogr., 36:127-162, 1996, and Griffin et al., Appl. Biochem. Biotechnol., 38:147-159, 1993).

### Antigen Specificity

Embodiments of the invention are applicable to antibodies of any appropriate antigen binding specificity. Preferably, the antibodies of the invention are specific to antigens that are biologically relevant polypeptides. More preferably, the antibodies of the invention are useful for therapy or diagnosis of diseases or disorders in a mammal, such as a human.

Modified IgG2 antibodies as described herein are particularly useful as therapeutic antibodies such as blocking antibodies, agonist antibodies, neutralizing antibodies or antibody conjugates. Non-limiting examples of therapeutic antibodies include anti-IL-1R (described in U.S. Patent Pub. No. 2004/097712), anti-RANKL (described in WO 03/002713), anti-EGFr (described in U.S. Patent No. 6,235,883), anti-IL-4 receptor (described in U.S. Patent Pub. No. 2005/0112694 and U.S. Patent No. 7,186,809), anti-HGF (described in U.S. Patent Pub. No. 2005/0118643), anti Ang-1 and anti-Ang-2 (described in U.S. Patent Pub. No. 2003/0124129 and WO 03/030833), anti-Ang-4, anti-OX-40, anti-GM-CSF, anti-NGF, anti-glucagon receptor, anti-sclerostin, anti-IL-17R, anti-CD30, anti-IL18, anti-activin, anti-VEGF, anti-IgE, anti-CD11, anti-CD18, anti-CD40, anti-tissue factor (TF), anti-HER2, and anti-TrkC antibodies. Antibodies directed against non-polypeptide antigens (such as tumor-associated glycolipid antigens) are also contemplated.

Described but not part of the invention is an anti-RANKL antibody with increased structural homogeneity. For example, antibodies against RANKL are described in WO 03/002713, and exemplified by heavy chain comprising the variable region amino acid sequence set forth herein as SEQ ID NO: 60 and light chain comprising the variable region amino acid sequence set forth herein as SEQ ID NO: 61. Other embodiments include antibodies comprising a heavy chain amino acid sequence set forth herein as SEQ ID NO: 58, and comprising a light chain amino acid sequence set forth herein as SEQ ID NO: 59. Accordingly, modifications can be made to an anti-RANKL antibody according to the present invention, such that the light chain polypeptide primarily forms an interchain disulfide bond only with amino acids in the hinge region through the most C-terminal cysteine residue of the light chain. Such modifications can include substitution or deletion of a cysteine residue at Eu position 131 of the heavy chain polypeptide of the anti-RANKL antibody. Other modifications can be made to an anti-RANKL antibody such that the light chain primarily forms an interchain disulfide bond only with amino acids outside the hinge region through the most C-terminal cysteine residue of the light chain. Such modifications can include substitution or deletion of a cysteine residue in the hinge region of the anti-RANKL antibody. Exemplary substitutions or deletions may be at Eu position 219 or 220 of the heavy chain polypeptide of the anti-RANKL antibody. Further modifications to anti-RANKL antibodies contemplated herein include insertions of one or more amino acids in the hinge region of the heavy chain polypeptide or insertions of one or more amino acids in the C-terminal region of the light chain polypeptide.

Herein disclosed is an anti-EGFR antibody with increased structural homogeneity. For example, antibodies against EGFR are described in U.S. Patent No. 6,235,883, and exemplified by heavy chain comprising the variable region amino acid sequence set forth herein as SEQ ID NO: 57 and light chain amino acid sequence set forth herein as SEQ ID NO: 49. Also disclosed herein are antibodies comprising a heavy chain variable region amino acid sequence set forth herein as any one of SEQ ID NOs: 34, 36, 38, 40, 42, 44, 46, 48 and 50-57, and comprising a light chain variable region amino acid sequence set forth herein as any one of SEQ ID NOs: 35, 37, 39, 41, 43, 45, 47 or 49. Accordingly, modifications can be made to an anti-EGFR antibody according to the present disclosure such that the light chain polypeptide primarily forms an interchain disulfide bond only with amino acids in the hinge region through the most C-terminal cysteine residue of the light chain. Such modifications can include substitution or deletion of a cysteine residue at Eu position 131 of the heavy chain polypeptide of the anti-EGFR antibody. Other modifications can be made to an anti-EGFR antibody such that the light chain primarily forms an interchain disulfide bond only with amino acids outside the hinge region through the most C-terminal cysteine residue of the light chain. Such modifications can include substitution of a cysteine residue in the hinge region of the anti-EGFR antibody. Substitutions are at Eu position 219 or 220 of the heavy chain polypeptide of the anti-EGFR antibody.

Described but not part of the invention is an anti-IL-1R antibody with increased structural homogeneity. In one particular embodiment, the antibody is an anti-IL-1R type 1 antibody. For example, antibodies against IL-1R are described in U.S. Patent Pub. No. 2004/097712, and exemplified by heavy chain comprising the variable region amino acid sequence set forth herein as any one of SEQ ID NOs: 62, 64,65, 78, 80 or 81 and light chain comprising the variable region amino acid sequence set forth herein as any one of SEQ ID NOs: 63,66, 79 or 82. Other embodiments include an antibody comprising a heavy chain amino acid sequence set forth herein as any one of SEQ ID NOs: 67-75, and comprising a light chain amino acid sequence set forth herein as any one of SEQ ID NOs: 76-77. Accordingly, modifications can be made to an anti-IL-1R antibody according to the present invention, such that the light chain polypeptide primarily forms an interchain disulfide bond only with amino acids in the hinge region through the most C-terminal cysteine residue of the light chain. Such modifications can include substitution or deletion of a cysteine residue at Eu position 131 of the heavy chain polypeptide of the anti-IL-1R antibody. Other modifications can be made to an anti- IL-1R antibody such that the light chain primarily forms an interchain disulfide bond only with amino acids outside the hinge region through the most C-terminal cysteine residue of the light chain. Such modifications can include substitution or deletion of a cysteine residue in the hinge region of the anti-IL-1R antibody. Exemplary substitutions or deletions may be at Eu position 219 or 220 of the heavy chain polypeptide of the anti-IL-1R antibody. Further modifications to anti-IL-1R antibodies contemplated herein include insertions of one or more amino acids in the hinge region of the heavy chain polypeptide or insertions of one or more amino acids in the C-terminal region of the light chain polypeptide.

Where the antigen is a polypeptide, it may be a transmembrane molecule (e.g. receptor) or a ligand such as a growth factor. Exemplary antigens include molecules such as renin; a growth hormone, including human growth hormone and bovine growth hormone; growth hormone releasing factor; parathyroid hormone; thyroid stimulating hormone; lipoproteins; alpha-1-antitrypsin; insulin A-chain; insulin B-chain; proinsulin; follicle stimulating hormone; calcitonin; luteinizing hormone; glucagon; Angiopoietin-1 (Ang-1), Angiopoietin-2 (Ang-2), Angiopoietin-4 (Ang-4), OX-40, GM-CSF, NGF, glucagon receptor, sclerostin, IL-17R, CD30, IL18, activin, VEGF, IgE, CD11, CD18, CD40, tissue factor (TF), HER2, TrkC clotting factors such as factor VIIIC, factor IX, tissue factor (TF), and von Willebrands factor; clotting factors such as Protein C; atrial natriuretic factor; lung surfactant; a plasminogen activator, such as urokinase or human urine or tissue-type plasminogen activator (t-PA); bombesin; thrombin; hemopoietic growth factor; tumor necrosis factor-alpha and -beta; enkephalinase; RANTES (regulated on activation normally T-cell expressed and secreted); human macrophage inflammatory protein (MIP-1-alpha); a serum albumin such as human serum albumin; Muellerian-inhibiting substance; relaxin A-chain; relaxin B-chain; prorelaxin; mouse gonadotropin-associated peptide; a microbial protein, such as beta-lactamase; DNase; IgE; a cytotoxic T-lymphocyte associated antigen (CTLA), such as CTLA-4; inhibin; activin; vascular endothelial growth factor (VEGF); receptors for hormones or growth factors such as epidermal growth factor receptor (EGFr); interleukin receptors such as IL-1R and IL-4R; protein A or D; rheumatoid factors; a neurotrophic factor such as bone-derived neurotrophic factor (BDNF), neurotrophin-3, -4, -5, or -6 (NT-3, NT-4, NT-5, or NT-6), or a nerve growth factor such as NGF-β; platelet-derived growth factor (PDGF); fibroblast growth factor such as aFGF and bFGF; epidermal growth factor (EGF); transforming growth factor (TGF) such as TGF-alpha and TGF-beta, including TGF-β1, TGF-β2, TGF-β3, TGF-β4, or TGF-p5; insulin-like growth factor-I and -II (IGF-I and IOF-II); des(1-3)-IGF-I (brain IGF-I), insulin-like growth factor binding proteins; CD proteins such as CD3, CD4, CD8, CD19, CD20 and CD40; erythropoietin; osteoinductive factors; immunotoxins; a bone morphogenetic protein (BMP); Receptor Activator of Nuclear Factor Kappa B Ligand (RANK-L); an interferon such as interferon-alpha, -beta, and - gamma; colony stimulating factors (CSFs), e.g., M-CSF, GM-CSF, and G-CSF; interleukins (ILs), e.g., IL-1 to IL-10; superoxide dismutase; T-cell receptors; surface membrane proteins; decay accelerating factor; viral antigen such as, for example, a portion of the HIV envelope; transport proteins; homing receptors; addressins; regulatory proteins; integrins such as CD11a, CD11b, CD11c, CD18, an ICAM, VLA-4 and VCAM; a tumor associated antigen such as HER2, HER3 or HER4 receptor; and fragments of any of the above-listed polypeptides.

Exemplary antigens for antibodies encompassed by the present disclosure include CD proteins such as CD3, CD4, CD8, CD19, CD20, CD34, and CD46; members of the ErbB receptor family such as the EGF receptor, HER2, HER3 or HER4 receptor; cell adhesion molecules such as LFA-1, Mac1, p150.95, VLA-4, ICAM-1, VCAM, a4/p7 integrin, and αv/β3 integrin including either α or β subunits thereof (e.g. anti-CD11a, anti-CD18 or anti-CD11b antibodies); growth factors such as VEGF; tissue factor (TF); TGF-β; alpha interferon (α-IFN); an interleukin, such as IL-8; IgE; blood group antigens Apo2, death receptor; flk2/flt3 receptor; obesity (OB) receptor; mpl receptor, CTLA-4; protein C etc. The most preferred targets herein are VEGF, TF, CD19, CD20, CD40, TGF-β, CD11a, CD18, Apo2 and C24.

Soluble antigens or fragments thereof, optionally conjugated to other molecules, can be used as immunogens for generating antibodies. For transmembrane molecules, such as receptors, fragments of these molecules (e.g. the extracellular domain of a receptor) can be used as the immunogen. Alternatively, cells expressing the transmembrane molecule can be used as the immunogen. Such cells can be derived from a natural source (e.g. cancer cell lines) or may be cells which have been transformed by recombinant techniques to express the transmembrane molecule. Other antigens and forms thereof useful for preparing antibodies will be apparent to those in the art.

The antibodies of the present disclosure may be monospecific, bispecific, trispecific or of greater multispecificity. Multispecific antibodies may be specific to different epitopes of a single molecule or may be specific to epitopes on different molecules. Methods for designing and making multispecific antibodies are known in the art. See, e.g., Millstein et al. (1983) Nature 305:537-539; Kostelny et al. (1992) J. Immunol. 148:1547-1553; WO 93/17715.

### Vector Construction

Polynucleotide sequences encoding the immunoglobulin polypeptides of the disclosure can be obtained using standard recombinant techniques. Desired polynucleotide sequences may be isolated and sequenced from antibody producing cells such as hybridoma cells. Alternatively, polynucleotides can be synthesized using nucleotide synthesizer or PCR techniques. Once obtained, sequences encoding the immunoglobulins are inserted into a recombinant vector capable of replicating and expressing heterologous polynucleotides in a host cell. Many vectors that are available and known in the art can be used for the purpose of the present invention. Selection of an appropriate vector will depend mainly on the size of the nucleic acids to be inserted into the vector and the particular host cell to be transformed with the vector. Each vector contains various components, depending on its function (amplification or expression of heterologous polynucleotide, or both) and its compatibility with the particular host cell in which it resides. The vector components generally include, but are not limited to: an origin of replication (in particular when the vector is inserted into a prokaryotic cell), a selection marker gene, a promoter, a ribosome binding site (RBS), a signal sequence, the heterologous nucleic acid insert and a transcription termination sequence.

### Production of Recombinant Antibodies

Methods for producing recombinant antibodies in mammalian cells are known. In such methods, the antibody production involves induction of protein expression. Nucleic acids encoding an IgG antibody or an IgG antibody fragment are conveniently rendered expressible by operative association with a promoter, preferably a controllable promoter functional in mammalian cells. Such recombinant constructs are designed for expression of IgG antibody protein in a suitable host (e.g., bacterial, murine, or human). Suitable promoters for expression of proteins and polypeptides herein are widely available and are well known in the art. Inducible promoters or constitutive promoters that are linked to regulatory regions (e.g., enhancers, operators, and binding regions for transcription or translation factors) are preferred. An "inducible" promoter is defined herein as a controllable promoter, including promoters typically referenced as inducible promoters (i.e., subject to positive regulation in being inactive until activated or induced by the presence of an activator or inducer) or as derepressible promoters (i.e., subject to negative regulation in being active unless a repressor is present, with removal of the repressor, or derepression, resulting in an increase in promoter activity). Promoters contemplated herein include, for example, but are not limited to, the trp, lpp, tac, and lac promoters, such as the lacUV5, from E. coli; the P10 or polyhedrin gene promoter of baculovirus/insect cell expression systems (see, e.g., U.S. Pat. Nos. 5,243,041, 5,242,687, 5,266,317, 4,745,051, and 5,169,784) and inducible promoters from other eukaryotic expression systems, as would be known in the art. For expression of the proteins, such promoters are inserted in a plasmid in operative linkage with a control region such as the operator region of the trp operon.

In addition to mammalian cells, the IgG2 molecules can be also produced in any other suitable eukaryotic or prokaryotic host cell using techniques know in the art. For example, the molecules can be produced in bacteria such as E. coli. As a further example, the molecules can be produced in insect cells transformed using baculovirus.

Described but not part of the invention are methods of treating a disease, disorder, or condition, in addition to the prophylactic methods or method of preventing such diseases, disorders and conditions, an effective amount of a recombinant polypeptide is an amount of the polypeptide that will produce the desired biological or physiological effect, as would be known in the art. Particularly with respect to treatment methods, as well as the methods of ameliorating a symptom associated with a disease, disorder or condition, an effective amount is used synonymously with a therapeutically effective amount. In such methods, a subject in need is any animal, e.g., a human, exhibiting a symptom of, at risk of developing, or diagnosed as having a disease, disorder or condition.

The antibody modifications provided herein find particular use in increasing homogeneity of IgG antibodies that are produced in e.g., mammalian cells. In some embodiments, the invention is specifically directed to improved production of IgG2 molecules. The heterogeneity of such proteins due to the presence of different disulfide bonded forms is significantly reduced as a result of the use of the modifications described herein. These beneficial results may be assessed by monitoring such heterogeneity using the LC and LC/MS methods known to those of skill in the art.

Specifically, antibodies that are secreted in mammalian cell systems will be glycosylated. Preferably, the proteins are secreted by mammalian production cells adapted to grow in cell culture. Examples of such cells commonly used in the industry are CHO, VERO, NSO, BK, HeLa, CV1 (including Cos), MDCK, 293, 3T3-myeloma cell lines (such as murine), PC12 and WI38 cells. Typical host cells are Chinese hamster ovary (CHO) cells, which are widely used for the production of several complex recombinant proteins, e.g. cytokines, clotting factors, and antibodies (Brasel et al., 1996, Blood 88:2004-2012; Kaufinan et al., 1988, J. Biol Chem 263: 6352-6362; McKinnon et al., 1991, J Mol Endocrinol 6:231-239; Wood et al., 1990, J. Immunol 145:3011-3016). The dihydrofolate reductase (DHFR) deficient mutant cell line (Urlaub et al., 1980, Proc Natl Acad Sci USA 77:4216-4220), DXB11 and DG-44, are the CHO host cell lines of choice because the efficient DHFR selectable and amplifiable gene expression system allows high level recombinant protein expression in these cells (Kaufman R. J., 1990, Meth Enzymol 185:527-566). In addition, these cells are easy to manipulate as adherent or suspension cultures and exhibit relatively good genetic stability. CHO cells and recombinant proteins expressed in them have been extensively characterized and have been approved for use in clinical manufacturing by regulatory agencies.

Host cells are transformed or transfected with the above-described expression vectors and cultured in conventional nutrient media modified as appropriate for inducing promoters, selecting transformants, or amplifying the genes encoding the desired sequences.

Transfection refers to the taking up of an expression vector by a host cell whether or not any coding sequences are in fact expressed. Numerous methods of transfection are known to the ordinarily skilled artisan, for example, CaPO₄ precipitation and electroporation. Successful transfection is generally recognized when any indication of the operation of this vector occurs within the host cell.

Transformation means introducing DNA into the prokaryotic host so that the DNA is replicable, either as an extrachromosomal element or by chromosomal integrant. Depending on the host cell used, transformation is done using standard techniques appropriate to such cells. The calcium treatment employing calcium chloride is generally used for bacterial cells that contain substantial cell-wall barriers. Another method for transformation employs polyethylene glycol/DMSO. Yet another technique used is electroporation.

The preparation of the recombinant modified IgG2 antibodies is preferably achieved in the media of the cell culture. The recombinant antibodies are produced by the cells in that culture and subsequently purified. The preparation of recombinant protein can be a cell culture supernatant, cell extract, but is preferably a partially purified fraction from the same. By partially purified means that some fractionation procedure, or procedures, have been carried out, but that more polypeptide species (at least 10%) than the desired protein or protein conformation is present. The recombinant protein can be at a fairly high concentration. Some concentration ranges are 0.1 to 20 mg/ml, more preferably from 0.5 to 15 mg/ml, and still more preferably from 1 to 10 mg/ml.

The preparation of recombinant modified IgG2 antibodies can be prepared initially by culturing recombinant host cells under culture conditions suitable to express the polypeptide. The polypeptide can also be expressed as a product of transgenic animals, e.g., as a component of the milk of transgenic cows, goats, pigs, or sheep which are characterized by somatic or germ cells containing a nucleotide sequence encoding the polypeptide. The resulting expressed polypeptide can then be purified, or partially purified, from such culture or component (e.g., from culture medium or cell extracts or bodily fluid) using known processes. While fractionation including but not limited to one or more steps of filtration, centrifugation, precipitation, phase separation, affinity purification, gel filtration, ion exchange chromatography, hydrophobic interaction chromatography (HIC; using such resins as phenyl ether, butyl ether, or propyl ether), HPLC, or some combination of above may be used herein, the advantageous methods of the present disclosure may employ LC fractionation and purification of the high molecular weight therapeutic proteins as described in U.S. patent application Pub. No: 2005/0161399, and Pub. No. 2006/194280.

The LC and LC/MS methods described herein also may be combined with other purification methods, such as for example, purification of the polypeptide using an affinity column containing agents which will bind to the polypeptide; one or more column steps over such affinity resins as concanavalin A-agarose, heparin-toyopearl^{™} or Cibacrom blue 3GA Sepharose^{™} one or more steps involving elution; and/or immunoaffinity chromatography. The polypeptide can be expressed in a form that facilitates purification. For example, it may be expressed as a fusion polypeptide, such as those of maltose binding polypeptide (MBP), glutathione-S-transferase (GST) or thioredoxin (TRX). Kits for expression and purification of such fusion polypeptides are commercially available from NEW ENGLAND BIOLAB^{®} (Beverly, Mass.), PHARMACIA^{®} (Piscataway, N.J.) and INVITROGEN^{®}, respectively. The polypeptide can be tagged with an epitope and subsequently purified by using a specific antibody directed to such epitope. One such epitope (FLAG^{™}) is commercially available from KODAK^{®} (New Haven, Conn.). It is also possible to utilize an affinity column comprising a polypeptide-binding polypeptide, such as a monoclonal antibody to the recombinant protein, to affinity-purify expressed polypeptides. Other types of affinity purification steps can be a Protein A or a Protein G column, which affinity agents bind to proteins, that contain Fc domains. Polypeptides can be removed from an affinity column using conventional techniques, e.g., in a high salt elution buffer and then dialyzed into a lower salt buffer for use or by changing pH or other components depending on the affinity matrix utilized, or can be competitively removed using the naturally occurring substrate of the affinity moiety. In one embodiment of the invention, the preparation of recombinant protein may be partially purified over a Protein A affinity column.

Some or all of the foregoing purification steps, in various combinations, can also be employed to prepare an appropriate preparation of a recombinant IgG2 for use in the methods of the disclosure, and/or to further purify such a recombinant polypeptide after contacting the preparation of the recombinant protein with a reduction/oxidation coupling reagent. The polypeptide that is substantially free of other mammalian polypeptides is defined as an isolated polypeptide. The specific LC methods that may be combined with the redox reagent-based methods described herein are described in further detail in U.S. patent application Pub. No: 2005/0161399, and Pub. No. 2006/194280.

The polypeptide can also be produced by known conventional chemical synthesis. Methods for constructing polypeptides by synthetic means are known to those skilled in the art. The synthetically-constructed polypeptide sequences can be glycosylated *in* The desired degree of final purity depends on the intended use of the polypeptide. A relatively high degree of purity is desired when the polypeptide is to be administered *in vivo,* for example. In such a case, the polypeptides are purified such that no polypeptide bands corresponding to other polypeptides are detectable upon analysis by SDS-polyacrylamide gel electrophoresis (SDS-PAGE). It will be recognized by one skilled in the pertinent field that multiple bands corresponding to the polypeptide can be visualized by SDS-PAGE, due to differential glycosylation, differential post-translational processing, and the like. Most preferably, the polypeptide of the invention is purified to substantial homogeneity, as indicated by a single polypeptide band upon analysis by SDS-PAGE. The polypeptide band can be visualized by silver staining, Coomassie blue staining, and/or (if the polypeptide is radiolabeled) by auto radiography.

### Characterization of Structural Isoforms of Modified Antibodies

Determination of the conformation of a protein, and the relative proportions of a conformation of a protein in a mixture, can be done using any of a variety of analytical and/or qualitative techniques. If there is a difference in activity between the conformations of the protein, determining the relative proportion of a conformation in the mixture can be done by way of an activity assay (e.g., binding to a ligand, enzymatic activity, biological activity, etc.). Biological activity of the protein also could be used. Alternatively, the binding assays can be used in which the activity is expressed as activity units/mg of protein.

If the two conformations resolve differently during separation techniques such as chromatography, electrophoresis, filtering or other purification technique, then the relative proportion of a conformation in the mixture can be determined using such purification techniques. For example, at least two different conformations of the recombinant IgG could be resolved by way of hydrophobic interaction chromatography. Further, since far UV Circular Dichroism has been used to estimate secondary structure composition of proteins (Percze1 et al., 1 991, Protein Engrg. 4:669-679), such a technique can determine whether alternative conformations of a protein are present. Still another technique used to determine conformation is fluorescence spectroscopy which can be employed to ascertain complementary differences in tertiary structure assignable to tryptophan and tyrosine fluorescence. Other techniques that can be used to determine differences in conformation and, hence, the relative proportions of a conformation, are on-line SEC to measure aggregation status, differential scanning calorimetry to measure melting transitions (Tm's) and component enthalpies, and chaotrope unfolding. In some embodiments described in detail herein below the invention uses LC/MS detection to determine the heterogeneity of the protein.

By the term isolating is meant physical separation of at least one component in a mixture away from other components in a mixture. Isolating components or particular conformations of a protein can be achieved using any purification method that tends to separate such components. Accordingly, one can perform multiple chromatography steps in addition to the RP-HPLC described below, including but not limited to HIC, hydroxyapatite chromatography, ion exchange chromatography, affinity, and SEC. Other purification methods are filtration (e.g., tangential flow filtration), electrophoretic techniques (e.g., electrophoresis, electroelution, isoelectric focusing), and phase separation (e.g., PEG-dextran phase separation), to name just a few. In addition, the fraction of the preparation of recombinant protein that contains the protein in the undesired conformation can be treated again in the methods of the disclosure, to further optimize the yields of protein with the desired conformation.

### Activity Assays

The immunoglobulins of the present disclosure can be characterized for their physical/chemical properties and biological functions by various assays known in the art. In one aspect of the invention, it is important to compare the altered immunoglobulin of the present invention to a reference (generally wild type counterpart) immunoglobulin. Particularly, the quantity of an altered immunoglobulin of the present invention expressed according to a method of the invention can be compared to that of a reference immunoglobulin expressed under similar culture conditions. Methods for protein quantification are well known in the art. For example, samples of the expressed proteins can be compared for their quantitative intensities on a Coomassie-stained SDS-PAGE. Alternatively, the specific band(s) of interest (e.g., the full length band) can be detected by, for example, western blot gel analysis and/or AME5-RP assay.

The purified immunoglobulins can be further characterized by a series of assays including, but not limited to, N-terminal sequencing, amino acid analysis, non-denaturing size exclusion high pressure liquid chromatography (HPLC), cation exchange chromatography (CEX), mass spectrometry, ion exchange chromatography and trypsin digestion.

In certain embodiments of the disclosure, the immunoglobulins produced herein are analyzed for their biological activity. In some embodiments, the immunoglobulins of the present disclosure are tested for their antigen binding activity. The antigen binding assays that are known in the art and can be used herein include without limitation any direct or competitive binding assays using techniques such as western blots, radioimmunoassays, ELISA (enzyme linked immnosorbent assay), "sandwich" immunoassays, immunoprecipitation assays, fluorescent immunoassays, and protein A immunoassays.

The disclosure also pertains to immunoconjugates comprising an immunoglobulin polypeptide of the invention conjugated to a cytotoxic agent such as a chemotherapeutic agent (as defined and described herein above), toxin (e.g. a small molecule toxin or an enzymatically active toxin of bacterial, fungal, plant or animal origin, including fragments and/or variants thereof), or a radioactive isotope (i.e., a radioconjugate).

Conjugates of an antibody and one or more small molecule toxins, such as a calicheamicin, a maytansine (U.S. Pat. No. 5,208,020), a trichothene, and CC1065 are also contemplated herein.

In one aspect of the disclosure, the immunoglobulin is conjugated to one or more maytansine molecules (e.g. about 1 to about 10 maytansine molecules per antibody molecule). Maytansine may, for example, be converted to May-SS-Me which may be reduced to May-SH3 and reacted with modified antibody (Chari et al. Cancer Research 52: 127-131 (1992)) to generate a maytansinoid-antibody immunoconjugate.

Enzymatically active toxins and fragments thereof which can be used include diphtheria A chain, nonbinding active fragments of diphtheria toxin, exotoxin A chain (from Pseudomonas aeruginosa), ricin A chain, abrin A chain, modeccin A chain, alpha-sarcin, Aleurites fordii proteins, dianthin proteins, Phytolaca americana proteins (PAPI, PAPII, and PAP-S), momordica charantia inhibitor, curcin, crotin, sapaonaria officinalis inhibitor, gelonin, mitogellin, restrictocin, phenomycin, enomycin and the tricothecenes. See, for example, WO 93/21232 published Oct. 28, 1993.

The present disclosure further contemplates an immunoconjugate formed between an immunoglobulin of the invention and a compound with nucleolytic activity (e.g. a ribonuclease or a DNA endonuclease such as a deoxyribonuclease; DNase).

A variety of radioactive isotopes are available for the production of radioconjugated antibodies. Examples include At²¹¹, I¹³¹, I¹²⁵, Y⁹⁰, Re¹⁸⁶, Re¹⁸⁸, Sm¹⁵³, Bi.²¹², P³² and radioactive isotopes of Lu.

Conjugates of the immunoglobulin of the invention and cytotoxic agent may be made using a variety of bifunctional protein coupling agents such as N-succinimidyl-3-(2-pyridyldithiol) propionate (SPDP), succinimidyl-4-(N-maleimidomethyl) cyclohexane-1-carboxylate, iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al. Science 238: 1098 (1987). Carbon-14-labeled 1-isothiocyanatobenzyl-3-methyldiethylene triaminepentaacetic acid (MX-DTPA) is an exemplary chelating agent for conjugation of radionucleotide to the antibody. See WO94/11026. The linker may be a "cleavable linker" facilitating release of the cytotoxic drug in the cell. For example, an acid-labile linker, peptidase-sensitive linker, dimethyl linker or disulfide-containing linker (Chari et al. Cancer Research 52: 127-131 (1992)) may be used.

Alternatively, a fusion protein comprising the immunoglobulin and cytotoxic agent may be made, e.g. by recombinant techniques or peptide synthesis.

In yet another aspect, an immunoglobulin of the disclosure may be conjugated to a "receptor" (such as streptavidin) for utilization in tumor pretargeting wherein the antibody-receptor conjugate is administered to the patient, followed by removal of unbound conjugate from the circulation using a clearing agent and then administration of a "ligand" (e.g. avidin) which is conjugated to a cytotoxic agent (e.g. a radionucleotide).

### Antibody Derivatives

The antibodies and antibody variants of the present disclosure can be further modified to contain additional nonproteinaceous moieties that are known in the art and readily available. Preferably, the moieties suitable for derivatization of the antibody are water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1,3-dioxolane, poly-1,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide copolymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymers are attached, they can be the same or different molecules. In general, the number and or type of polymers used for derivatization can be determined based on considerations including, but not limited to, the particular properties or functions of the antibody to be improved, whether the antibody derivative will be used in a therapy under defined conditions.

### Fusion Proteins

It has been found that the antibody modifications provided herein effectively reduce the heterogeneity of recombinant IgG2 molecules that can adopt multiple conformations and/or contain more than one domain. A domain is a contiguous region of the polypeptide chain that adopts a particular tertiary structure and/or has a particular activity that can be localized in that region of the polypeptide chain. For example, one domain of a protein can have binding affinity for one ligand, and one domain of a protein can have binding affinity for another ligand. In a thermostable sense, a domain can refer to a cooperative unfolding unit of a protein. Such proteins that contain more than one domain can be found naturally occurring as one protein or genetically engineered as a fusion protein. In addition, domains of a polypeptide can have subdomains.

The antibody modifications provided herein are also useful for preparation of other types of recombinant IgG2 proteins, including immunoglobulin molecules or portions thereof, and chimeric antibodies (e.g., an antibody having a human constant region coupled to a murine antigen binding region) or fragments thereof. Numerous techniques are known by which DNA encoding immunoglobulin molecules can be manipulated to yield DNAs capable of encoding recombinant proteins such as single chain antibodies, antibodies with enhanced affinity, or other antibody based polypeptides (see, for example, Larrick et al., 1989, Biotechnology 7:934-938; Reichmann et al., 1988, Nature 332:323-327; Roberts et al., 1987, Nature 328:731-734; Verhoeyen et al., 1988, Science 239:1534-1536; Chaudhary et al., 1989, Nature 339:394-397). Preparations of fully human antibodies (such as are prepare using transgenic animals, and optionally further modified in vitro), as well as humanized antibodies, can also be used in the invention. The term humanized antibody also encompasses single chain antibodies. See, e.g., Cabilly et al., U.S. Pat. No. 4,816,567; Cabilly et al., European Pat. No. 0,125,023 B1; Boss et al., U.S. Pat. No. 4,816,397; Boss et al., European Pat. No. 0,120,694 B1; Neuberger, M. S. et al., WO 86/01533; Neuberger, M. S. et al., European Pat. No. 0,194,276 B1; Winter, U.S. Pat. No. 5,225,539; Winter, European Pat. No. 0,239,400 B1; Queen et al., European Pat. No. 0 451 216 B1; and Padlan, E. A. et al., EP 0 519 596 A1. The antibody modifications provided herein may also be used during the preparation of conjugates comprising an antibody and a cytotoxic or luminescent substance. Such substances include: maytansine derivatives (such as DM1); enterotoxins (such as a Staphlyococcal enterotoxin); iodine isotopes (such as iodine-125); technium isotopes (such as Tc-99m); cyanine fluorochromes (such as Cy5.5.18); and ribosome-inactivating proteins (such as bouganin, gelonin, or saporin-S6).

Preparations of various fusion proteins can also be prepared using the modified antibodies provided herein. Examples of such fusion proteins include proteins expressed as a fusion with a portion of a recombinant IgG (i.e., the IgG1, IgG2, IgG3, or IgG4) molecule, proteins expressed as fusion proteins with a zipper moiety, and novel polyfunctional proteins such as a fusion proteins of a cytokine and a growth factor (i.e., GM-CSF and IL-3, MGF and IL-3). WO 93/08207 and WO 96/40918 describe the preparation of various soluble oligomeric forms of a molecule referred to as CD40L, including an immunoglobulin fusion protein and a zipper fusion protein, respectively; the techniques discussed therein are applicable to other proteins. Any of the above molecules can be expressed as a fusion protein including but not limited to the extracellular domain of a cellular receptor molecule, an enzyme, a hormone, a cytokine, a portion of an immunoglobulin molecule, a zipper domain, and an epitope.

### Pharmaceutical Formulations

Therapeutic formulations comprising an antibody of the disclosure are prepared for storage by mixing the antibody having the desired degree of purity with optional physiologically acceptable carriers, excipients or stabilizers (Remington's Pharmaceutical Sciences 16th edition, Osol, A. Ed. (1980)), in the form of aqueous solutions, lyophilized or other dried formulations. Acceptable carriers, excipients, or stabilizers are nontoxic to recipients at the dosages and concentrations employed, and include buffers such as phosphate, citrate, histidine and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glutamic acid, proline, glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN^{™}, PLURONICS^{™} or polyethylene glycol (PEG).

The formulation herein may also contain more than one active compound as necessary for the particular indication being treated, preferably those with complementary activities that do not adversely affect each other. Such molecules are suitably present in combination in amounts that are effective for the purpose intended.

The active ingredients may also be entrapped in microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained-release preparations include semipermeable matrices of solid hydrophobic polymers containing the immunoglobulin of the invention, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-glutamic acid and γ-ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated immunoglobulins remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions. In this regard, reduction/elimination of disulfide forming cysteine residues as described herein may be particularly advantageous.

### Uses

An immunoglobulin of the present disclosure may be used, for example, to purify, detect, and target a specific polypeptide it recognizes, including both in vitro and in vivo diagnostic and therapeutic methods.

In one aspect, an immunoglobulin of the disclosure can be used in immunoassays for qualitatively and quantitatively measuring specific antigens in biological samples. Conventional methods for detecting antigen-antibody binding includes, for example, an enzyme-linked immunosorbent assay (ELISA), a radioimmunoassay (RIA) or tissue immunohistochemistry. Many methods may use a label bound to an antibody for detection purposes. The label used with the antibody is any detectable functionality (moiety) that does not interfere with its binding to antibody. Numerous labels are known, including the radioisotopes ³²P, ³²S, ¹⁴C, ¹²⁵I, ³H, and ¹³¹I, fluorophores such as rare earth chelates or fluorescein and its derivatives, rhodamine and its derivatives, dansyl, umbelliferone, luceriferases, e.g., firefly luciferase and bacterial luciferase (U.S. Pat. No. 4,737,456), luciferin, 2,3-dihydrophthalazinediones, horseradish peroxidase (HRP), alkaline phosphatase, beta-galactosidase, glucoamylase, lysozyme, saccharide oxidases, e.g., glucose oxidase, galactose oxidase, and glucose-6-phosphate dehydrogenase, heterocyclic oxidases such as uricase and xanthine oxidase, lactoperoxidase, biotin/avidin, spin labels, bacteriophage labels, stable free radicals, imaging radionuclides (such as Technecium) and the like. Production and detection of signal associated with certain labels can be direct, or indirect involving interactions between two or more interactive moieties, such as a ligand-receptor pair, enzyme-substrate pair and fluorescence resonance energy transfer pair.

Conventional methods are available to bind these labels covalently to the immunoglobulin polypeptides. For instance, coupling agents such as dialdehydes, carbodiimides, dimaleimides, bis-imidates, bis-diazotized benzidine, and the like may be used to tag the antibodies with the above-described fluorescent, chemiluminescent, and enzyme labels. See, for example, U.S. Pat. No. 3,940,475 (fluorimetry) and U.S. Pat. No. 3,645,090 (enzymes); Hunter et al. Nature 144: 945 (1962); David et al. Biochemistry 13:1014-1021 (1974); Pain et al. J. Immunol. Methods 40:219-230 (1981); and Nygren Histochem. and Cytochem 30:407-412 (1982). Preferred labels herein are enzymes such as horseradish peroxidase and alkaline phosphatase. The conjugation of such label, including the enzymes, to the immunoglobulin polypeptide is a standard manipulative procedure for one of ordinary skill in immunoassay techniques. See, for example, O'Sullivan et al., "Methods for the Preparation of Enzyme-antibody Conjugates for Use in Enzyme Immunoassay," in Methods in Enzymology, ed. J. J. Langone and H. Van Vunakis, Vol. 73 (Academic Press, New York, N.Y., 1981), pp. 147-166. Such bonding methods are suitable for use with the immunoglobulin polypeptides of this disclosure.

Alternative to labeling the immunoglobulin, antigen can be assayed in biological fluids by a competition immunoassay utilizing a competing antigen standard labeled with a detectable substance and an unlabeled antibody. In this assay, the biological sample, the labeled antigen standards and the antibody are combined and the amount of labeled antigen standard bound to the unlabeled antibody is determined. The amount of tested antigen in the biological sample is inversely proportional to the amount of labeled antigen standard bound to the antibody.

An immunoglobulin of the disclosure may be used as an affinity purification agent. In this process, the immunoglobulin polypeptide is immobilized on a solid phase such as Sephadex resin or filter paper, using methods well known in the art. The immobilized immunoglobulin is contacted with a sample containing the antigen to be purified, and thereafter the support is washed with a suitable solvent that will remove substantially all the material in the sample except the antigen to be purified, which is bound to the immobilized antibody. Finally, the support is washed with another suitable solvent, such as glycine buffer, pH 5.0, that will release the antigen from the immunoglobulin.

The immunoglobulins of the disclosure can be used as an antagonist to partially or fully block the specific antigen activity both in vitro and in vivo. Moreover, at least some of the immunoglobulins of the disclosure can neutralize antigen activity from other species. Accordingly, the antibodies of the disclosure can be used to inhibit a specific antigen activity, e.g., in a cell culture containing the antigen, in human subjects or in other mammalian subjects having the antigen with which an antibody of the invention cross-reacts (e.g. chimpanzee, baboon, marmoset, cynomolgus and rhesus, pig or mouse). In one embodiment, the immunoglobulin of the disclosure can be used for inhibiting antigen activities by contacting the immunoglobulin with the antigen such that antigen activity is inhibited. Preferably, the antigen is a human protein molecule.

In another aspect, an immunoglobulin of the disclosure can be used in a method for inhibiting an antigen in a subject suffering from a disorder in which the antigen activity is detrimental, comprising administering to the subject an immunoglobulin of the invention such that the antigen activity in the subject is inhibited. Preferably, the antigen is a human protein molecule and the subject is a human subject. Alternatively, the subject can be a mammal expressing the antigen with which an antibody of the invention binds. Still further the subject can be a mammal into which the antigen has been introduced (e.g., by administration of the antigen or by expression of an antigen transgene). An immunoglobulin of the disclosure can be administered to a human subject for therapeutic purposes. Moreover, an immunoglobulin of the disclosure can be administered to a non-human mammal expressing an antigen with which the immunoglobulin cross-reacts (e.g., a primate, pig or mouse) for veterinary purposes or as an animal model of human disease. Regarding the latter, such animal models may be useful for evaluating the therapeutic efficacy of antibodies of the invention (e.g., testing of dosages and time courses of administration). Blocking antibodies of the disclosure that are therapeutically useful include, for example but not limited to, anti-VEGF, anti-IgE, anti-CD11, anti-interferon and anti-tissue factor antibodies. The immunoglobulins of the disclosure can be used to diagnose, treat, inhibit or prevent diseases, disorders or conditions associated with abnormal expression and/or activity of one or more antigen molecules, including but not limited to malignant and benign tumors; non-leukemias and lymphoid malignancies; neuronal, glial, astrocytal, hypothalamic and other glandular, macrophagal, epithelial, stromal and blastocoelic disorders; and inflammatory, angiogenic and immunologic disorders.

In one aspect, a blocking antibody of the disclosure is specific to a ligand antigen, and inhibits the antigen activity by blocking or interfering with the ligand-receptor interaction involving the ligand antigen, thereby inhibiting the corresponding signal pathway and other molecular or cellular events. The disclosure also features receptor-specific antibodies which do not necessarily prevent ligand binding but interfere with receptor activation, thereby inhibiting any responses that would normally be initiated by the ligand binding. The disclosure also encompasses antibodies that either preferably or exclusively bind to ligand-receptor complexes. An immunoglobulin of the disclosure can also act as an agonist of a particular antigen receptor, thereby potentiating, enhancing or activating either all or partial activities of the ligand-mediated receptor activation.

In certain aspects, an immunoconjugate comprising an immunoglobulin conjugated with a cytotoxic agent is administered to the patient. Preferably, the immunoconjugate and/or antigen to which it is bound is/are internalized by the cell, resulting in increased therapeutic efficacy of the immunoconjugate in killing the target cell to which it binds. In one aspect, the cytotoxic agent targets or interferes with nucleic acid in the target cell. Examples of such cytotoxic agents include any of the chemotherapeutic agents noted herein (such as a maytansinoid or a calicheamicin), a radioactive isotope, or a ribonuclease or a DNA endonuclease.

Modified antibodies of the present disclosure can be used either alone or in combination with other compositions in a therapy. For instance, an antibody of the disclosure may be co-administered with another antibody, chemotherapeutic agent(s) (including cocktails of chemotherapeutic agents), other cytotoxic agent(s), anti-angiogenic agent(s), cytokines, and/or growth inhibitory agent(s). Where an antibody of the disclosure inhibits tumor growth, it may be particularly desirable to combine it with one or more other therapeutic agent(s) which also inhibits tumor growth. For instance, anti-VEGF antibodies blocking VEGF activities may be combined with anti-ErbB antibodies (e.g. HERCEPTIN^{™} anti-HER2 antibody) in a treatment of metastatic breast cancer. Alternatively, or additionally, the patient may receive combined radiation therapy (e.g. external beam irradiation or therapy with a radioactive labeled agent, such as an antibody). Such combined therapies noted above include combined administration (where the two or more agents are included in the same or separate formulations), and separate administration, in which case, administration of the immunoglobulin of the disclosure can occur prior to, and/or following, administration of the adjunct therapy or therapies.

The immunoglobulin of the disclosure (and adjunct therapeutic agent) is/are administered by any suitable means, including parenteral, subcutaneous, intraperitoneal, intrapulmonary, and intranasal, and, if desired for local treatment, intralesional administration. Parenteral infusions include intramuscular, intravenous, intraarterial, intraperitoneal, or subcutaneous administration. In addition, the immunoglobulin is suitably administered by pulse infusion, particularly with declining doses of the antibody. Preferably the dosing is given by injections, most preferably intravenous or subcutaneous injections, depending in part on whether the administration is brief or chronic.

The immunoglobulin composition of the disclosure will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The immunoglobulin need not be, but is optionally formulated with one or more agents currently used to prevent or treat the disorder in question. The effective amount of such other agents depends on the amount of immunoglobulins of the disclosure present in the formulation, the type of disorder or treatment, and other factors discussed above. These are generally used in the same dosages and with administration routes as used hereinbefore or about from 1 to 99% of the heretofore employed dosages.

For the prevention or treatment of disease, the appropriate dosage of an immunoglobulin of the disclosure (when used alone or in combination with other agents such as chemotherapeutic agents) will depend on the type of disease to be treated, the type of antibody, the severity and course of the disease, whether the immunoglobulin is administered for preventive or therapeutic purposes, previous therapy, the patient's clinical history and response to the immunoglobulin, and the discretion of the attending physician. The immunoglobulin is suitably administered to the patient at one time or over a series of treatments. Depending on the type and severity of the disease, about 1 µg/kg to 15 mg/kg (e.g. 0.1 mg/kg-10 mg/kg) of immunoglobulin is an initial candidate dosage for administration to the patient, whether, for example, by one or more separate administrations, or by continuous infusion. A typical daily dosage might range from about 1 µg/kg to 100 mg/kg or more, depending on the factors mentioned above. For repeated administrations over several days or longer, depending on the condition, the treatment is sustained until a desired suppression of disease symptoms occurs. The preferred dosage of the antibody or antibody fragment will be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, e.g. about six doses of the antibody). An initial higher loading dose, followed by one or more lower doses may be administered. An exemplary dosing regimen comprises administering an initial loading dose of about 4 mg/kg, followed by a weekly maintenance dose of about 2 mg/kg of the immunoglobulin. However, other dosage regimens may be useful. The progress of this therapy is easily monitored by conventional techniques and assays.

### Articles of Manufacture

Described, but not part the invention is an article of manufacture containing materials useful for the treatment of the disorders described above is provided. The article of manufacture comprises a container and a label or package insert on or associated with the container. Suitable containers include, for example, bottles, vials, syringes, etc. The containers may be formed from a variety of materials such as glass or plastic. The container holds a composition which is effective for treating the condition and may have a sterile access port (for example the container may be an intravenous solution bag or a vial having a stopper pierceable by a hypodermic injection needle). At least one active agent in the composition is an immunoglobulin of the disclosure.

The label or package insert indicates that the composition is used for treating the condition of choice, such as cancer. Moreover, the article of manufacture may comprise (a) a first container with a composition contained therein, wherein the composition comprises an immunoglobulin of the disclosure; and (b) a second container with a composition contained therein, wherein the composition comprises a further cytotoxic agent. The article of manufacture in this aspect of the disclosure may further comprise a package insert indicating that the first and second immunoglobulin compositions can be used to treat cancer. Alternatively, or additionally, the article of manufacture may further comprise a second (or third) container comprising a pharmaceutically-acceptable buffer, such as bacteriostatic water for injection (BWFI), phosphate-buffered saline, Ringer's solution and dextrose solution. It may further include other materials desirable from a commercial and user standpoint, including other buffers, diluents, filters, needles, and syringes.

### EXAMPLES

The following examples are included to demonstrate some embodiments of the invention. It should be appreciated by those of skill in the art that the techniques disclosed in the examples which follow represent techniques discovered by the inventors to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the scope of the invention.

The following Examples provide methods of modifying antibodies to produce structurally homogenous populations of recombinant antibody molecules.

### EXAMPLE 1

### MODELING OF IGG2 C_{H}1 AND HINGE PROXIMITY

A three-dimensional model of an IgG2 antibody was created to study the location of disulfide bonds in wild-type antibody molecules. This model illustrated the close spatial proximity of the IgG2 C_{H}1 Cys residue (Cys-131) with the hinge residues Cys-219 and Cys-220 and the inter-chain disulfide bonding light chain Cys residue (Cys-214). The three-dimensional model of an IgG2 antibody was constructed as follows:

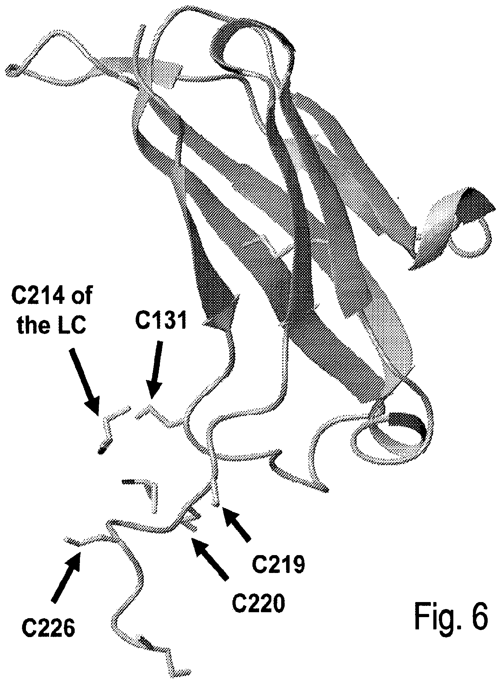

The C_{H}1 amino acid sequence of human IgG2 monoclonal antibody anti-EGFr (as described in U.S. Patent No. 6,235,883, incorporated herein by reference in its entirety) was modeled against the known C_{H}1 structure of an IgG4 antibody (pdb accession code 1ad9; Banfield, M.J. and Brady, R.L. (1997) Proteins 29: 161-171). The amino acid sequence identity between the C_{H}1 sequence of anti-EGFr and the template was 96% and the positions and identity of all Cys residues were absolutely conserved. The resulting IgG2 C_{H}1 model was superposed onto the C_{H}1 structure of a human IgG1 that includes the hinge region (pdb accession code lhzh; Saphire, E.O., et al., (2001) Science 293: 1155-1159). The IgG2 C_{H}1 model was then computationally fused to the IgG1 structure at the end of the C-terminal β-strand of the C_{H}1 domain to produce a pseudo-IgG2 C_{H}1 and hinge. The amino acids remaining from the IgG1 structure were then computationally mutated to their corresponding residues in the anti-EGFr sequence to produce the final IgG2 C_{H}1 and hinge illustration. The positions of the LC Cys residue from an IgG1 or IgG4 were taken from the 1hzh and 1ad9 structures respectively; it is noteworthy that the constant light chain domain of 1ad9 is identical to that of anti-EGFr and the constant light chain domain of 1hzh differs from anti-EGFr at a single residue distant from Cys-214. The light chain Cys residues shown in Figure 6 differ in their orientation. However, flexibility at this residue is expected since it is the last amino acid in the light chain. Three dimensional modeling was performed using MOE (Chemical Computing Group, Montreal, Quebec, Canada), and Figure 6 was produced using the PyMOL Molecular Graphics System (DeLano Scientific, San Carlos, CA).

Thus, the aforementioned antibody sequences were useful to produce a three-dimensional model of an IgG2 antibody as shown in Figure 6. In this model, four cysteines, at positions (HC)131, (HC)219, (HC)220 and (LC)214 are in close spatial proximity (Figure 6). This supports the discovery that these cysteine residues produce alternative disulfide linkages and modifications of these cysteine residues can generate structural isoforms of an IgG2 antibody.

### EXAMPLE 2

### MUTATION OF MONOCLONAL ANTIBODY ANTI-RANKL

Anti-RANKL is a human monoclonal IgG2 antibody directed against Receptor Activator of Nuclear Factor Kappa B Ligand (RANK-L). Anti-RANKL is composed of two HCs and two LCs that are linked through intermolecular disulfide bonds. Each HC is encoded to yield 448 amino acids and each LC, which belongs to the kappa subtype, consists of 215 amino acids with a Cys residue at the C-terminus. As discussed above, human IgG2 antibodies in solution exhibit heterogeneity among the inter-chain disulfide bridges.

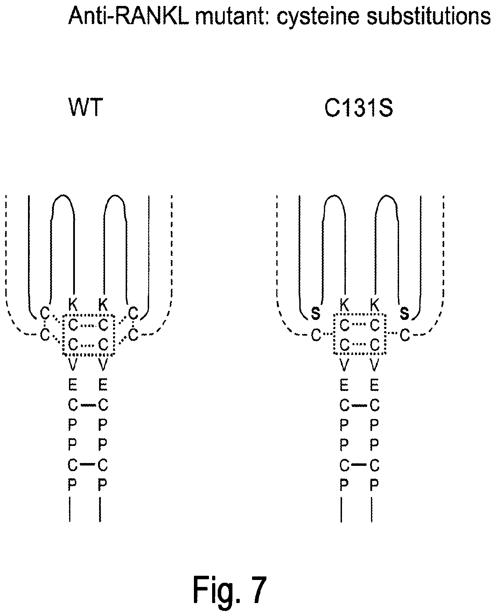

In order to simplify the disulfide connectivity among the C_{L}, C_{H}1 domains and the hinge, a mutant form of anti-RANKL was produced. In this mutant form, the third Cys residue in both of the heavy chains, Cys131, was replaced with a serine residue (Figure 7) to make as C131S mutant. Briefly, DNA encoding wild-type anti-RANKL was obtained as described in WO 03/002713. The anti-RANKL C131S mutant was then constructed using well-known methods by site-directed mutagenesis. The C131S mutant was then expressed in CHO cells by standard methods. The secreted antibodies were then purified. The final product concentration was 3.8 mg/mL. Wild type antibody was obtained at 31.9 mg/mL and at 61.2 mg/mL. Both the wild-type and mutant anti-RANKL protein were formulated with 10 mM sodium acetate, 5% sorbitol, pH 5.2.

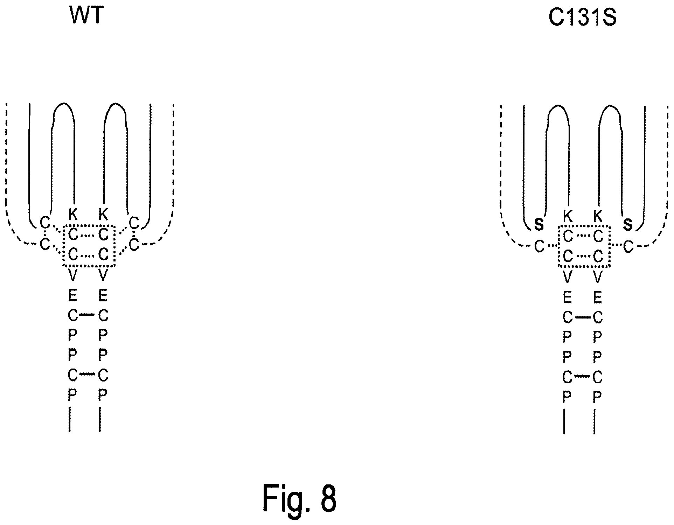

Figure 8 shows the predicted disulfide connectivity of the main IgG₂ - A structural isoform wild-type heavy and light chains of the anti-RANKL antibody. The structural analysis in the previous examples demonstrated that the LC is also able to link to the HC through the Cys residues of the hinge to form alternative structural isoforms. Thus, when replacing Cys131 of the HC with a Ser residue in the C131S form, it was predicted that the C-terminal Cys 214 of the LC would form a disulfide bond with one of the other Cys residues of the hinge of the HC (Cys 219, 220), thereby maintaining a four-chain disulfide linked antibody.

Because the light chains would no longer be able to create a disulfide bond at position 131 in the C131S mutant, the anti-RANKL (Cys131Ser) mutant was predicted to exhibit a more homogenous profile than wild-type anti-RANKL, which exists in multiple possible isoforms (see Figure 2). The following two examples describe the characterization of the structure and activity of the C131S mutant anti-RANKL antibody.

### EXAMPLE 3

### STRUCTURAL CHARACTERIZATION OF ANTI-RANKL MUTANT ANTIBODIES

The C131S mutant form of anti-RANKL was characterized side-by-side with the wild-type anti-RANKL using a variety of analytical techniques as described below (SE-HPLC, CEX-HPLC, CE-SDS, SDS-PAGE and RP-HPLC. The disulfide linkages of the mutant form were also characterized by a non-reduced peptide mapping technique.

### SE-HPLC of anti-RANKL Antibody

SE-HPLC is a reliable technique for determining molecular size or hydrodynamic volume of proteins under non-denaturing conditions. The SE-HPLC analysis of anti-RANKL was performed to determine its purity. The method utilized two Toso Hass TSK-GEL G3000SW_{XL} columns in series and 20 mM sodium phosphate, 250 mM NaCl, pH 7.0 as the mobile phase in order to distinguish monomeric anti-RANKL from aggregates and lower molecular weight clipped forms. The elution profile was monitored at 215 nm rather than 280 nm due to the low concentration (3.8 mg/mL) of the anti-RANKL Cys131Ser mutant. The results for the aggregate, Main Peak and clipped forms were expressed as relative area percentages.

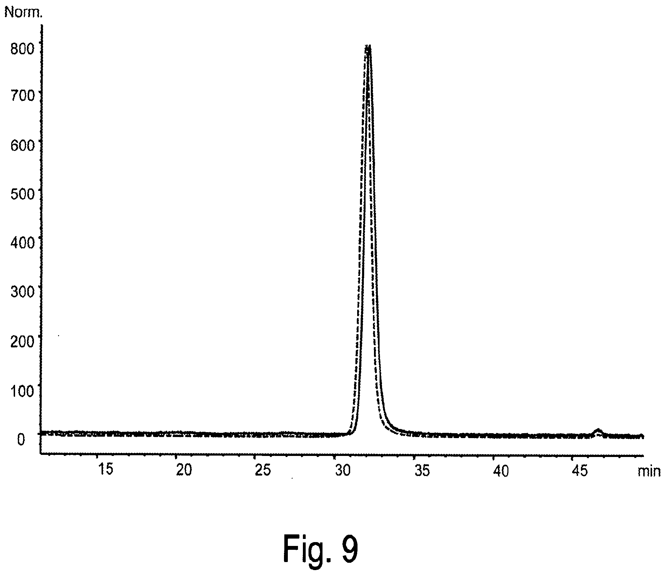

The results from the SE-HPLC analysis of the anti-RANKL (Cys131Ser) mutant are shown in Figure 9 as a chromatogram (dotted line) overlaid with the elution profile of the anti-RANKL wild-type protein (solid line). As shown in Figure 9, the elution profile of the mutant is dominated by one main peak and also has a minor aggregate peak. The integration results for the mutant are very comparable to the results obtained for the wild-type anti-RANKL. Despite the slight difference in elution positions between main peak of the anti-RANKL (cys131Ser) mutant and that of the wild-type, the SE-HPLC results fully support the conclusion that the mutant is composed of two HC and two LC polypeptide chains just as the anti-RANKL wild-type. The observed difference in elution of main peak is not sufficiently large to suggest a different stoichiometry of the HC and LC polypeptides.

### CEX-HPLC Analysis of anti-RANKL

The charge heterogeneity of anti-RANKL is typically evaluated using CEX-HPLC analysis. Various surface charge variants of anti-RANKL were separated by binding at pH 7.5 to a weak cation exchange column followed by elution employing a salt gradient. The method used a Dionex ProPac WCX 10 column at 40°C using 20 mM sodium phosphate at pH 7.5 when binding anti-RANKL and monitoring the profile at 215 nm during the elution with the NaCl containing gradient. The flowrate was 0.6 mL/min and the injected amount was 180 µg of protein. The method separated the acidic variants as Pre-Peaks and the basic variants as a specific Post Shoulder and Post Peaks. The results for the Pre-Peaks, Post Shoulder and Post Peaks as well as the Main Peak were expressed as area percentages. The linear gradient during elution was from 0 to 79 mM NaCl at a rate of approximately 1.25 mM NaCl per min.

An additional CEX-HPLC technique was used for the analysis of the anti-RANKL (Cys131Ser) mutant. Rather than mainly separating the IgG₂ subtype of antibodies based on modifications of the primary sequence that can result in charge variants, this method which employs a mobile phase at pH 5 partly separates the structural isoforms of the IgG₂ subtype described in Figure 8. The procedure used a Dionex ProPac WCX 10 column and a 20 mM sodium acetate buffer at pH 5. The protein was eluted with a NaCl gradient and was monitored at 215 nm. The characterization of the eluted peaks A, B, C and D was expressed as relative area percentages. The column temperature was ambient and the flow rate was 0.8 mL/min. The linear gradient for elution was from 100 to 175 mM NaCl at an increase of approximately 1.1 mM NaCl per min.

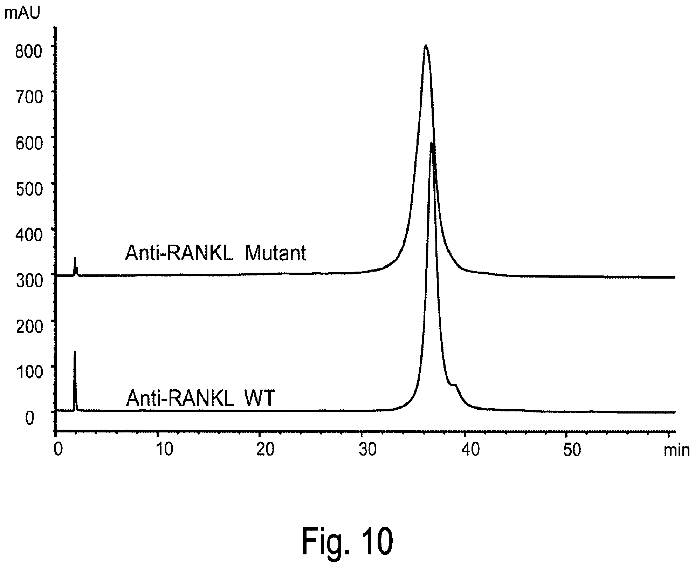

The CEX-HPLC analysis at pH 7.5 of anti-RANKL mutant and wild-type forms is shown in Figure 10. The anti-RANKL (Cys131Ser) elution chromatogram is shown on the top in comparison to the chromatogram for the wild-type shown on the bottom. The elution positions were comparable with the mutant eluting slightly earlier than the wild-type suggesting a slightly more acidic exposed surface. The late eluting shoulder at approximately 39 min for the wild-type is absent in the chromatogram of the mutant, which displays a somewhat broader but symmetrical peak. The shoulder has been characterized for anti-RANKL wild-type and was shown to be enriched in structural isoform IgG₂ - B2 (See Figure 2 for schematic details of the four possible structures for anti-RANKL). The IgG₂ - B2 structural isoform is not a possible structure when HC Cys131 is replaced by a Ser residue. Therefore it is not surprising that the late eluting shoulder was absent when analyzing the anti-RANKL (Cys 131 Ser) mutant by the CEX-HPLC pH 7.5 method.

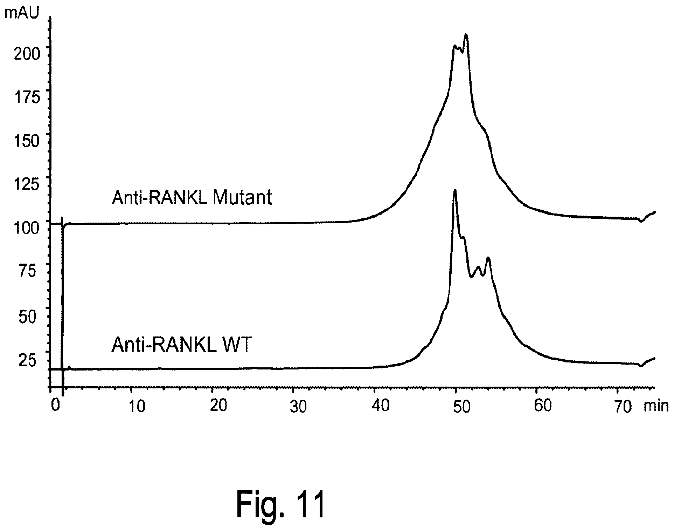

CEX-HPLC analysis at pH 5, which was used for partial purification and enrichment of the structural isoforms of wild-type anti-RANKL, was then employed to evaluate the profile of the anti-RANKL mutant. Figure 11 below shows the obtained profile for the mutant in red compared to the profile of anti-RANKL wild-type in blue.

### CE-SDS Analysis of anti-RANKL

CE-SDS was first performed under non-reducing conditions after treatment with SDS, heat at 70°C and iodoacetamide to block any exposed free thiol groups after the molecule is denatured. When anti-RANKL was incubated with SDS the protein exhibited an overall negative charge and migrated in an electrical field towards the anode. CE-SDS separates based on molecular size (hydrodynamic volume) after SDS and heat have denatured the protein. The analysis was performed using both non-reducing and reducing conditions. The analysis was performed on an Agilent HP^{3D} Capillary Electrophoresis system with UV/PDA detection. The capillary was a CE standard bare fused silica capillary and the CE-SDS sample and run buffer was supplied from Bio-Rad.

anti-RANKL migrated under non-reducing conditions as a heterogeneous split main peak identified as Peak 1A and Peak 1B. The split main peak is another measure of the disulfide mediated isoforms for the IgG₂ subtype. Under the non-reducing conditions the results were expressed as Pre-Peaks, Main Peaks (Peak 1A + Peak 1B) and Post Peaks in relative area percentages. anti-RANKL was reduced with 2-mercaptoethanol for the reduced CE-SDS. The elution profile shows a distinct LC and HC and the results reported as LC, HC and non-main in relative area percentages.

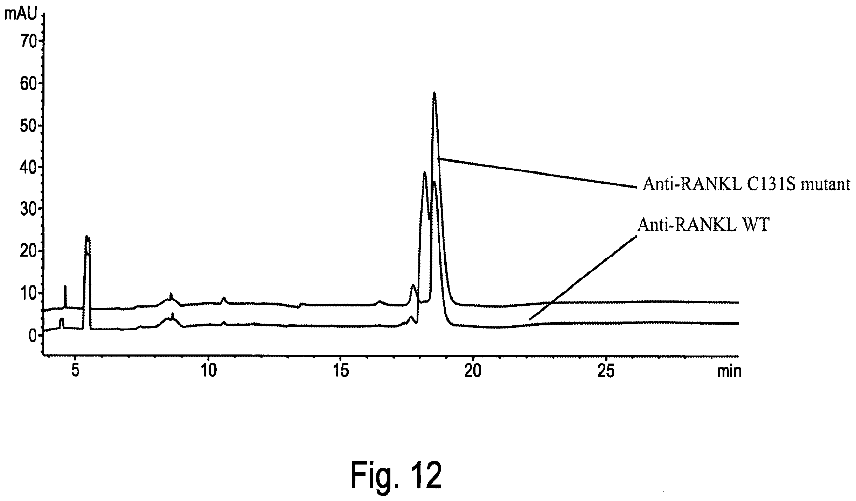

These results are shown in Figure 12, which displays the electropherogram of the mutant compared to the results obtained for the anti-RANKL wild-type. As seen in Figure 12, the electropherogram of the anti-RANKL mutant presented a symmetrical main peak which lacked the IgG₂ characteristic signature split which as expected was observed for the anti-RANKL wild-type. The lack of the split main peak for the mutant suggested that the disulfide structure is different between the two forms. The symmetrical peak of the mutant suggests that only one or a few structural isoforms exist.

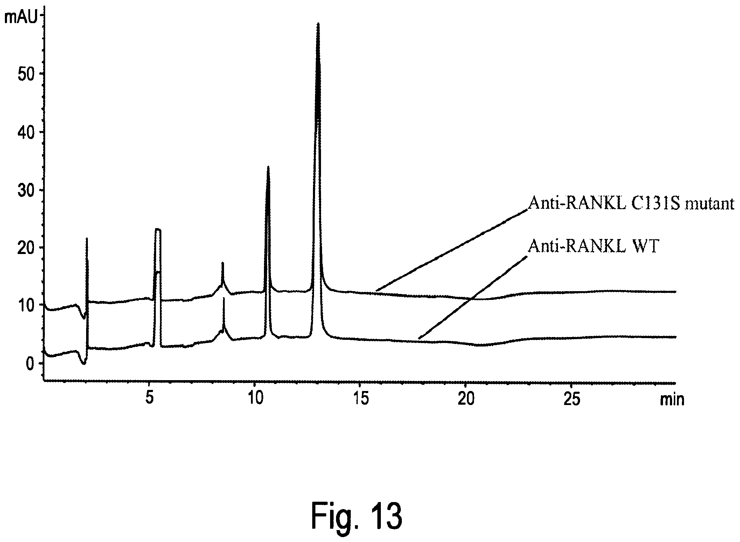

Next, CE-SDS was performed under reducing conditions after treatment with SDS, heat at 70°C and DTT to release the HC and LC. The resulting electropherogram of the mutant compared to the wild-type is shown in Figure 13.

The ratio of HC to LC for both the constructs exhibited a value of 2.1 which compared well to the theoretical value of 2.1, based on a calculation using average mass values for the HC and LC polypeptides. This result underscored that the mutant just as the wild-type was assembled with two HC and two LC polypeptide chains.

### SDS-PAGE Analysis of anti-RANKL

anti-RANKL (Cys131Ser) was analyzed side-by-side with anti-RANKL wild-type by non-reducing SDS-PAGE to monitor the size distribution of anti-RANKL and variants after SDS and heat denaturation. The method used a pre-cast 4-20% polyacrylamide gel under non-reducing conditions useful for detection of covalently linked multimers or potential fragments, which can be either molecules not completely assembled (lacking either one or two LC) or which can be fragmented by peptide bond cleavage. Free LC and HC may also be detected under non-reducing conditions. anti-RANKL migrated under non-reducing conditions as an intact molecule with an apparent molecular weight of approximately 150 kDa, at a position between the molecular weight markers of 116.25 and 200 kDa. Low levels of covalently linked dimer were also detected by this method.

Disulfide bonds can be disrupted with either 2-mercaptoethanol or dithiothreitol (DTT) to reduce anti-RANKL into its subunits: LC and HC. anti-RANKL was reduced by employing DTT as the reducing agent since minimal smearing and artifacts were observed when using this reagent. The HC and LC of anti-RANKL migrated with apparent molecular weights of approximately 50 and 26 kDa, respectively. Fragments produced by peptide bond cleavage were also detected under reducing conditions as well as under non-reducing conditions. Bio-Safe Coomassie Stain was used to visualize the peptide bands of anti-RANKL separated by SDS-PAGE.

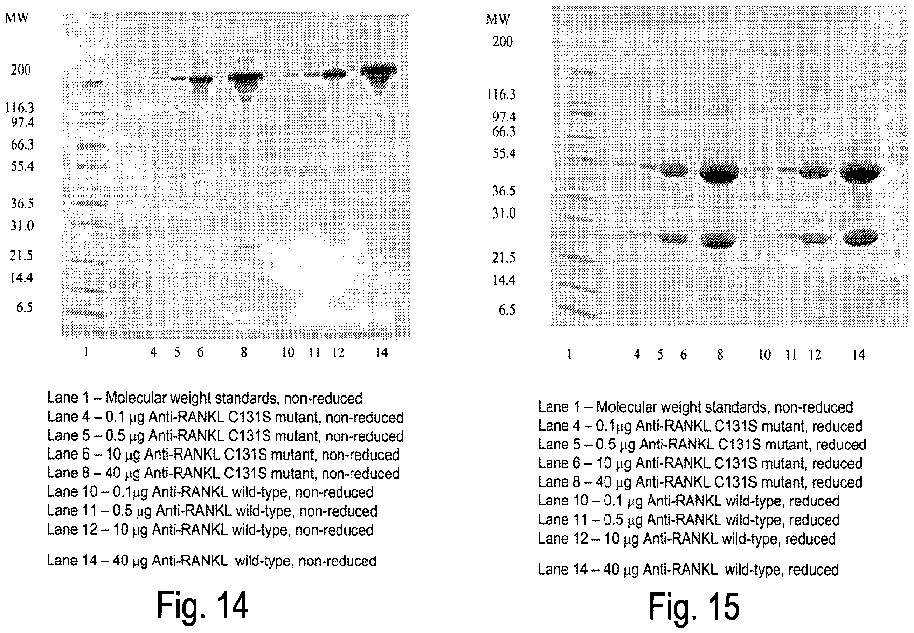

The results for non-reducing SDS-PAGE are shown in Figure 14. The results of the non-reduced gel indicate that the mutant construct displayed slightly higher levels of minor variants such as covalently linked aggregate which likely is dimeric in nature since the migration position indicated a size that is larger than the 200 kDa molecular weight standard. The mutant displayed a similar array of minor lower molecular forms compared to the wild-type anti-RANKL, albeit the levels of the minor forms are slightly higher in the sample representing the anti-RANKL (Cys131Ser) mutant.

The results for reducing SDS-PAGE analysis are shown in Figure 15, and show comparable band pattern for the anti-RANKL mutant and wild-type forms. The intensities of the minor bands in the gel varied slightly between the two constructs, however the variation is well within expected ranges from different preparations of one construct.

### RP-HPLC Analysis of anti-RANKL

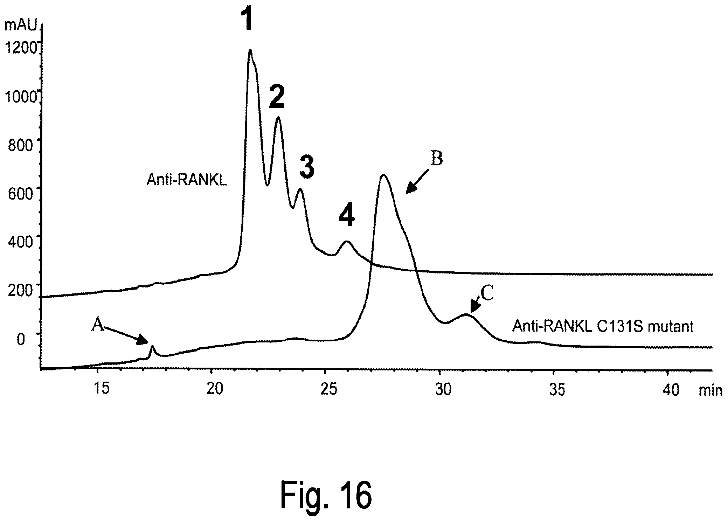

RP-HPLC was used to partly separate the structural isoforms inherent to the IgG₂ subclass. Samples were injected onto a Zorbax 300 SB C₈ column (5 µ, 2.1 × 150 mm) equilibrated in mobile phase A (0.1% TFA in 95/3.9/1.1 (v/v/v) H₂O/ n-Propanol/ACN) at 64°C, and eluted at a flow rate of 0.5 mL/min. The applied gradient for elution was mobile phase B (0.1% TFA in 10/70/20 (v/v/v) H₂O/ n-Propanol/ACN/) from 25 to 30% over a time period of 23 min and the eluted protein was monitored at 215 nm. Figure 16 shows the chromatogram of the anti-RANKL mutant compared to that of the wild-type. As shown, RP-HPLC demonstrated the increased hydrophobic nature of the mutant which eluted several minutes later than the wild-type construct. The well defined four peaks 1, 2, 3 and 4 which represent structural isoforms and were present in the wild-type, were clearly absent in the profile of the mutant.

### Peptide Mapping of anti-RANKL

The removal of Cys131 in the HC was expected to simplify the disulfide connectivity relating to the hinge region. The most direct way of testing for the disulfide connectivity was to perform peptide mapping under non-reducing conditions. Non-reduced peptide mapping using the endoproteinase Lys-C was applied to the mutant and wild-type anti-RANKL constructs. The peptide mapping of anti-RANKL was performed as outlined below for non-reducing conditions to establish the disulfide connectivity between the peptides containing Cys residues. The established non-reduced digest was also reduced by treatment with TCEP as outlined below to obtain a reduced map for the purpose of identifying the sequence.

Lys-C digestion. Three µL of sample at 30 - 60 mg/mL mixed with 7 µL of 8 M GuHCl, 0.1 M NaOAc, 20 mM NEM, pH 5.0 buffer was incubated at 37°C for 3 hour, followed by a 30-fold dilution into 4 M Urea, 20 mM NH₂OH, 0.1 M Sodium Phosphate, pH 7.0 buffer. The endoproteinase Lys-C was added at an enzyme to substrate ratio of 1: 10 (w/w) and the digestion was carried out at 37°C overnight. For the non-reduced peptide mapping analysis, TFA was added to the sample digests to a final concentration of 0.1% (v/v) to quench the reaction, and the peptides were analyzed directly by liquid chromatography/ electrospray ionization tandem mass spectrometry (LC/ESI-MS/MS). For the reduced peptide mapping analysis, TCEP was added to the digested samples to a final concentration of 10 mM and samples were incubated at room temperature for approximately 30 min to reduce the disulfide-linked peptides. TFA was added to a final concentration of 0.1% (v/v) before analysis.

On-line LC/ESI-MS/MS Peptide Analysis. The LC/ESI-MS/MS system was composed of an Agilent 1100 HPLC system and a ThermoFinnigan LCQ ion trap mass spectrometer equipped with an electrospray interface. The column used was a Vydac 214TP52 column (C-4, 300Å pore, 5µm particle size, 2.1mm id x 250mm length) set at 60°C. Mobile phase A was a H₂O:TFA mixture at a 1000:1 ratio (v/v), and Mobile phase B was an ACN: H₂O:TFA mixture at a 900:100:1 ratio (v/v/v). Peptide fragments were eluted at a flow rate of 0.2 mL/min using a linear gradient of 0 - 45% B over 90 min or 0 - 50% B over 120 min. The API source voltage of the LCQ was set at 4.5 kV, the N₂ pressure was 80 psi and the capillary temperature was set at 220 °C. The LC/ESI-MS/MS run was diverted at approximately 10 min to avoid salt contaminants entering the mass spectrometer. For the LC/ESI-MS/MS data acquisition program set up, three different scan modes (full scan, zoom scan and MS/MS scan) were performed. First, the most abundant peptide ion peak in a full scan (m/z 300 - 2000) was selected as the precursor ion. Second, a zoom scan was performed to determine the charged state of the precursor ion. Finally, an MS/MS scan was used to determine the sequence of the precursor ion using collision induced dissociation (CID) with relative collision energy of 35%.

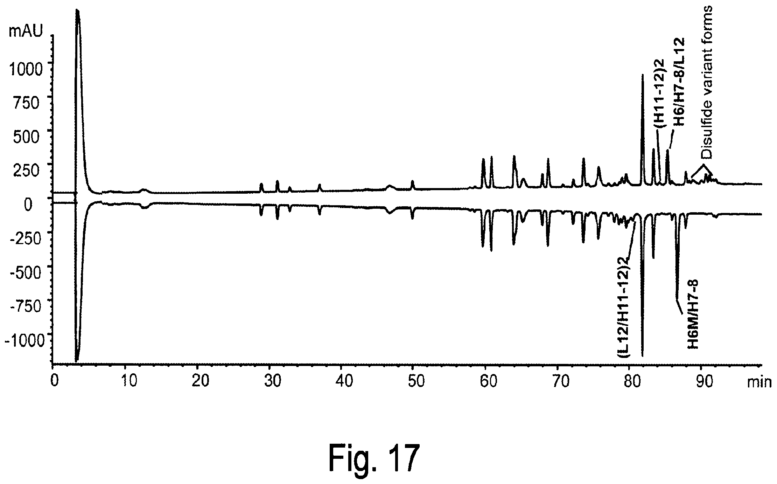

Figure 17 shows the UV chromatogram of a non-reducing Lys-C map of anti-RANKL wild-type (up) and anti-RANKL mutant (down). The most obvious difference between the two maps was that the map of the mutant did not contain the very hydrophobic late eluting peptides that distinguish the disulfide variant peptides involving the hinge (H10-11-12, H10-11 and H11) peptides and the H6/H7-8 peptide of the HC together with the L12 peptide from the LC; these peptides as expected were present in the map of the anti-RANKL wild-type. The disulfide-linked peptide that connects Cys 131 of HC and Cys 214 of LC; H6/H7-8/L12 and eluted at approximately 85 min, but as expected, was missing in the mutant. Instead, the H6(Cys131Ser)/H7-8 peptide (labeled as H6M/H7-8 in Figure 17), eluting at approximately 87 min, was present in the map of the anti-RANKL (Cysl31Ser) mutant. A peptide with a mass value of 6974 Da was observed in the map of the mutant at the elution position around 80 min; this peptide corresponded to disulfide linked peptide (L12-H11-12)₂ and showed linkage of LC to the hinge region of the HC.

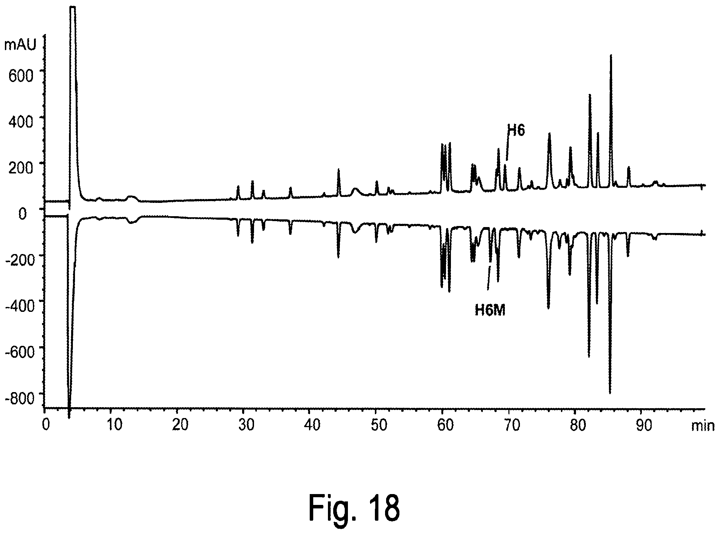

The reduced Lys-C peptide map is shown in Figure 18 with the anti-RANKL wild-type profile (up) and the anti-RANKL (Cys131Ser) mutant (down). The maps were almost identical with the exception of the well resolved H6 peptide at approximately 60 min that was present only in the map of the anti-RANKL wild-type and the corresponding H6M peptide which represents the H6Cys131Ser mutation at approximately 67 min, present only in the map of the anti-RANKL (Cys131Ser) mutant.

### Conclusions

The evaluation of the anti-RANKL (Cys 131 Ser) mutant by SE-HPLC, CE-SDS and SDS-PAGE confirmed that the constructed molecule is expressed and purified as a four polypeptide antibody containing two copies each of LC and HC which are held together by disulfide bonds. The elution position by SE-HPLC suggested a slightly larger hydrodynamic radius for the mutant when compared to the wild-type anti-RANKL. The profiles by CEX-HPLC at pH 7.5 and by non-reduced CE-SDS were more uniform and simple for the anti-RANKL (Cys131Ser) mutant than the profiles observed for the anti-RANKL wild-type. The non-reducing CE-SDS, the CEX-HPLC at pH 5 and the RP-HPLC analysis indicated a less complex array of disulfide mediated structural isoforms for the anti-RANKL (Cys131Ser) mutant than observed for the wild-type anti-RANKL, which represents a typical IgG₂ subtype antibody. Non-reduced peptide mapping showed that when Cys 131 of the HC is replaced by a Ser residue, the LC is connected to the hinge region of the HC through Cys 215 (Cys 214 in Edelman numbering) of the LC and one of the four Cys residues of the HC. Taken together, these results confirm that mutation of Cys 131 results in increased structural homogeneity in the IgG2 antibody.

### EXAMPLE 4

### POTENCY ANALYSIS OF ANTI-RANKL MUTANT ANTIBODIES

The mutant form of anti-RANKL was characterized side-by-side with the wild-type anti-RANKL to assess *in-vitro* potency by a homogenous time-resolved fluorescence (HTRF) assay. The potency of anti-RANKL is quantified by its ability to block the binding of RANK-L to its cognate receptor "Receptor Activator of Nuclear Factor kappa B" (RANK). A homogenous time-resolved fluorescence (HTRF) assay was utilized to provide quantitative results. Briefly, RANK-L was labeled with europium+3 (Eu+3), which emits light at 620 nm when excited with 337 nm light. The receptor, produced as a fusion protein using recombinant techniques, RANK-FLAG, was labeled with an anti-FLAG antibody linked to allophycocyanin (APC). APC is a fluorophore that emits 665 nm light when excited by light at 620 nm. Therefore, when Eu+3 labeled RANK-L binds to the RANK-FLAG/anti-FLAG-APC complex, the tertiary complex emits 635 nm light when excited with 337 nm light. When anti-RANKL was incubated with Eu+3 RANK-L and RANK-FLAG/anti-FLAG-APC, the fluorescence intensity at 665 nm decreased in a dose dependent fashion.

The potency of each test sample was compared to a Reference Standard and the data reported as relative potency as compared to the Reference Standard. The potency of the mutant anti-RANKL was assessed by the HTRF assay. The obtained results indicated that the mutant with 92% relative potency has comparable activity to the wild-type which showed 101% relative potency in the assay. The HTRF assay was performed once with three replicate sample preparations and the results are summarized in Table 3 below.

**Table 3. Potency by HTRF analysis of anti-RANKL mutant and wild-type**

| **anti-RANKL** | **Expected Protein Concentration (mg/mL)** | **Biological Active Protein Concentration (mg/mL)** | **Relative Potency Mean (%)** | **Relative Potency CV (%)** |
|---|---|---|---|---|
| Wild-type | 61.2 | 62.0 | 101 | 0 |
| Cys131Ser mutant | 3.8 | 3.5 | 92 | 1 |

These results demonstrate that the anti-RANKL (Cys131Ser) mutant displayed comparable activity to the wild-type form when tested *in-vitro* in the HTRF based potency assay.

### EXAMPLE 5

### MUTATION OF ANTI-IL-1R ANTIBODIES

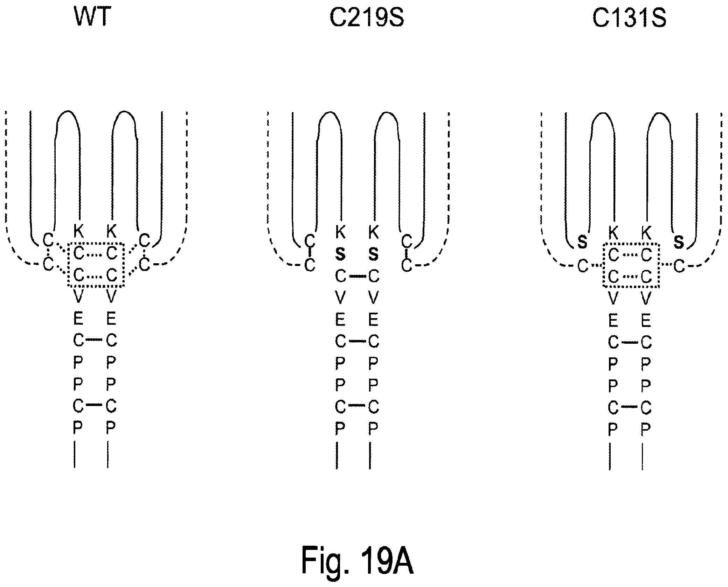

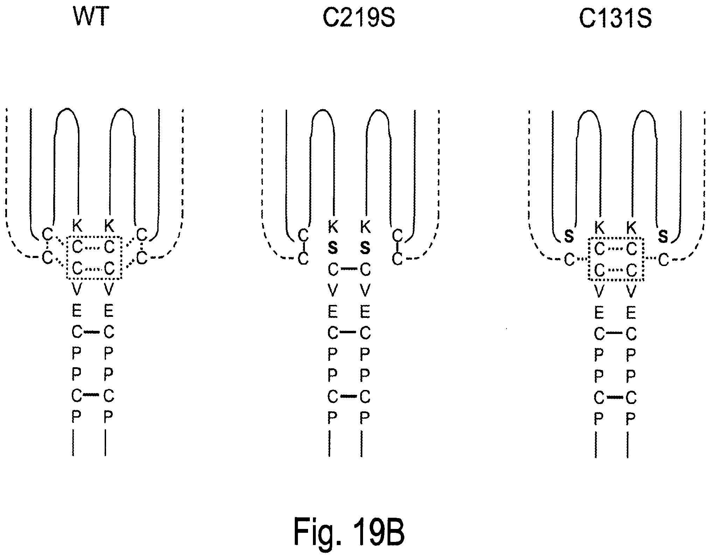

In order to further understand the effect of mutations to disulfide bond-forming cysteines in IgG2 antibodies, two mutant anti-IL-1R antibodies were generated and characterized as set forth below (see Figure 19A). The first mutant (C219S) was predicted to be enriched for disulfide structure form-3, where the LC disulfide bonding is forced to C131 (see Figure 19B). The second mutant (C131S) was predicted to be enriched for disulfide structure form-1, where the LC disulfide bonding is forced to the HC hinge region (see Figure 19B). The nucleotide and encoded amino acid sequences of anti-IL-IR is set forth in U.S. Patent Pub. No: 2004/097712, Varnum et al. C219S and C131S mutations were made using standard site-directed mutagenesis, as described in Example 2 above. The mutations were confirmed by DNA sequencing, and the mutated expression constructs were transiently transfected in COS cells. Secreted antibody was purified, and then characterized as described in the next two examples.

### EXAMPLE 6

### STRUCTURAL CHARACTERIZATION OF ANTI-IL-1R MUTANT ANTIBODIES

The C219S and C131S mutant antibodies generated in Example 5 were analyzed by RP-HPLC and compared to wild type anti-IL-1R from stably transfected CHO cells and transiently transfected CHO cells. The RP-HPLC procedure was performed as follows.

Briefly, an Agilent 1100 HPLC system with a binary pump was equipped with a UV detector and an Agilent 1100 Capillary HPLC system was connected on-line to a Micromass Q- TOF Micro mass spectrometer equipped with an electrospray ionization (ESI) source. The proteins were injected onto a Zorbax 300SB C8 column (150 × 2.1 mm, 5 µm) operated at 75°C. The flow rate was 0.5 mL/min. Mobile-phase A was water containing 0.1% TFA. Mobile-phase B was 70% isopropyl alcohol, 20% acetonitrile, and aqueous 0.1% TFA. Samples were injected at a loading condition of 10% B and increased to 19% B over 2 min. A linear elution gradient of 1.1% B/min started at 2 min and ended at 24 min. The column was then flushed for 5 min with 95% B. The column was re-equilibrated with the loading condition for 5 min. An Agilent 1100 HPLC system with a binary pump was used for standard analysis and an Agilent 1100 Capillary HPLC system was connected on-line to a Micromass Q-TOF Micro mass spectrometer equipped with an electrospray ionization (ESI) source.

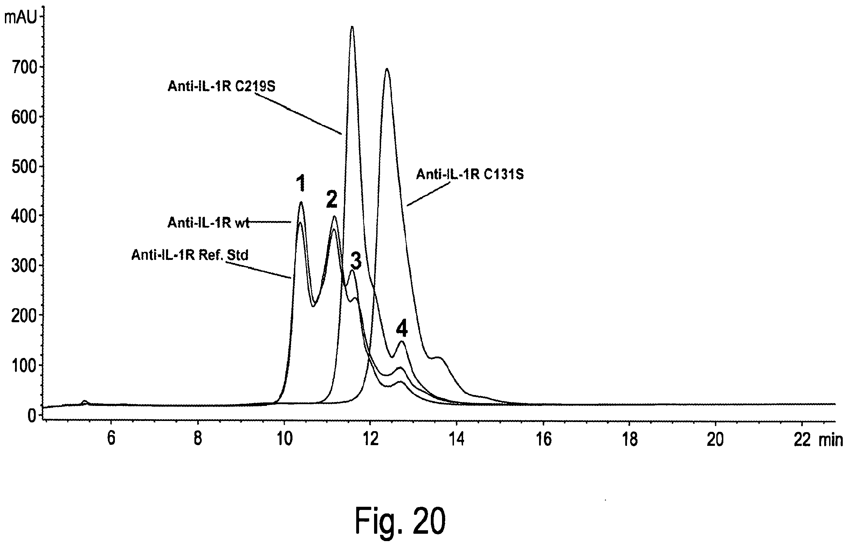

The results are shown in Figure 20. The chromatogram shows that although wild type anti-IL-1R separated into four peaks, C219S and C131S each eluted as a single peak, with different retention times. Specifically, the C219 and C131 mutants co-eluted with the later eluting forms of wild type IgG2, suggesting that the mutants posses the increased flexibility and increased potency similar to the later eluting forms of wild IgG2. The measured masses by an on-line mass spectrometer of the eluting isoforms agreed with the cysteine to serine substitutions. These data strongly suggest that mutation of either C219 or C131 results in enrichment of a single structural isoform.

### EXAMPLE 7

### POTENCY OF ANTI-IL-1R MUTANT ANTIBODIES

Potency of each anti-IL-1R mutant was compared to wild type using chondrocyte and whole blood bioassays as described below.

The antibody in these studies was designed to bind specifically to interleukin-1 cell surface receptor type 1 (IL-1RI); it competes with the interleukin-1 beta (IL-1b) ligand, thereby inhibiting IL-1 mediated cellular events, including the production of IL-6. Thus, the bioactivity of the antibody was assessed by monitoring the inhibition of IL-1b induced IL-6 production by primary human chondrocytes and human whole blood.

For the chondrocyte assay, the IL-I receptor mAb (anti-IL-1R) and the C219S and C131S mutants were serially diluted from 40 nM to 1.5256 pM in assay media. The diluted test antibodies (50 µL) were added to the wells 96-well plates seeded with human chondrocytes at a density of 10000 cells/well in a 100 µL volume. The final antibody concentration ranged from 10 nM to 0.3815 pM. After a 30 min incubation, 50 µL of recombinant human IL-1 beta was added to a final concentration of 10 pM. After incubation overnight, the antibody activities were analyzed using an IL-6 immunoassay with electrochemiluminescence detection (Meso Scale Discovery, Gaithersburg, MD). The inhibition of IL-6 production was calculated as a percentage of maximum IL-1 beta activity. The inhibition response curve for each test antibody was established and the corresponding IC50 values (the concentration of antibody which reduces the signal by 50%) were derived using GraphPad PRISM software.

For the whole blood assay, antibodies were evaluated in 50% human whole blood (final concentration) from 10 nM to 0.0003 nM in half-log increments for a 10-point IC50 curve. After a 45 minute pre-incubation with antibodies, the blood was stimulated with recombinant IL-1 beta for a final concentration of 30 pM (-IC50). After incubation overnight, the antibody activities were analyzed using an IL-6 immunoassay with electrochemiluminescence detection (Meso Scale Discovery, Gaithersburg, MD). The inhibition of IL-6 production was calculated as a percentage of maximum IL-1 beta activity. IC50 values were calculated (Graph Pad PRISM software) using six separate donors (3 donors on two different days).

Bioassay data were analyzed using GraphPad Prism Software. IC50 values were derived by non-linear regression (variable slope). Where dose-response curves were incomplete, the bottom was constrained to 0. Significance was calculated using a 1-way ANOVA with Tukey multiple comparison post-test. A P-value <0.05 was considered significant.

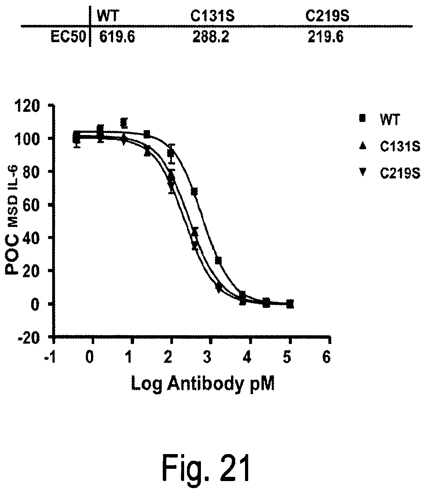

A representative result is shown in Figure 21. The results of repeated assays are summarized in Table 4 below. As shown in Figure 21 and Table 4, anti-IL-1R C131S and anti-IL-1R C219S consistently demonstrated 2-3 times greater potency compared to wild type in the inhibition of IL-1b induced IL-6 production by primary human chondrocytes and human whole blood.

**Table 4. Potency of Cysteine Mutants of anti-IL-1R**

| **Sample** | **Relative Potency (compared to WT CHO Stable)** |
|---|---|
| anti-IL-1R WT CS-9 CHO Stable | |
| anti-IL-1R C219S | X 2-3 |
| anti-IL-1R C131S | X 2-3 |

### EXAMPLE 8

### MUTATION OF ANTI-EGFR ANTIBODIES

As shown in Example 1, modeling of an IgG2 antibody sequence based on three-dimensional structure of antibodies positions the 4 cysteines, at positions (HC)131, 219, 220 and (LC)214, in close spatial proximity (Figure 6), suggesting that a variable arrangement of these residues could generate IgG2 structural isoforms. The possibility that mutation of the closely spaced cysteines could eliminate these forms was tested as follows.

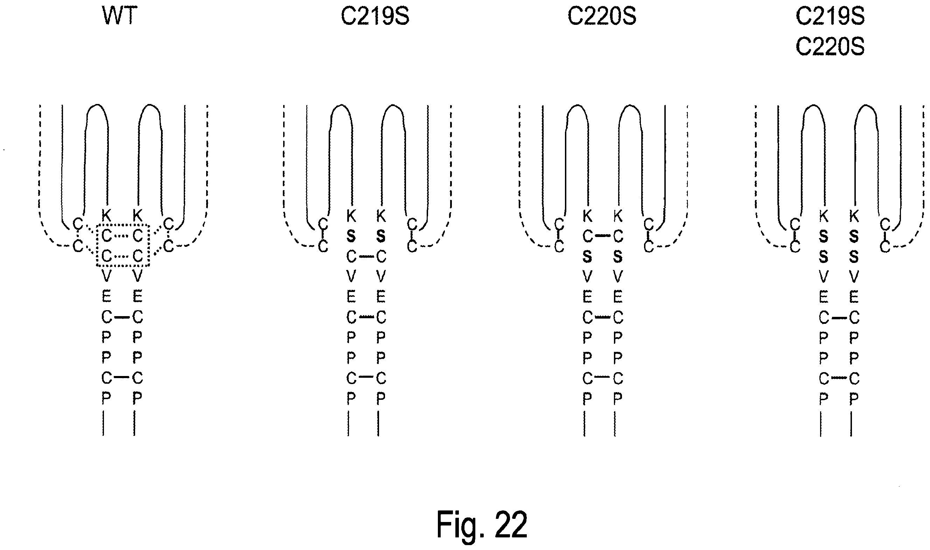

Two specific cysteine-to-serine mutants were prepared by site-directed mutagenesis at either position 219 or 220 (see Figure 22). Site directed mutagenesis was performed by using the QuickChange site-directed mutagenesis kit (Stratagene, La Jolla, CA) on vectors encoding fully human monoclonal antibody anti-EGFr (described as mAb E7.6.3 in U.S. Patent No. 6,235,883, and the presence of the desired mutations was confirmed by DNA sequencing. Expression plasmids with the desired mutation were stably transfected into suspension-adapted CHO cells deficient in dihydrofolate reductase activity. Cells expressing the antibody of interest were selected by growth in medium lacking glycine, hypoxanthine, and thymine. Following recovery from transfection and selection, CHO cell pools were grown in production spinner flasks. Expressed antibody was purified from harvested cell culture supernatant by Protein A affinity chromatography. Mutants C219S and C220S exhibited no significant difference in expression and purification characteristics when compared to wild type anti-EGFr.

### EXAMPLE 9

### CHARACTERIZATION OF STRUCTURE OF ANTI-EGFR MUTANT ANTIBODIES

The mutant anti-EGFr antibodies were then comparatively analyzed by non-reduced CE-SDS and native peptide mapping as follows.

### Evaluation of Disulfide Bond Heterogeneity of Mutant Antibodies

First, the apparent size of antibodies was evaluated by non-reduced capillary electrophoresis sodium dodecyl sulfate (nrCE-SDS), a gel sieving method performed on fully denatured molecules.

Briefly, antibody samples at a minimum concentration of 2.5 mg/ml were diluted to a final concentration of 1mg/ml according to the following procedure: 150 µg of antibody solution was combined to 10 µl of iodoacetamine (IAM), 3 µl of internal standard (10 kDa molecular weight marker, Beckman) were added and the solution was brought to 150 µl with CE-SDS sample buffer (BioRad). The solution was mixed, centrifuged and heated at 75°C in water bath for 10 min. The mixture was cooled at room temperature, centrifuged at 12,000 rpm for 6 min and transferred to sample vials for injection.

The nrCE-SDS analytical method was also performed on anti-EGFr molecules subjected to a refolding procedure using a cysteine / cystine redox potential, to determine whether disulfide bond arrangements were responsible for the distinct structural isoforms. To generate redox-refolded antibodies, native antibody samples were treated with 6 mM Cysteine and 0.6 mM Cystine in 0.2 M Tris-HCl at pH 8.6 for 96 hours at 2-8°C. Samples were then dialyzed with their formulation buffers. Human IgG2 from human myeloma was dialyzed into its original formulation buffer, 40 mM phosphate and 150 mM NaCl, pH 7.4. Refolded samples were then subjected to nrCE-SDS as described above. The results are shown in (Figure 23). Figure 23A shows that wild-type anti-EGFr exists in two structural isoforms, represented by two peaks in nrCD-SDS. Treatment of wild-type anti-EGFr with a redox potential modified the structural isoforms, resulting in a significant decrease of isoform 1 (40% to 12%) concomitant with an increase of isoform 2 (60% to 88%). This result confirms that the structural isoforms are distinct based on disulfide bond arrangements. Similar results were obtained for a commercially available IgG2 molecule isolated from a myeloma patient (Figure 23B). In contrast, mutant anti-EGFr lost the typical IgG2 doublet and was resolved in a single peak by nrCE-SDS (Figure 23C). Thus, the structural isoforms were shown to be unrelated to glycosylation but dependent on the presence of the complete covalent structure of the form (HC-LC)₂.

### Peptide Mapping

The disulfide structures of wild-type and C219S anti-EGFr were investigated by non-reduced peptide mapping. Free cysteines were alkylated with N-ethylmaleimide (NEM) at acidic pH as described by (Bures et al, 1998, Biochemistry 37:12172). After NEM alkylation, the solution was buffer exchanged and an enzymatic digest was performed on the non-reduced molecule. Briefly, the NEM labeled material was digested with 10% w/w Trypsin in 0.1M Tris/2M Urea pH 8.3 at a concentration of lmg/mL for 4 hours at 37°C. The digest reaction was quenched with the addition of 10µL of 10% TFA. The trypsin digested sample was then analyzed via RP-HPLC with column heating at 60°C. Mobile phases consisted of (A) 0.12% TFA in water (w:v), and (B) 0.11% TFA in 40:40:20 acetonitrile:isopropanol:water (w:v). Separation was performed on a Jupiter C5 (2.1 × 250mm) column, conditioned in mobile phase A for 20 minutes. 100µg of digest was injected and the peptide fragments eluted at a flow rate of 0.2 mL/min with a gradient of 0-65% B inl65 min. Peptide elution was monitored by UV absorbance at 214 nm and on-line mass acquisition.

Disulfide pair assignments were made by identification of bridged peptides using mass analysis. All mass analyses were performed using an LCQ Deca ion-trap instrument (Thermo-Finnigan, San Jose, CA) equipped with an electrospray source connected to an Agilent 1100 pump (Agilent, USA). Analysis was carried out in a positive ion mode using a spray voltage of 5.0kV and the MS capillary temperature was maintained at 225°C. The instrument was calibrated in the m/z range of 500 to 2000 using a mixture of caffeine, MRFA peptide and Ultramark 1621. Deconvolution of electrospray ionization mass spectra for the heavy and light chain was done using ProMass for Xcalibur software. Spectral data for the protein digests were acquired on-line in the range 200-2000 m/z. MS/MS analysis was performed in data dependent mode. Collision data were obtained using 40% relative collision energy.

The results are shown in Figure 24, and demonstrate that native tryptic peptide mapping of the mutants showed quantitative recovery of the hinge dimer and the H₁₀-L₁₈ heteropeptide expected from the conventional inter-chain connection C_{H}1-C_{L}. (Figure 24).

In conclusion, when analyzed by nrCE-SDS and native peptide mapping, the mutant molecules behaved differently than wild type. These results indicate that mutation of a single cysteine residue specifically abolished the IgG2 structural isoforms and generated a solution having antibodies with a homogenous structure.

### EXAMPLE 10

### CHARACTERIZATION OF BIOLOGICAL ACTIVITY OF ANTI-EGFR MUTANT ANTIBODIES

The potency of mutants C219S, C220S and wild type anti-EGFr is compared by EGFr ligand and phosphorylation bioassays, performed as follows. Anti-EGFr is a fully human recombinant IgG2 monoclonal antibody directed against the human epidermal growth factor receptor (EGFr). Thus, potency is measured by the ability of the antibody to inhibit ligand-receptor binding at the cell surface therefore preventing ligand-induced activation of a signaling pathway. Two potency assays are used measuring either blockage of the EGF-meditated autophosphorylation of EGFr, or inhibition of cell proliferation by reporter gene expression assay. To measure inhibition of EGFr autophosphorylation, ninety-six well tissue culture plates are seeded with A431 cells. Serial dilutions of reference standard and test samples are made to give a concentration range from 0.1125 mg/ml to 0.02 µg/ml. Fifty µl of each serial dilution is added to the tissue culture plate containing the A431 cells. To each cell is also added 50 µl of rhEGF diluted to the working dilution range. The plate is incubated for 60 minutes at 37C and 10% CO₂. The supernatant is discarded and the cells lysed with 105 µl of buffer (10 mM Tris, 150 mM sodium chloride, 5 mM EDTA, 1% Triton X-100) per well. The plate is incubated at 2 to 8°C for 60 minutes. Eighty-five µl of the cell lysate is added to an ELISA plate coated with E752 EGF antibody. The plate is incubated for 90 minutes at room temperature and in the dark. The plate is then washed and 100 µl of HRP conjugated detection antibody is added to each well. The plate is incubated for 60 minutes and then washed prior to the addition of 100 µl of substrate. The plate is developed for 15 to 25 minutes. The development is stopped by adding 50 µl of 2M H₂SO₄ and the plate is read at 450 and 650 nm. The potency of each test sample is calculated from triplicates and the mean value is reported.

The gene expression assay is performed using an engineered cell line that expresses the human EGFr and a luciferase reporter gene construct responsive to EGFr signal transduction. Anti-EGFr competes with TGF-α, for binding to the EGFr and causes a dose dependent inhibition of luciferase production. Luciferase production is measured by luminescence after cell lysis and addition of luciferin. Reference standard, control and test samples are diluted to 440 ng/ml in assay medium. Serial dilutions to a concentration range of 400 to 87 ng/ml are prepared. An equal volume of fixed concentration of TGF-α is added to each tube. The final concentration in the assay wells is 110 to 22 ng/ml of anti-EGFr after addition of cells. Cells grown in suspension are added to four 96-well plates and 25 µl of each serial dilution of anti-EGFr. A typical dose response shows a sigmoidal response with an IC50 value of approximately 87 ng/ml. The signal response is measured and the potency of the isoforms is determined by comparing the sample's response to that of a reference standard.

As indicated by both bioassays, both mutants have similar potency as wild type in these assays.

### EXAMPLE 11

### ELIMINATION OF DISULFIDE HETEROGENEITY BY INSERTION MUTATION IN THE HINGE REGION OF IGG2 ANTIBODIES

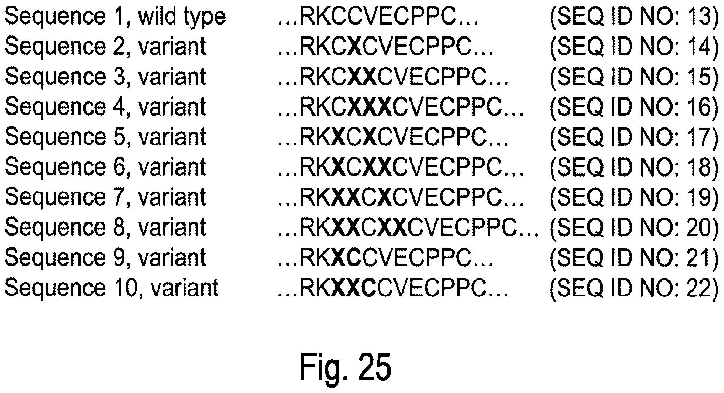

As discussed above, human IgG2 antibodies have structural variants due to the disulfide heterogeneity at the hinge. The disulfide heterogeneity is induced by a reactive and unique cysteine219-cysteine220 (C219C220) motif in the hinge, which occurs only in heavy chains of IgG2 antibodies. In this example, the disulfide heterogeneity is eliminated by inserting one, two, or more amino acid residues between or above residues C219 and C220 of the heavy chain as set forth in Figure 25.

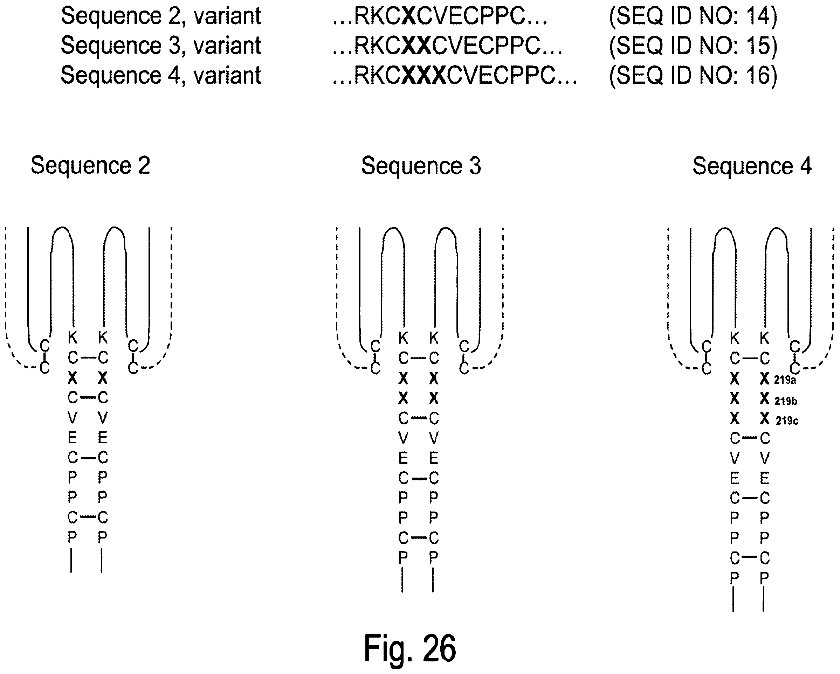

The insertion of one or more amino acids between or above the two tandem cysteines (anti-IL-1R heavy chain amino acid numbers 219 & 220) in the hinge of a human IgG2 is designed to lower the reactivity of C219 and/or C220 in the upper hinge region by separating the two disulfide bonds or elongating the hinge section and therefore forcing the production of a homogeneous disulfide form. The expected disulfide form produced from these mutants is Form 3, with LC C214 forming a disulfide bond with HC C131. This is done without mutating native cysteine residues (Figures 26-28). The inserted residues are selected from the list including glycine, proline, serine and other amino acid residues.

The insertion mutations set forth in Figures 25-28 are generated using site-directed insertion mutagenesis. Different versions of sequences 2-4 are generated. In one version of sequence 2, a single glycine is inserted between C219 and C220. In another version of sequence 2, a single proline is inserted between the C219 and C220. In different versions of sequences 3 and 4, either two or three glycines, two or three prolines, or a combination of two or three glycines or prolines is inserted between C219 and C220, as set forth in Figure 26.

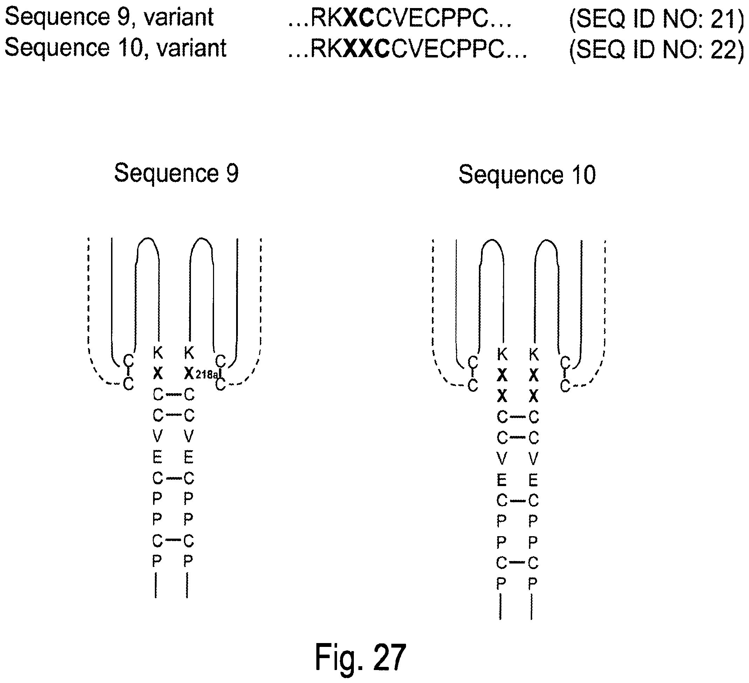

Different versions of sequence 9 and 10 are also generated by insertion mutagenesis, set forth in Figure 27. In one version of sequence 9, a single glycine is inserted between K218 and C219. In another version of sequence 9, a single proline is inserted between K218 and C219. In different versions of sequence 10, either two glycines, two prolines, or a combination of one glycine and one proline is inserted between K218 and C219.

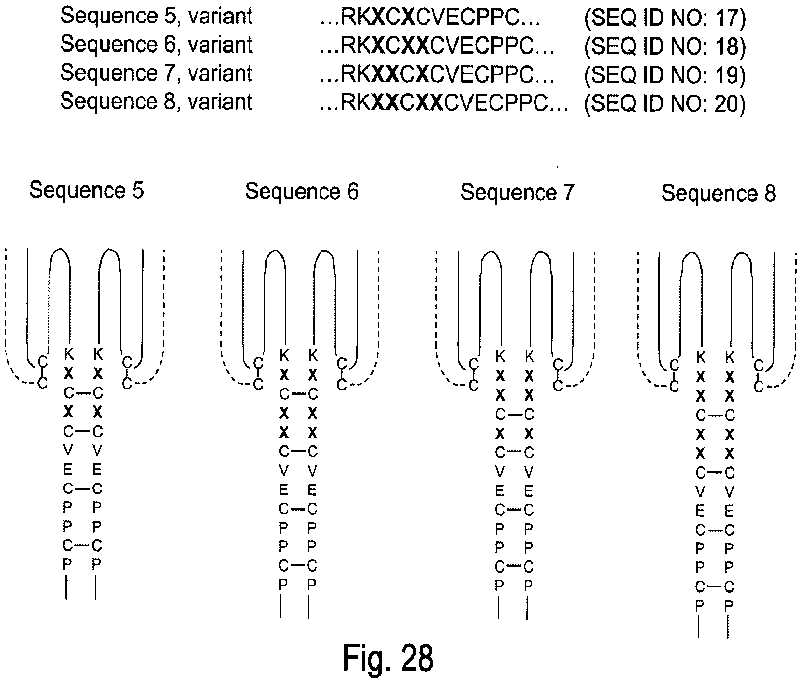

Different versions of sequences 5-8 are also generated by insertion mutagenesis, as set forth in Figure 28. As done with sequences 1-4 and 9-10, different combinations of glycines, prolines or other amino acids are used to insert one or two amino acids between K218 and C219 and also between C219 and C220.

Insertion mutagenesis of the IgG2 hinge region prevents disulfide bond formation between LC214 and either HC219 or HC220. In some or all mutants, LC C214 forms a disulfide bond with HC C131.

Heterogeneity of structural isoforms in the insertion mutants is analyzed, showing that heterogeneity is reduced by the mutations. CD-SDS, CEX and RP-HPLC analyses indicate a significant reduction in structural heterogeneity in some or all of the insertion mutants, compared to wild type IgG2 antibody. In some cases, the disulfide structure appears completely homogeneous.

Potency of the insertion mutants is analyzed using a bioassay specific for the anti-IL-IR antibody. Chondrocyte and whole blood IL-6 production bioassays, performed as described in Example 7, reveal that many or all of the insertion mutants display enhanced potency for their target molecule.

### EXAMPLE 12

### ENRICHMENT OF IGG2 ANTIBODY CONFORMATIONAL ISOFORMS BY REDOX CONVERSION

### Redox Conversion

Oxidative refolding of proteins from the inclusion body state is a common practice in the microbial production of recombinant proteins but is not typically implemented in mammalian cell production. However, since the heterogeneity of the IgG2 was thought to be disulfide related, the reactivity of the forms to redox treatment was tested. A wild-type anti-IL-1R antibody (described in U.S. Patent Pub. No: 2004/097712 ) was subjected to a variety of redox conditions in the presence and absence of guanidine HCl (GuHCl).

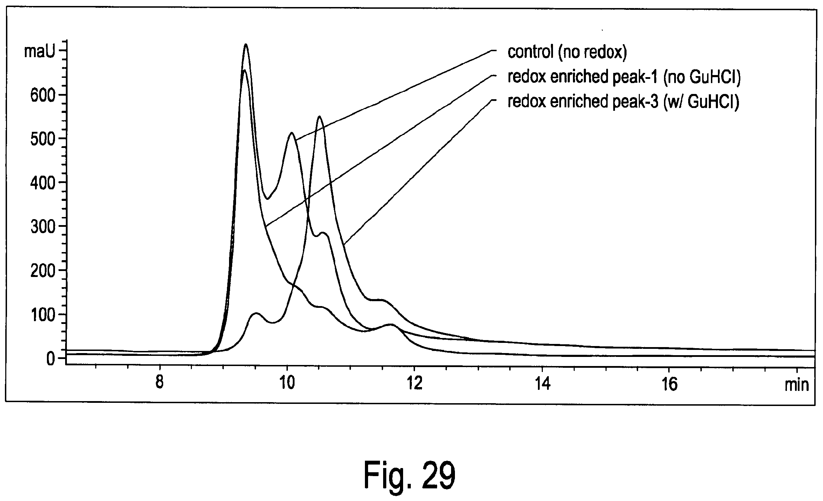

The anti-IL-IR antibody was incubated at 3 mg/mL in one of two buffers: 1) 200 mM Tris buffer at pH 8.0; 2) 200 mM Tris buffer at pH 8.0 with 0.9 M GuHCl. A combination of cysteine and cystamine was added at the molar ratio of 6 mM: 0.6 mM. The samples were placed at 2-8 °C for 24-48 hours. The optimized concentration of GuHCl used in these experiments, 0.9 M, was below that known to affect overall secondary or tertiary structure of the antibody (Teerinen et al., 2006, J. Mol. Biol. 361:687-697), inducing only a minor change in structure to enable the formation of an alternative conformation of IgG2κ. Thus, by changing the parameters of the redox treatment, RP-HPLC peak 1 and peak 3 were preferentially enriched using zero and 0.9 M GuHCl, respectively (Fig. 29). The refolded materials eluted essentially as single homogeneous species that aligned with the peaks seen in the control (no redox). Additionally, several other IgG2 mAbs were redox treated using the same conditions. The RP-HPLC results showed that all IgG2 mAbs tested were reactive to redox treatment. In general, RP-HPLC peaks 3 and 1 were preferentially enriched when the antibodies were exposed to redox with and without GuHCl present, respectively. The enriched forms showed no further conversion after being removed from the redox solution and stored in a PBS buffer.

### EXAMPLE 13

### BIOACTIVITY OF REDOX REFOLDED IGG2 ANTIBODY CONFORMATIONAL ISOFORMS

The bioactivity of IgG2 redox-refolded conformational isoforms was tested in order to determine whether each isoform retained the potency of the bulk, untreated antibody.

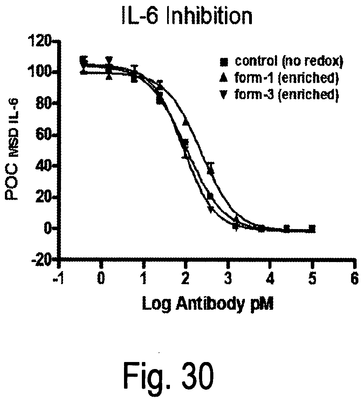

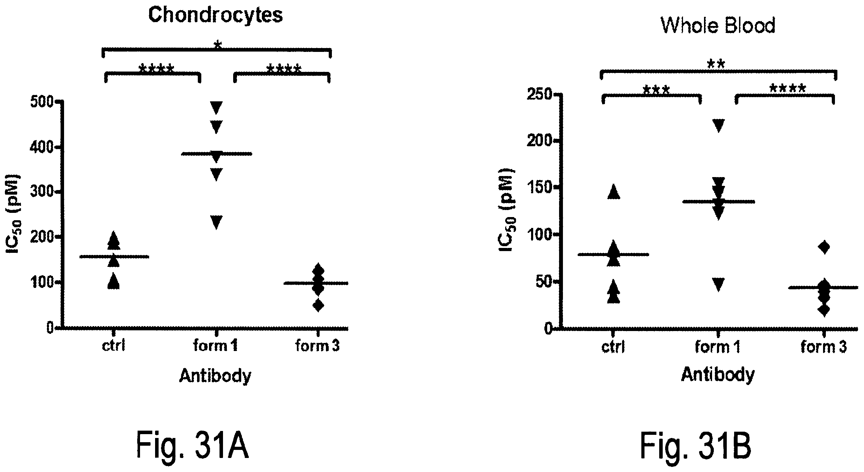

Refolded anti-IL-1R isoforms were tested using the chondrocyte and whole-blood assays as described in Example 7. The results of the IL-1R bioassays showed differences in 11-6 inhibition of the redox enriched material (Figure 30), and demonstrate that the refolded materials were at least as potent, or more potent, than the bulk, heterogeneous antibody. On average, there was a statistically significant difference in IC50 values between the redox enriched materials, and form 1 showed an approximate three-fold lower activity than form 3 (Figures 31A-B).

Refolded anti-IL-4R antibodies were tested using a bioactivity assay that measures inhibition of IL-4R activity. The results are set forth in Table 5 below, and show that the refolded materials were at least as potent, or more potent, than the bulk, heterogeneous antibody.

**Table 5. Anti-IL-4R Bioactivity Data.**

| **Isoform** | **Average Bioactivity (%)** | **Standard Deviation (%)** |
|---|---|---|
| **Form 3** | 142.0 | 32.1 |
| **Form 1** | 78.7 | 23.5 |
| **Bulk (Mixed forms)** | 85.6 | 13.6 |

Refolded anti-HGF antibodies were tested using a bioactivity assay that measures inhibition of HGF signaling activity. The results are set forth in Table 6 below, and show that the refolded materials were at least as potent, or more potent, than the bulk, heterogeneous antibody.

**Table 6. Anti-HGF Bioactivity Data.**

| **Isoform** | **Average Bioactivity (% Potency)** | **Normalized Potency** |
|---|---|---|
| **Form 3** | 85.9 | 1.12 |
| **Form 1** | 96.5 | 1.26 |
| **Bulk (Mixed forms)** | 76.8 | 1 |

Refolded antibody against human glucagon receptor was tested using a bioactivity assay that measures potency of the antibodies. The results are set forth in Table 7 below, and show that the refolded materials were at least as potent, or more potent, than the bulk, heterogeneous antibody.

**Table 7. Anti-Glucagon Receptor Bioactivity Data.**

| **Isoform** | **% Relative Potency** | **%CV** |
|---|---|---|
| **Lot #1 (control)** | 92 | 26.2 |
| **Lot #2 (500 L tank)** | 96 | 10.9 |
| **Lot #3 (formulated)** | 93 | 21.6 |
| **Refold w/ GuHCl at 2-8°C** | 109.9 | 4.8 |
| **Refold no GuHCl at RT** | 76 | 6.3 |

| | | |
|---|---|---|
| Average (n=3) | | |

Refolded anti-EGFr antibody was tested using the EGFr reporter gene expression assay described in Example 10. The results are set forth in Table 8 below, and show that the refolded materials have bioactivity similar to the bulk, heterogeneous antibody.

**Table 8. Anti-EGFr Bioactivity Data.**

| **Sample** | **Specific Activity (Weighted Mean)** |
|---|---|
| **Bulk (Mixed forms)** | 98% |
| **Form 1** | 91% |
| **Form 3** | 87% |

### EXAMPLE 14

### CHARACTERIZATION OF STRUCTURE OF INSERTION MUTANTS OF IGG2 ANTIBODIES

Hinge region insertion mutants described in Example 11 were generated in an anti-IL-1R antibody. Mutants were generated by insertion of alanine in sequence 2 (SEQ ID NO: 14) to generate an IgG2 antibody with the hinge region sequence CACVECPPC (SEQ ID NO: 32), and by insertion of two prolines in sequence 3 (SEQ ID NO: 15) to generate an IgG2 antibody with the hinge region sequence CPPCVECPPC (SEQ ID NO: 33).

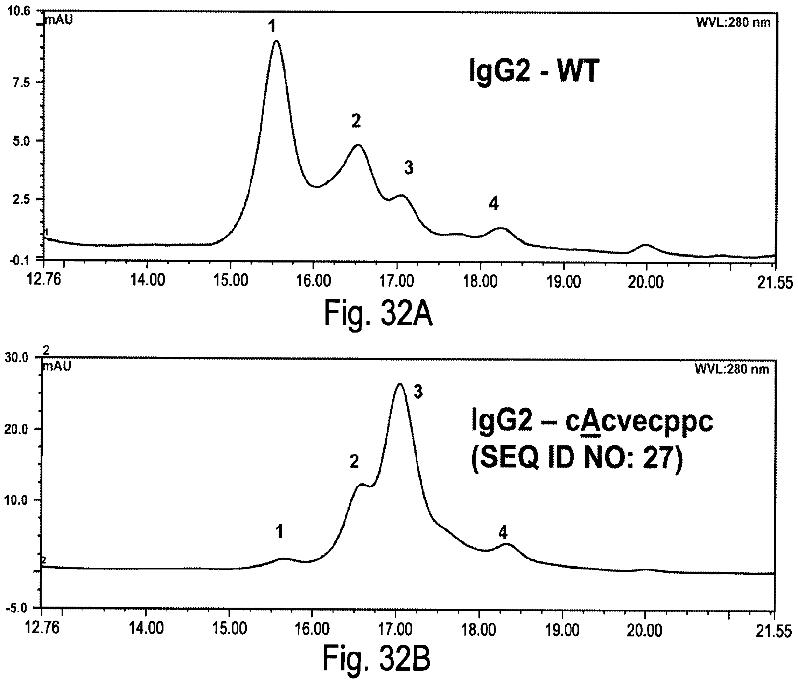

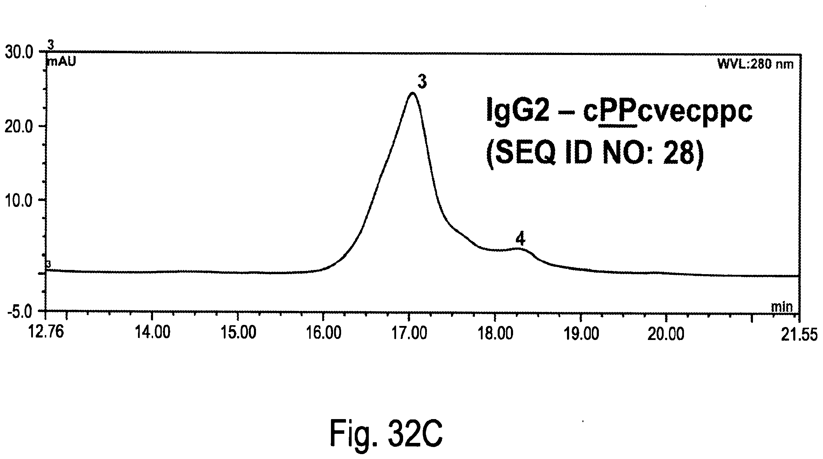

Structural heterogeneity was analyzed by RP-HPLC and the results are set forth in Figure 32. The wild type (WT) sample (Figure 32A) showed a typical four peak profile as seen in other human IgG2 samples. The cAc construct (Figure 32B) showed improved homogeneity. The cPPc construct (Figure 32C) showed a higher degree of homogeneity with no detectable amounts of peak 1 and 2. These results suggest a critical distance and orientation between cysteines was achieved that may be required in order to stop disulfide exchange.

### EXAMPLE 15

### ELIMINATION OF DISULFIDE HETEROGENEITY BY INSERTION MUTATION IN THE C-TERMINAL REGION OF THE LIGHT CHAIN OF IGG2 ANTIBODIES

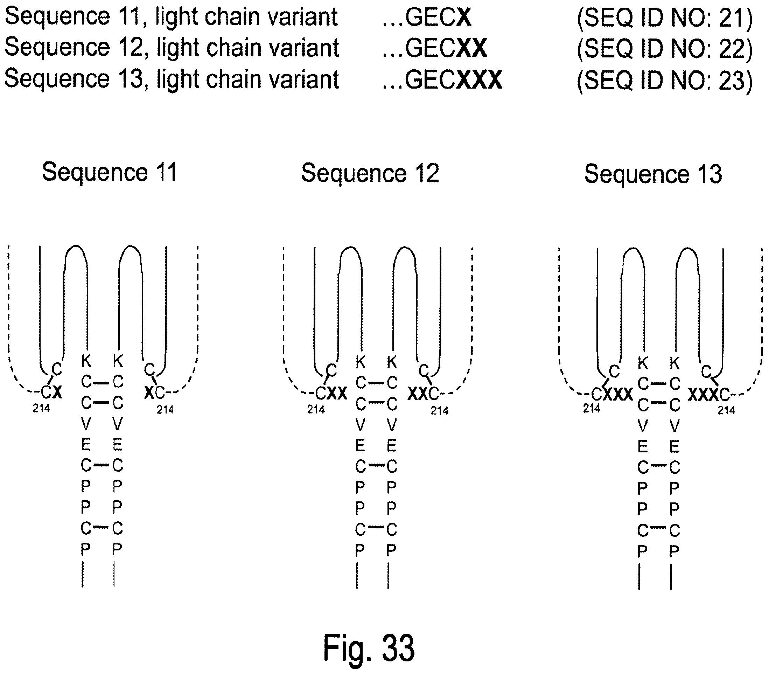

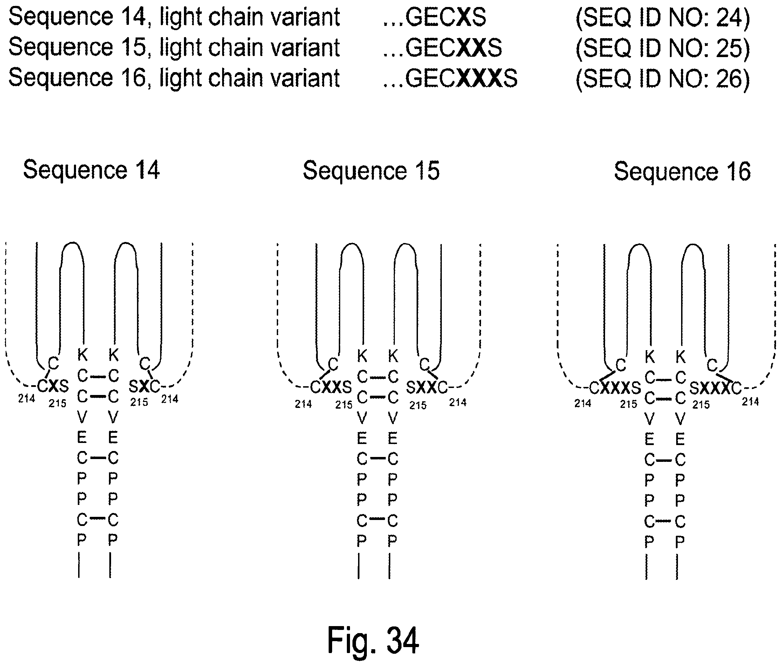

As discussed above, human IgG2 antibodies have structural variants due to the disulfide heterogeneity at the hinge. The disulfide heterogeneity is induced by a reactive and unique cysteine219-cysteine220 (C219C220) motif in the hinge, which reacts with the C-terminal cysteine residue (C214) of the light chain of IgG2 antibodies. In this example, the disulfide heterogeneity is eliminated by inserting one, two, or more amino acid residues after residues C214 and C215 of the light chain as set forth in Figures 33 and 34.

The insertion of one or more amino acids after residues C214 and C215 of the C-terminal region of the light chain of a human IgG2 is designed to lower the redox potential of C214 by separating the C-terminal cysteine from the hinge section of the heavy chain and therefore forcing the production of a homogeneous disulfide form. The expected disulfide form produced from these mutants is Form 3, with LC C214 forming a disulfide bond with HC C131. This is done without mutating native cysteine residues (Figures 33-36). The inserted residues are selected from the list including glycine, proline, serine and other amino acid residues.

The insertion mutations set forth in Figures 33-35 are generated using site-directed insertion mutagenesis. Different versions of sequences 11-13 are generated for IgG2 antibody variants with κ-light chains. In one version of sequence 11, a single glycine is inserted after C214. In another version of sequence 11, a single proline is inserted after C214. In still another version of sequence 11, a single serine is inserted after C214. In different versions of sequences 12 and 13, either two or three glycines, two or three prolines, two or three serines or a combination of two or three glycines or prolines or serines is inserted after C214, as set forth in Figure 33.

Different versions of sequences 14, 15 and 16 are also generated by insertion mutagenesis for IgG2 antibody variants with λ-light chains, set forth in Figure 34. In one version of sequence 14, a single glycine is inserted between C214 and C215. In another version of sequence 14, a single proline is inserted between C214 and C215. In still another version of sequence 14, a single serine is inserted between C214 and C215. In different versions of sequences 15 and 16, either two or three glycines, two or three prolines, two or three serines or a combination of two or three glycines or prolines or serines is inserted between C214 and C215.

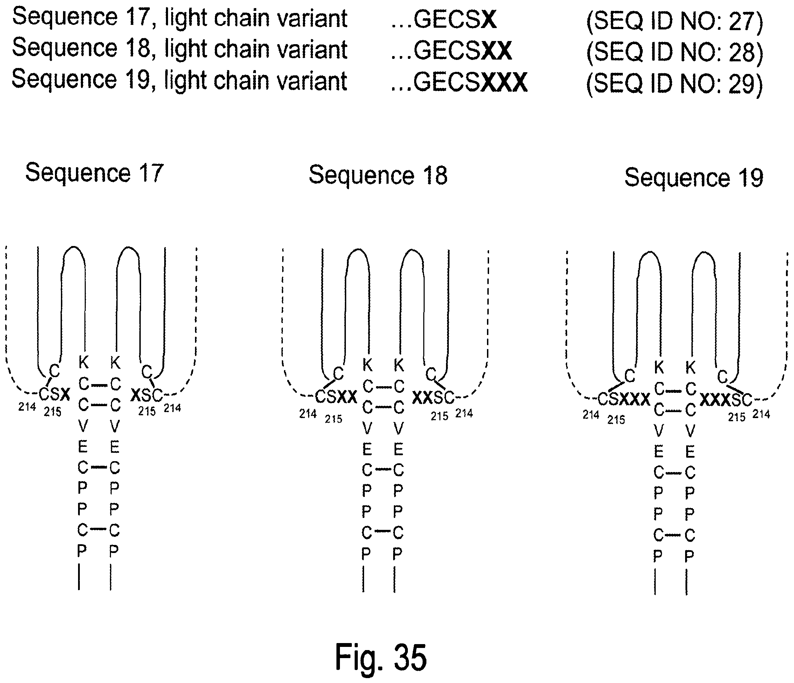

Different versions of sequences 17, 18 and 19 are also generated by insertion mutagenesis for IgG2 antibody variants with λ-light chains, set forth in Figure 35. In one version of sequence 17, a single glycine is inserted after S215. In another version of sequence 17, a single proline is inserted after S215. In still another version of sequence 17, a single serine is inserted after S215. In different versions of sequences 18 and 19, either two or three glycines, two or three prolines, two or three serines or a combination of two or three glycines or prolines or serines is inserted after S215.

Insertion mutagenesis of the IgG2 light chain C-terminal region prevents disulfide bond formation between LC214 and either HC219 or HC220. In some or all mutants, LC C214 forms a disulfide bond with HC C131.

Heterogeneity of structural isoforms in the insertion mutants is analyzed, showing that heterogeneity is reduced by the mutations. CD-SDS, CEX and RP-HPLC analyses indicate a significant reduction in structural heterogeneity in some or all of the insertion mutants, compared to wild type IgG2 antibody. In some cases, the disulfide structure appears completely homogeneous.

Potency of the insertion mutants is analyzed using a bioassay specific for the anti-IL-1R antibody. Chondrocyte and whole blood IL-6 production bioassays, performed as described in Example 7, reveal that many or all of the insertion mutants display enhanced potency for their target molecule.

### EXAMPLE 16

### MUTATION AND CHARACTERIZATION OF ADDITIONAL ANTI-EGFR ANTIBODIES

In addition to the anti-EGFR antibody mutations generated in Example 8 and characterized in Examples 9 and 10, other mutations to anti-EGFR antibodies were made. This example describes the generation of the mutants and the characterization of their structural and biological activities.

Various mutations were made to the heavy chain of the anti-EGFR antibody (described as mAb E7.6.3 in U.S. Patent No. 6,235,883 and comprising the variable region set forth herein as SEQ ID NO: 57). The mutants were designed and then expressed in either CHO stable pools or COS transient production systems, using techniques similar to those described in the above examples. Some mutants were designed to generate antibodies with a single cysteine-to-serine mutation. Other mutants were designed to incorporate two or three cysteine-to-serine mutations. The decision to express in CHO cells versus COS cells was made based only on convenience and was not based on the ability or inability to express a given construct in either expression system. Table 9 below summarizes the mutations that were made.

**Table 9. Anti-EGFR Mutations**

| **Mutation Type** | **Residues Mutated** | **Expression System** |
|---|---|---|
| **Single** | C131S | CHO stable |
| | C219S | CHO stable |
| | C220S | CHO stable |
| | C226S | CHO stable |
| **Double** | C131S / C219S | COS transient |
| | C131S / C220S | COS transient |
| | C219S / C220S | CHO stable |
| **Triple** | C131S / C219S / C220S | COS transient |

The mutant antibodies were purified and then analyzed as described in the examples above using native and reduced Lys-C peptide mapping, non-reduced CE-SDS and NEM / CnBr / trpysin / dSEC separation and isolation of complex disulfide-linked peptides. Partial reduction and alkylation of the C131S mutation was also performed. Non-reduced CE-SDS showed clear differences between mutants and wild type. Peptide mapping showed low recovery of hinge peptide in anti-EGFR reference and wild type, indicating the presence of complex disulfide-linked structures involving the hinge. However, peptide mapping showed good recovery of hinge peptide for mutants at 219 and 220 positions, indicating an absence of complex disulfide-linked structures involving the hinge.

The separation of C226S molecule by nrCE-SDS had a different profile than the wild-type, and exhibited a different peak 1 / peak 2 ratio. Peptide mapping of C226S showed the same distribution of complex disulfide-linked structures compared to wild-type reference, indicating the presence of complex disulfide-linked structures involving the hinge.

Analysis of mutant C131S showed that only forms involving hinge and light chain were observed. Thus, structural forms with C131S are simpler than wild type forms, which exhibit heterogeneity. Confirming this observation, partial reduction-alkylation experiments showed connection of the light chain to C220.

Biological activity of each purified mutant was then assessed using the ELISA binding assay and the potency assay described above in Example 10. The potency and ELISA binding assay results of molecules with mutations in the hinge region were comparable to reference and wild type. However, the potency and ELISA binding assay results of molecules with mutation in the heavy chain outside the hinge region (C131S) were 20-25% lower than reference and wild type.

Thus, these experiments show that mutation of the anti-EGFR heavy chain cysteine residues listed in Table 9 results in no impact on potency and generates molecules that are 100% active. Furthermore, these data show that mutation of a single residue at 219 or 220 is enough to abolish the heterogeneity of structural isoforms. These experiments also show that mutation of heavy chain residue C131 results in lower potency, and generates a homogenous structural form, different from the multiple distribution observed with the wild-type, and having light chain linked to C220. Finally, mutation of C226 maintains the structural isoforms with (hinge-HC-LC) complexes that are similar to the wild-type complexes.

### EXAMPLE 17

### CHARACTERIZATION OF STRUCTURE, STABILITY, AND BIOLOGICAL ACTIVITY OF INSERTION MUTANTS OF IGG2 ANTIBODIES

In order to confirm and expand upon the results described in Example 14, the following experiments were performed. In this example, the disulfide heterogeneity was eliminated in two different mAbs by inserting two amino acid residues between residues C219 and C220 of the heavy chain. Additionally, the insertion mutants of an anti-IL-1RI were tested for formulated stability, disulfide stability in a redox environment, and biological activity. As shown below, the insertion mutants were less heterogeneous, more stable in blood-like redox environment and showed significant increase in potency. Among several inserted residues tested, the cPPc insertion mutant showed overall the greatest formulated stability, relative to the wild-type mAb, in addition to increased biological activity and non reactivity to redox treatment.

Several insertion mutant constructs (set forth in Table 10) were prepared for an anti-IL-1RI IgG2 mAb and tested for their properties.

**Table 10. Insertion Mutant Constructs**

| **Insertion Mutant Construct** | **Core Hinge Sequence** | **SEQ ID NO** |
|---|---|---|
| Wild-type (**WT**) | RKCCVECPPC | 13 |
| Single Ala insertion between Cys²¹⁹/Cys²²⁰ (**cAc**) | RKC**A**CVECPPC | 83 |
| Double Pro insertion between Cys²¹⁹/Cys²²⁰ (**cPPc**) | RKC**PP**CVECPPC | 84 |
| Ala/Asn insertion above Cys²¹⁹/Cys²²⁰ (**ANcc**) | RKA**N**CCVECPPC | 85 |
| Pro/Asn insertion above Cys²¹⁹/Cys²²⁰ (**PNcc**) | RK**PN**CCVECPPC | 86 |

### Disulfide Connectivity

The disulfide connectivity of the insertion mutants was assessed by non-reduced reversed phase (RP) analysis in tandem with ESI-MS in addition to non-reduced Lys-C peptide mapping. As discussed in the previous examples, intact RP analysis of IgG2 mAbs is a sensitive and selective method for assessing IgG2 disulfide isoforms. The wild-type (WT) anti-IL-1RI IgG2 mAb displayed a typical four peak heterogeneous IgG2 profile by RP-HPLC. Analysis of the insertion mutants by RP analysis showed the affect of inserting amino acids before and between Cys²¹⁹/Cys²²⁰ of the heavy chain (HC) on the disulfide isoform distribution. Three out of the four insertion mutants (cAc, PNcc, and ANcc) displayed multiple peaks, although at different distributions, that were comparable to the WT. The two insertions made before Cys²¹⁹/Cys²²⁰ showed a predominant increase in the amount of RP peak 2 and subsequent decrease in the amount of the other peaks. This suggests that by lowering the hinge region by the distance of two amino acids it is possible to shift the disulfide distribution to the intermediate isoform (RP peak 2) but not totally eliminate the disulfide heterogeneity. On the other hand, insertion of amino acids between the two tandem cysteines (Cys²¹⁹/Cys²²⁰) eliminated the disulfide heterogeneity. The insertion of one amino acid (cAc) between the two tandem cysteines produced results comparable to the insertions made above the cysteines, thus suggesting that a single alanine amino acid insertion may not be the most effective in eliminating the disulfide heterogeneity. However, it is possible that a different choice of a single amino acid, larger than alanine, could stop the disulfide heterogeneity. Subsequent mass analysis showed no significant mass difference between the IgG2 RP peaks.

To confirm the exact disulfide connectivity of the anti-IL-1RI insertion mutants, nonreduced Lys-C peptide mapping was performed. Incubation of the mAb with Lys-C protease creates a predictable fragmentation of the peptide backbone that can be subsequently analyzed by RP-LC/MS analysis to determine the disulfide connectivity of the molecule. Detailed characterization of IgG2 disulfide connectivity, including protocols for nonreduced peptide mapping and mass spectrometry identification, is described in the examples above. In addition, the grouping of certain disulfide linked peptides defining the IgG2 isoforms has been described in detail in example 3 and is also briefly described below and in Table 11.

**Table 11. Disulfide linked peptides**

| **Peptide** | **Fragments** |
|---|---|
| P1 | LC fragment L12 + HC disulfide-linked fragments H6-7-8 |
| P2 | L12 fragment + H6-7-8 fragment + two H11-12 fragments |
| P3 | two L12 fragments + two H6-7-8 fragments + two H11-12 fragments |

Nonreduced Lys-C endopeptidase peptide maps of the IgG2 insertion mutants (cAc insertion mutant was excluded because of insufficient sample) and IgG2 WT material showed the same chromatographic profiles with the exception of several late eluting peaks. These peaks contained the expected LC to HC disulfide linked peptides (P1) as well as two other distinct disulfide peptides, where the hinge is covalently linked to one or both C-terminal LC peptides and one or both HC peptides containing Cys131 (P2, P3). The anti-IL-1RI mAb cPPc insertion mutant was the construct that contained only the disulfide-bridged peptide P1, with expected LC to HC disulfide linkage (PI = LC fragment L12 + HC disulfide-linked fragments H6-7-8), and did not contain peptides P2 or P3 (P2 = L12 fragment + H6-7-8 fragment + two H11-12 fragments, P3 = two L12 fragments + two H6-7-8 fragments + two H11-12 fragments). The other IL-1RI mAb constructs (WT, ANcc, and PNcc) contained all of the disulfide-bridged peptides P1, P2, and P3, although P1 was partially enriched in the mutants.

### Stability (formulated)

The stability of the anti-IL-1RI insertion mutants was tested by formulating the protein at 2 mg/mL in PBS (pH 7.2) and incubating the samples at elevated temperatures (37°C and 45°C) and monitoring by size exclusion chromatography. Size-exclusion chromatography (SEC) was run on an Agilent 1100 HPLC system (Agilent Technologies, Inc., Palo Alto CA USA) on two Tosoh Bioscience TSK-Gel G3000 SWxl columns in series (7.8 ID x 30 cm, 5 µm particles). Samples analyzed at 2 mg/mL from which 20 ug were loaded onto the instrument. The mobile phase contained 100 mM Sodium Phosphate, 500 mM Sodium Chloride, 5% Ethanol, pH 7.0. The flow rate was 0.5 mL/min, and column temperature was controlled at 25°C. The signal was monitored by absorbance at wavelengths of 215 and 280 nm.

The cPPc insertion mutant showed greater stability over time with a slower rate of loss of the main peak. The relative percent increase of aggregates and clips was significantly lower for cPPc insertion mutant relative to the WT and other constructs. Additionally, the structural stability was assessed using differential scanning calorimetry. The anti-IL-1RI samples were monitored at 0.5 mg/mL in PBS from 30°C to 95°C, with a scan rate of 1 °C/min. Samples were analyzed using a MicroCal VP-Capillary differential scanning calorimeter system.

The thermal melting profiles of the insertion mutants were compared to the WT. No significant differences in thermal stability could be detected in PBS (pH 7.2). Thus, the formulated stability was improved with the insertion mutations described herein.

### Stability (redox)

As discussed previously, the IgG2 disulfide isoforms are labile and inter-convert when placed in a redox environment. The anti-IL-1RI cPPc insertion mutant was tested for redox stability since it was shown to be comprised of a single IgG2 disulfide isoform. The sample was incubated at approximately 1 mg/mL in a PBS (pH 7.2) solution containing a starting concentration of 4 µM cysteine. The cysteine concentration was not maintained in a steady-state redox environment because cysteine rapidly converts to cystine in an aqueous solution. The sample was incubated for up to 144 hours and monitored by RP chromatography for disulfide conversion. The antibody was analyzed by intact RP-HPLC using a Zorbax 300SB C8 column (150 × 2.1 mm, 5 um or 50 × 1 mm, 3.5 um). The column temperature was 75°C, and the flow rate was 0.5 mL/min. Mobile phase A was 4% IPA/1% ACN/0.11% TFA and mobile phase B was 70% IPA/20% ACN/0.10% TFA.

No conversion was seen by RP for the cPPc insertion mutant over the course of the experiment. Contrary to the mutant, WT was converted to RP peak 1 under similar redox conditions. These data strongly suggest that the cPPc insertion mutant is stable in circulation *in vivo.*

### Biological Activity

The potency of the anti-IL-1RI insertion mutants was compared to the WT using a chondrocyte bioassay. The anti-IL-1RI IgG samples were serially diluted from 400 nM to 1.5 pM in assay media. The diluted test antibodies (50 µl) were added to the wells of 96-well plates seeded with human chondrocytes at a density of 10,000 cells/well in a 100-µl volume. The final antibody concentration ranged from 100 nM to 0.38 pM. After a 30-min incubation, 50 µl of recombinant human IL-1β was added to a final concentration of 10 pM. After incubation overnight, the antibody activities were analyzed using an IL-6 immunoassay with electrochemiluminescence detection (Meso Scale Discovery, Gaithersburg, MD). The inhibition of IL-6 production was calculated as a percentage of maximum IL-1β activity. The inhibition-response curve for each test antibody was established, and the corresponding IC50 values (the concentration of antibody which reduces the signal by 50%) were derived using GraphPad Prism software.

The data from three independent experiments show that the WT anti-IL-1RI mAb was significantly (p<0.001) less active than any of the mutants. There was no significant difference between any of the mutants (p>0.05). This data is consistent with results in example 13 above that showed a significant difference in activity between IgG2 isoforms studied by redox enrichment.

During transient production of the insertion mutants, a higher yield was observed for at least one of the insertion mutant constructs. Additionally, at least one of the insertion mutant constructs showed less IgG fragments during production. These data suggest that restructuring cysteines in the IgG2 hinge can have an impact on yield. Without being bound by theory, these data indicate that disulfide bond formation in and around the IgG hinge creates a bottle neck in protein folding which can affect IgG expression and yields.

The results set forth in this and the preceding examples demonstrates that it is possible to engineer the hinge of IgG2 mAbs in a way that stabilizes the disulfide structure of the molecule for production and *in vivo* stability.

## Claims

1. A method of making a modified IgG2 anti-EGFR antibody comprising:
modifying a nucleotide sequence encoding the heavy chain polypeptide of an IgG2 anti-EGFR antibody such that at least one encoded amino acid is modified;
introducing said nucleic acid into a host cell; and
culturing said host cell such that a plurality of modified IgG2 anti-EGFR antibodies is expressed and secreted,
wherein a substantial population that is at least 50% of the total population of said plurality of modified IgG2 antibodies are a single conformational isoform,
wherein said modification comprises a heavy chain polypeptide modification of said antibody, and wherein said heavy chain polypeptide modification comprises a substitution of the cysteine residue at Eu position 131 of said heavy chain polypeptide with serine (C131S), and
wherein said heavy chain polypeptide modification further comprises a mutation of a cysteine residue within the hinge region, and
wherein said mutation of a cysteine residue within said hinge region of said heavy chain polypeptide comprises a mutation of the cysteine residue at Eu position 219 or 220 of said heavy chain polypeptide to serine (C219S or C220S), and
wherein the heavy chain of said modified IgG2 anti-EGFR antibody comprises the variable region SEQ ID NO: 57.

## Patentansprüche

1. Verfahren zum Herstellen eines modifizierten IgG2-Anti-EGFR-Antikörpers, umfassend:
Modifizieren einer Nukleotidsequenz, die für das Polypeptid der schweren Kette eines IgG2-Anti-EGFR-Antikörpers codiert, so dass mindestens eine codierte Aminosäure modifiziert wird,
Einbringen der Nukleinsäure in eine Wirtszelle und
Kultivieren der Wirtszelle, so dass eine Vielzahl modifizierter IgG2-Anti-EGFR-Antikörper exprimiert und sezerniert wird,
wobei eine wesentliche Population, die mindestens 50 % der Gesamtpopulation der Vielzahl modifizierter IgG2-Antikörper ausmacht, eine einzelne Isoform einer Konformation ist,
wobei die Modifikation eine Polypeptidmodifikation der schweren Kette des Antikörpers umfasst und
wobei die Polypeptidmodifikation der schweren Kette eine Substitution des Cysteinrests an EU-Position 131 des Polypeptids der schweren Kette durch Serin (C131S) umfasst, und
wobei die Polypeptidmodifikation der schweren Kette ferner eine Mutation eines Cysteinrests innerhalb der Hinge-Region umfasst und
wobei die Mutation eines Cysteinrests innerhalb der Hinge-Region des Polypeptids der schweren Kette eine Mutation des Cysteinrests an EU-Position 219 oder 220 des Polypeptids der schweren Kette zu Serin (C219S oder C220S) umfasst und wobei die schwere Kette des modifizierten IgG2-Anti-EGFR-Antikörpers die variable Region SEQ ID NO: 57 umfasst.

## Revendications

1. Procédé de fabrication d'un anticorps IgG2 anti-EGFR modifié comprenant :
la modification d'une séquence nucléotidique codant pour le polypeptide de chaîne lourde d'un anticorps IgG2 anti-EGFR de telle sorte qu'au moins un acide aminé codé soit modifié ;
l'introduction dudit acide nucléique dans une cellule hôte ; et
la culture de ladite cellule hôte de telle sorte qu'une pluralité d'anticorps IgG2 anti-EGFR modifiés soit exprimée et excrétée,
dans lequel une population substantielle qui représente au moins 50 % de la population totale de ladite pluralité d'anticorps IgG2 anti-EGFR modifiés relève d'une seule isoforme conformationnelle,
dans lequel ladite modification comprend une modification du polypeptide de chaîne lourde dudit anticorps, et
dans lequel ladite modification du polypeptide de chaîne lourde comprend un remplacement du résidu cystéine à la position Eu 131 dudit polypeptide de chaîne lourde par une sérine (C131S), et
dans lequel ladite modification du polypeptide de chaîne lourde comprend en outre une mutation d'un résidu cystéine dans la région charnière, et
dans lequel ladite mutation de résidu cystéine dans ladite région charnière dudit polypeptide de chaîne lourde comprend une mutation du résidu cystéine à la position Eu 219 ou 220 dudit polypeptide de chaîne lourde en sérine (C219S ou C220S), et
dans lequel la chaîne lourde dudit anticorps IgG2 anti-EGFR modifié comprend la région variable SEQ ID NO : 57.
